# EUROPEAN PATENT APPLICATION

(11) **EP 4 578 877 A1**
(43) Date of publication of application: **02.07.2025**
(21) Application number: 23849475.1
(22) Date of filing: 02.08.2023
(51) Int. Cl.: C07K 19/00, C07K 16/18, C07K 14/71, C12N 15/62, C12N 15/13, A61P 43/00, A61K 38/16

(54) **ANTIBODY FUSION PROTEIN TARGETING FAP AND TGF-BETA, AND USE THEREOF**

(30) Priority: 03.08.2022 CN 202210935307
(71) Applicant: Nanjing Leads Biolabs Co., Ltd., Jiangbei New Area Nanjing Jiangsu 210031 (CN)
(72) Inventor: LING, Hong, Nanjing, Jiangsu 210019 (CN); HUANG, Xiao, Nanjing, Jiangsu 210019 (CN); DANG, Yujia, Nanjing, Jiangsu 210019 (CN); ZHANG, Peng, Nanjing, Jiangsu 210019 (CN)
(74) Representative: Bianchetti & Minoja with Trevisan & Cuonzo IPS SRL
(86) International application number: PCT/CN2023/110800
(87) International publication number: WO 2024/027771

(57) **Abstract**

Provided in the present invention is an antibody fusion protein, which can specifically bind to an FAP protein and block a TGFβ signaling pathway. Further provided in the present invention are a nucleic acid molecule encoding the antibody fusion protein, an expression vector for expressing the antibody fusion protein, a host cell and a preparation method therefor. Further provided in the present invention is the use of the antibody fusion protein.

## Description

### TECHNICAL FIELD

The present disclosure provides a bifunctional antibody fusion protein targeting FAP and TGFβ. The antibody fusion protein is capable of specifically binding to FAP protein and blocking the TGFβ signaling pathway. The present disclosure further provides a nucleic acid molecule encoding the antibody fusion protein, an expression vector for expressing the antibody fusion protein, a host cell, and a method for preparing same. The present disclosure further provides use of the antibody fusion protein.

### BACKGROUND

Fibrotic diseases have the pathological feature of excessive fibrotic connective tissues in organs, e.g., the accumulation of collagen, and the etiology of which generally includes factors of chemical injury, persistent infection, metabolism, autoimmune reaction, etc. Fibrotic diseases are a consequential, key pathological feature of chronic inflammations that may affect the pathological progression. They also represent the progression of the disease to a more severe stage, greatly affecting the functions of important organs, e.g., the heart, lung, liver, and kidney. Clinically, fibrosis occurs in numerous diseases as a main pathological feature and process, and among the diseases, liver cirrhosis caused by HBV, renal fibrosis induced by diabetes, as well as idiopathic pulmonary fibrosis (IPF) with unknown cause, and skin diseases are commonly seen. Fibrosis is a repairing reaction for chronic injuries and persistent inflammations and may be a reversible process in the early stages. However, fibrosis may cause structural damages to tissues and dysfunction in organs with its continuous progression. The fibrotic response is associated with TGFβ-driven fibroblast changes, including: the activation and overgrowth of fibroblasts in the resting state; the phenotype transition to myofibroblasts; increased production of matrix proteins and thus induced extracellular collagen deposition; inhibited MMP synthesis, induced expression and synthesis of protease inhibitor TIMP-1, inhibited enzymatic degradation of matrices, and up-regulated connective tissue growth factor (CTGF), which further promote fibroblast proliferation (ANNA BIERNACKA, et al. (2011). TGF-β signaling in fibrosis).

The activation of fibroblasts plays a prominent role in fibrosis, and the involved proteins include fibroblast-activation protein (FAP), α-smooth muscle actin (α-SMA), vimentin, etc. Since other molecules are also expressed in resting fibroblasts, pericytes, as well as vascular smooth muscle cells but FAP is only highly specifically expressed in activated fibroblasts, FAP is a better molecular marker for fibrosis. Meanwhile, FAP is also an important molecule that potentially drives the fibrotic process. FAP is a cell surface glycoprotein expressed on activated fibroblasts. It is barely found in healthy adults, but is expressed to some extent in the conditions of wound healing, fibrotic diseases, and the like involved in some sites with active tissue repair (Pinak S., et al. (2006). "Fibroblast activation protein: a serine protease expressed at the remodeling interface in idiopathic pulmonary fibrosis" Human Pathology (2006) 37, 352-360).

TGF-β molecule regulates a variety of biological processes and relates to a variety of diseases, including cardiovascular diseases, fibrotic diseases, reproductive diseases, tumors, etc. The TGF-β signaling pathway is primarily transduced through the phosphorylation of Smad2/3. TGF-β produced in cells is in an inactive form, and its activation requires a complicated process: Initially, TGF-β, in a homodimeric form, covalently binds to latent transforming growth factor β binding protein (LTBP). The activation of TGF-β requires proteolysis in the matrix, and the subsequent activation processes are related to integrin-mediated conformational changes before active TGF-β is ultimately released. Subsequently, the binding of TGF-β to TGFβ-RII may further recruit TGFβ-RI and result in the binding of the three, thereby activating the catalytic activity of TGFβ-RI. The latter can trigger the phosphorylation of serine residues of Smad2 and Smad3. Phosphorylated Smad2 and Smad3, along with Smad4, form a trimeric complex, which enters the nucleus and regulates the transcription of target genes. The regulated target genes include collagens, fibronectin, tissue inhibitors of metalloproteinase (TIMPs), α-SMA, and the like (Xiao-ming Meng, et al. (2016). TGF-β: the master regulator of fiberosis).

The pathological characteristics and mechanisms of the development and progression of fibrosis and the effects on functionality are similar in different organs, including the coincidence of cells involved in the pathological process of the fibrosis, e.g., the key role played by the TGF-β signaling pathway in the activation of fibroblasts. Currently, no studies on bifunctional antibody fusion proteins targeting FAP and TGFβ have bee reported worldwide.

The present disclosure explored the bifunctional antibody fusion protein targeting FAP and TGFβ without precedents, and found that the delivery of a TGFβR2 fusion protein to fibrotic tissues by FAP-targeting functionality may bring favorable influences by the antagonizing effect on TGFβ signaling pathway. Meanwhile, in the present disclosure, the activated fibroblasts generating fibrotic components in the extracellular matrix can be killed and scavenged using the Fc effect of an anti-FAP antibody, such that by coupling with TGF-β antagonizing effect, tissue and cell damages are ameliorated through various mechanisms, and the generation of fibrotic components such as collagen is reduced and inhibited.

### SUMMARY

The present disclosure relates to a bifunctional antibody fusion protein targeting FAP and TGFβ.

In some aspects, the present disclosure relates to the following embodiments:
1. A fusion protein molecule specifically binding to human FAP and human TGFβ, e.g., human TGFβ1, TGFβ2, and/or TGFβ3.
2. The fusion protein molecule according to embodiment 1, comprising a first target-binding region and a second target-binding region, wherein the first target-binding region specifically binds to FAP, and the second target-binding region specifically binds to TGFβ.
3. The fusion protein molecule according to embodiment 2, wherein the first target-binding region and the second target-binding region are connected with a linker or directly connected without a linker; for example, the linker is (G₄S)ₙ, wherein n = 1, 2, 3, 4, 5, or 6, e.g., n = 4.
4. The fusion protein molecule according to embodiment 2 or 3, wherein the first target-binding region is derived from an anti-FAP antibody; e.g., an F19 antibody or a humanized antibody thereof or a 28H1 antibody or a humanized antibody thereof.
5. The fusion protein molecule according to any one of embodiments 2-4, wherein the first target-binding region comprises 3 CDRs of a heavy chain variable region VH: an HCDR1, an HCDR2, and an HCDR3, and 3 CDRs of a light chain variable region VL: an LCDR1, an LCDR2, and an LCDR3, wherein
   (i) the HCDR1, the HCDR2, and the HCDR3 are selected from the three complementarity determining regions HCDR1, HCDR2, and HCDR3 in the VH set forth in SEQ ID NO: 7; and the LCDR1, the LCDR2, and the LCDR3 are the three complementarity determining regions LCDR1, LCDR2, and LCDR3 in the VL set forth in SEQ ID NO: 1;
   (ii) the HCDR1, the HCDR2, and the HCDR3 are selected from the three complementarity determining regions HCDR1, HCDR2, and HCDR3 in the VH set forth in SEQ ID NO: 13; and the LCDR1, the LCDR2, and the LCDR3 are the three complementarity determining regions LCDR1, LCDR2, and LCDR3 in the VL set forth in SEQ ID NO: 15;
   (iii) the HCDR1, the HCDR2, and the HCDR3 are selected from the three complementarity determining regions HCDR1, HCDR2, and HCDR3 in the VH set forth in SEQ ID NO: 21; and the LCDR1, the LCDR2, and the LCDR3 are the three complementarity determining regions LCDR1, LCDR2, and LCDR3 in the VL set forth in SEQ ID NO: 15;
   (iv) the HCDR1, the HCDR2, and the HCDR3 are selected from the three complementarity determining regions HCDR1, HCDR2, and HCDR3 in the VH set forth in SEQ ID NO: 30; and the LCDR1, the LCDR2, and the LCDR3 are the three complementarity determining regions LCDR1, LCDR2, and LCDR3 in the VL set forth in SEQ ID NO: 27; or
   (v) relative to the sequence in any one of (i)-(iv), a sequence comprising at least one and no more than 5, 4, 3, 2, or 1 amino acid modification (preferably amino acid substitution, and more preferably conservative substitution) in the three HCDRs or the three LCDRs; for example, the amino acid modification is a mutation enhancing the stability of the antibody, e.g., a mutation at a deamidation site in, e.g., the HCDR2, including a G56R substitution;
   preferably, the CDRs are determined by the Kabat numbering scheme.
6. The fusion protein molecule according to any one of embodiments 2-4, wherein the first target-binding region comprises:
   (i) the HCDR1 set forth in SEQ ID NO: 10, the HCDR2 set forth in SEQ ID NO: 11, the HCDR3 set forth in SEQ ID NO: 12; the LCDR1 set forth in SEQ ID NO: 4, the LCDR2 set forth in SEQ ID NO: 5, and the LCDR3 set forth in SEQ ID NO: 6;
   (ii) the HCDR1 set forth in SEQ ID NO: 10, the HCDR2 set forth in SEQ ID NO: 22, the HCDR3 set forth in SEQ ID NO: 12; the LCDR1 set forth in SEQ ID NO: 4, the LCDR2 set forth in SEQ ID NO: 5, and the LCDR3 set forth in SEQ ID NO: 6;
   (iii) the HCDR1, HCDR2, HCDR3 and LCDR1, LCDR2, and LCDR3 of the 28H1 antibody disclosed in WO2012020006; or
   (iv) relative to the sequence in any one of (i), (ii), or (iii), a sequence comprising at least one and no more than 5, 4, 3, 2, or 1 amino acid modification (preferably amino acid substitution, and more preferably conservative substitution) in the three HCDRs or the three LCDRs; for example, the amino acid modification is a mutation enhancing the stability of the antibody, e.g., a mutation at a deamidation site in, e.g., the HCDR2, including a G56R substitution.
7. The fusion protein molecule according to any one of embodiments 2-6, wherein the first target-binding region comprises a heavy chain variable region VH, and the heavy chain variable region:
   (i) comprises or consists of an amino acid sequence having at least 90%, 91%, 92%, 93%, 94%, 95%, 96%, 97%, 98%, or 99% identity to the amino acid sequence of SEQ ID NO: 7, 13, 21, or 30; or
   (ii) comprises or consists of the amino acid sequence of SEQ ID NO: 7, 13, 21, or 30.
8. The fusion protein molecule according to any one of embodiments 2-7, wherein the first target-binding region comprises a light chain variable region VL, and the light chain variable region:
   (i) comprises or consists of an amino acid sequence having at least 90%, 91%, 92%, 93%, 94%, 95%, 96%, 97%, 98%, or 99% identity to the amino acid sequence of SEQ ID NO: 1, 15, or 27; or
   (ii) comprises or consists of the amino acid sequence of SEQ ID NO: 1, 15, or 27.
9. The fusion protein molecule according to any one of embodiments 2-8, comprising a heavy chain variable region VH and a light chain variable region VL, wherein:
   (i) the heavy chain variable region comprises or consists of the amino acid sequence set forth in SEQ ID NO: 7, or an amino acid sequence having at least 90%, 91%, 92%, 93%, 94%, 95%, 96%, 97%, 98%, or 99% identity to the amino acid sequence; and the light chain variable region comprises or consists of the amino acid sequence set forth in SEQ ID NO: 1, or an amino acid sequence having at least 90%, 91%, 92%, 93%, 94%, 95%, 96%, 97%, 98%, or 99% identity to the amino acid sequence;
   (ii) the heavy chain variable region comprises or consists of the amino acid sequence set forth in SEQ ID NO: 13, or an amino acid sequence having at least 90%, 91%, 92%, 93%, 94%, 95%, 96%, 97%, 98%, or 99% identity to the amino acid sequence; and the light chain variable region comprises or consists of the amino acid sequence set forth in SEQ ID NO: 15, or an amino acid sequence having at least 90%, 91%, 92%, 93%, 94%, 95%, 96%, 97%, 98%, or 99% identity to the amino acid sequence;
   (iii) the heavy chain variable region comprises or consists of the amino acid sequence set forth in SEQ ID NO: 21, or an amino acid sequence having at least 90%, 91%, 92%, 93%, 94%, 95%, 96%, 97%, 98%, or 99% identity to the amino acid sequence; and the light chain variable region comprises or consists of the amino acid sequence set forth in SEQ ID NO: 15, or an amino acid sequence having at least 90%, 91%, 92%, 93%, 94%, 95%, 96%, 97%, 98%, or 99% identity to the amino acid sequence; or
   (iv) the heavy chain variable region comprises or consists of the amino acid sequence set forth in SEQ ID NO: 30, or an amino acid sequence having at least 90%, 91%, 92%, 93%, 94%, 95%, 96%, 97%, 98%, or 99% identity to the amino acid sequence; and the light chain variable region comprises or consists of the amino acid sequence set forth in SEQ ID NO: 27, or an amino acid sequence having at least 90%, 91%, 92%, 93%, 94%, 95%, 96%, 97%, 98%, or 99% identity to the amino acid sequence.
10. The fusion protein molecule according to any one of embodiments 2-9, wherein the first target-binding region comprises a heavy chain variable region VH and a light chain variable region VL, and the VH and the VL respectively comprise or consist of the amino acid sequences set forth as follows:
   (i)SEQ ID NO: 7 and SEQ ID NO: 1;
   (ii)SEQ ID NO: 13 and SEQ ID NO: 15;
   (iii)SEQ ID NO: 21 and SEQ ID NO: 15; or
   (iv)SEQ ID NO: 30 and SEQ ID NO: 27.
11. The fusion protein molecule according to any one of embodiments 2-10, wherein the first target-binding region further comprises an Fc or a heavy chain constant region CH; for example,
   the Fc region is the Fc region of an IgG1, IgG2, IgG3, or IgG4, preferably the Fc region of an IgG1; and optionally, amino acid K at the terminus of the Fc region is deleted or mutated to A; or
   the antibody heavy chain constant region CH is the heavy chain constant region of an IgG1, IgG2, IgG3, or IgG4, preferably the heavy chain constant region of an IgG1; and optionally, amino acid K at the terminus of the heavy chain constant region is deleted or mutated to A.
12. The fusion protein molecule according to embodiment 11, wherein the heavy chain constant region CH:
   (i) comprises or consists of an amino acid sequence having at least 85%, 90%, 91%, 92%, 93%, 94%, 95%, 96%, 97%, 98%, or 99% identity to the amino acid sequence of SEQ ID NO: 8; or
   (ii) comprises or consists of the amino acid sequence of SEQ ID NO: 8; or
   the Fc region:
   (i) comprises or consists of an amino acid sequence having at least 85%, 90%, 91%, 92%, 93%, 94%, 95%, 96%, 97%, 98%, or 99% identity to the amino acid sequence of SEQ ID NO: 40; or
   (ii) comprises or consists of the amino acid sequence of SEQ ID NO: 40.
13. The fusion protein molecule according to any one of embodiments 2-12, wherein the first target-binding region further comprises a light chain constant region; for example, the light chain constant region is a lambda or kappa light chain constant region.
14. The fusion protein molecule according to embodiment 13, wherein the light chain constant region:
   (i) comprises or consists of an amino acid sequence having at least 85%, 90%, 91%, 92%, 93%, 94%, 95%, 96%, 97%, 98%, or 99% identity to the amino acid sequence of SEQ ID NO: 2; or
   (ii) comprises or consists of the amino acid sequence of SEQ ID NO: 2.
15. The fusion protein molecule according to any one of embodiments 2-14, wherein the first target-binding region comprises a heavy chain, and the heavy chain comprises or consists of the amino acid sequence of SEQ ID NO: 9, 14, 23, 35, 41, or 42, or an amino acid sequence having at least 85%, 90%, 91%, 92%, 93%, 94%, 95%, 96%, 97%, 98%, or 99% identity to the amino acid sequence.
16. The fusion protein molecule according to any one of embodiments 2-15, wherein the first target-binding region comprises a light chain, and the light chain comprises or consists of the amino acid sequence of SEQ ID NO: 3, 16, or 28, or an amino acid sequence having at least 85%, 90%, 91%, 92%, 93%, 94%, 95%, 96%, 97%, 98%, or 99% identity to the amino acid sequence.
17. The fusion protein molecule according to embodiment 15 or 16, wherein the first target-binding region comprises a heavy chain and a light chain, wherein
   (i) the heavy chain comprises or consists of the amino acid sequence of SEQ ID NO: 9, or an amino acid sequence having at least 85%, 90%, 91%, 92%, 93%, 94%, 95%, 96%, 97%, 98%, or 99% identity to the amino acid sequence; and the light chain comprises or consists of the amino acid sequence of SEQ ID NO: 3, or an amino acid sequence having at least 85%, 90%, 91%, 92%, 93%, 94%, 95%, 96%, 97%, 98%, or 99% identity to the amino acid sequence;
   (ii) the heavy chain comprises or consists of the amino acid sequence of SEQ ID NO: 14, or an amino acid sequence having at least 85%, 90%, 91%, 92%, 93%, 94%, 95%, 96%, 97%, 98%, or 99% identity to the amino acid sequence; and the light chain comprises or consists of the amino acid sequence of SEQ ID NO: 16, or an amino acid sequence having at least 85%, 90%, 91%, 92%, 93%, 94%, 95%, 96%, 97%, 98%, or 99% identity to the amino acid sequence;
   (iii) the heavy chain comprises or consists of the amino acid sequence of SEQ ID NO: 23, or an amino acid sequence having at least 85%, 90%, 91%, 92%, 93%, 94%, 95%, 96%, 97%, 98%, or 99% identity to the amino acid sequence; and the light chain comprises or consists of the amino acid sequence of SEQ ID NO: 16, or an amino acid sequence having at least 85%, 90%, 91%, 92%, 93%, 94%, 95%, 96%, 97%, 98%, or 99% identity to the amino acid sequence;
   (iv) the heavy chain comprises or consists of the amino acid sequence of SEQ ID NO: 35, or an amino acid sequence having at least 85%, 90%, 91%, 92%, 93%, 94%, 95%, 96%, 97%, 98%, or 99% identity to the amino acid sequence; and the light chain comprises or consists of the amino acid sequence of SEQ ID NO: 16, or an amino acid sequence having at least 85%, 90%, 91%, 92%, 93%, 94%, 95%, 96%, 97%, 98%, or 99% identity to the amino acid sequence; or
   (v) the heavy chain comprises or consists of the amino acid sequence of SEQ ID NO: 41 or 42, or an amino acid sequence having at least 85%, 90%, 91%, 92%, 93%, 94%, 95%, 96%, 97%, 98%, or 99% identity to the amino acid sequence; and the light chain comprises or consists of the amino acid sequence of SEQ ID NO: 28, or an amino acid sequence having at least 85%, 90%, 91%, 92%, 93%, 94%, 95%, 96%, 97%, 98%, or 99% identity to the amino acid sequence.
18. The fusion protein molecule according to embodiment 17, wherein the first target-binding region comprises a heavy chain and a light chain respectively comprising or consisting of the amino acid sequences set forth in the following SEQ ID NOs:
   (i)SEQ ID NO: 9 and SEQ ID NO: 3;
   (ii)SEQ ID NO: 14 and SEQ ID NO: 16;
   (iii)SEQ ID NO: 23 and SEQ ID NO: 16;
   (iv)SEQ ID NO: 35 and SEQ ID NO: 16;
   (v)SEQ ID NO: 41 and SEQ ID NO: 28; or
   (vi)SEQ ID NO: 42 and SEQ ID NO: 28.
19. The fusion protein molecule according to any one of embodiments 2-18, wherein the first target-binding region is a humanized antibody or a chimeric antibody.
20. The fusion protein molecule according to any one of embodiments 2-19, wherein the first target-binding region is a monoclonal antibody.
21. The fusion protein molecule according to any one of embodiments 2-20, wherein the first target-binding region is an antigen-binding fragment selected from a Fab, a Fab', a Fab'-SH, an Fv, a single-chain antibody (e.g., scFv), (Fab')₂, a single-domain antibody (e.g., VHH), a domain antibody (dAb), and a linear antibody.
22. The fusion protein molecule according to any one of embodiments 2-21, wherein the second target-binding region comprises a TGFβ receptor specifically binding to TGFβ or a functional fragment thereof.
23. The fusion protein molecule according to embodiment 22, wherein the TGFβ receptor is a TGFβRII molecule; e.g., the TGFβRII molecule:
   (i) comprises or consists of an amino acid sequence having at least 85%, 90%, 91%, 92%, 93%, 94%, 95%, 96%, 97%, 98%, or 99% identity to an amino acid sequence selected from SEQ ID NO: 34;
   (ii) comprises or consists of an amino acid sequence selected from SEQ ID NO: 34; or
   (iii) comprises or consists of an amino acid sequence having 1 or more (preferably no more than 20 or 10, and more preferably no more than 5, 4, 3, 2, or 1) amino acid modifications (preferably amino acid substitutions, and more preferably amino acid conservative substitutions) compared to an amino acid sequence selected from SEQ ID NO: 34.
24. The fusion protein molecule according to embodiment 22 or 23, wherein the functional fragment comprises an ECD or is an ECD.
25. The fusion protein molecule according to embodiment 24, wherein the ECD is an ECD of a wild-type TGFβ receptor, e.g., an ECD of a wild-type TGFβRII molecule, or a variant ECD comprising a modification relative to a wild-type ECD; for example, the modification comprises a truncation at the N-terminus or the C-terminus or a substitution.
26. The fusion protein molecule according to embodiment 25, wherein
   the substitution is selected from: a substitution of G at position 20 of the ECD with T, i.e., G20T; a substitution of N at position 19 of the ECD with E; and a combination of the two; or
   the truncation is an N-terminus truncation, e.g., a deletion of any or all of the amino acids at positions 1-19 at the N-terminus, for example, selected from a deletion of amino acids at positions 1-19 of the ECD, a deletion of amino acids at positions 4-19 of the ECD, and a deletion of amino acids at positions 18-19 of the ECD.
27. The fusion protein molecule according to any one of embodiments 24-26, wherein the ECD is an ECD of a TGFβRII molecule; for example, the ECD of the TGFβRII molecule:
   (i) comprises or consists of an amino acid sequence having at least 85%, 90%, 91%, 92%, 93%, 94%, 95%, 96%, 97%, 98%, or 99% identity to the amino acid sequence of SEQ ID NO: 24, 37, or 39;
   (ii) comprises or consists of the amino acid sequence of SEQ ID NO: 24, 37, or 39;
   (iii) comprises or consists of an amino acid sequence having 1 or more (preferably no more than 20 or 10, and more preferably no more than 5, 4, 3, 2, or 1) amino acid modificationss (preferably amino acid substitutions, and more preferably amino acid conservative substitutions) compared to the amino acid sequence of SEQ ID NO: 24, 37, or 39; wherein preferably, the amino acid modification is selected from the presence of T at position 20 (i.e., comprising G20T), and/or the presence of E at position 19 (i.e., comprising N19E);
   (iv) comprises an amino acid sequence having a truncation at the N-terminus and/or C-terminus compared to any one of (i)-(iii), wherein preferably, the truncation refers to a deletion of any or all of the amino acids at positions 1-19 at the N-terminus, e.g., a deletion of amino acids at positions 1-19 of the ECD, a deletion of amino acids at positions 4-19 of the ECD, or a deletion of amino acids at positions 18-19 of the ECD.
28. The fusion protein molecule according to any one of embodiments 1-27, comprising:
   (i) chain A: an anti-FAP antibody light chain; and
   (ii) chain B: an anti-FAP antibody heavy chain connected, at the N-terminus or C-terminus, to a TGFβ receptor or a functional fragment thereof with or without a linker.
29. The fusion protein molecule according to embodiment 28, consisting of 2 chains A and 2 chains B.
30. The fusion protein molecule according to embodiment 28 or 29, wherein chain A comprises or consists of the amino acid sequence set forth in SEQ ID NO: 16, or an amino acid sequence having at least 85%, 90%, 91%, 92%, 93%, 94%, 95%, 96%, 97%, 98%, or 99% identity to the amino acid sequence.
31. The fusion protein molecule according to any one of embodiments 28-30, wherein chain B comprises or consists of the amino acid sequence set forth in SEQ ID NO: 25, 36, or 38, or an amino acid sequence having at least 85%, 90%, 91%, 92%, 93%, 94%, 95%, 96%, 97%, 98%, or 99% identity to the amino acid sequence.
32. The fusion protein molecule according to embodiment 28 or 29, wherein
   chain A comprises or consists of the amino acid sequence set forth in SEQ ID NO: 16, or an amino acid sequence having at least 85%, 90%, 91%, 92%, 93%, 94%, 95%, 96%, 97%, 98%, or 99% identity to the amino acid sequence; and
   chain B comprises or consists of the amino acid sequence set forth in SEQ ID NO: 25, 36, or 38, or an amino acid sequence having at least 85%, 90%, 91%, 92%, 93%, 94%, 95%, 96%, 97%, 98%, or 99% identity to the amino acid sequence; or
   chain A comprises or consists of the amino acid sequence set forth in SEQ ID NO: 28, or an amino acid sequence having at least 85%, 90%, 91%, 92%, 93%, 94%, 95%, 96%, 97%, 98%, or 99% identity to the amino acid sequence; and
   chain B comprises or consists of the amino acid sequence set forth in SEQ ID NO: 29, or an amino acid sequence having at least 85%, 90%, 91%, 92%, 93%, 94%, 95%, 96%, 97%, 98%, or 99% identity to the amino acid sequence.
33. The fusion protein molecule according to embodiment 32, wherein chain A comprises or consists of the amino acid sequence set forth in SEQ ID NO: 16, and chain B comprises or consists of the amino acid sequence set forth in SEQ ID NO: 25, 36, or 38; or chain A comprises or consists of the amino acid sequence set forth in SEQ ID NO: 28, and chain B comprises or consists of the amino acid sequence set forth in SEQ ID NO: 29.
34. An anti-FAP antibody or an antigen-binding fragment thereof, comprising:
   the HCDR1 set forth in SEQ ID NO: 10, the HCDR2 set forth in SEQ ID NO: 22, the HCDR3 set forth in SEQ ID NO: 12; the LCDR1 set forth in SEQ ID NO: 4, the LCDR2 set forth in SEQ ID NO: 5, and the LCDR3 set forth in SEQ ID NO: 6.
35. An anti-FAP antibody or an antigen-binding fragment thereof, comprising a heavy chain variable region VH and a light chain variable region VL, wherein
   (i) the heavy chain variable region comprises or consists of the amino acid sequence set forth in SEQ ID NO: 13, or an amino acid sequence having at least 90%, 91%, 92%, 93%, 94%, 95%, 96%, 97%, 98%, or 99% identity to the amino acid sequence; and the light chain variable region comprises or consists of the amino acid sequence set forth in SEQ ID NO: 15, or an amino acid sequence having at least 90%, 91%, 92%, 93%, 94%, 95%, 96%, 97%, 98%, or 99% identity to the amino acid sequence; or
   (iii) the heavy chain variable region comprises or consists of the amino acid sequence set forth in SEQ ID NO: 21, or an amino acid sequence having at least 90%, 91%, 92%, 93%, 94%, 95%, 96%, 97%, 98%, or 99% identity to the amino acid sequence; and the light chain variable region comprises or consists of the amino acid sequence set forth in SEQ ID NO: 15, or an amino acid sequence having at least 90%, 91%, 92%, 93%, 94%, 95%, 96%, 97%, 98%, or 99% identity to the amino acid sequence.
36. The anti-FAP antibody or the antigen-binding fragment thereof according to embodiment 34 or 35, comprising a heavy chain variable region VH and a light chain variable region VL, wherein the VH and the VL respectively comprise or consist of the amino acid sequences set forth as follows:
   (i)SEQ ID NO: 13 and SEQ ID NO: 15; or
   (ii)SEQ ID NO: 21 and SEQ ID NO: 15.
37. The anti-FAP antibody or the antigen-binding fragment thereof according to any one of embodiments 34-36, further comprising an Fc or a heavy chain constant region CH, wherein for example,
   the Fc region is the Fc region of an IgG1, IgG2, IgG3, or IgG4, preferably the Fc region of an IgG1; and optionally, amino acid K at the terminus of the Fc region is deleted or mutated to A; or
   the antibody heavy chain constant region CH is the heavy chain constant region of an IgG1, IgG2, IgG3, or IgG4, preferably the heavy chain constant region of an IgG1; and optionally, amino acid K at the terminus of the heavy chain constant region is deleted or mutated to A.
38. The anti-FAP antibody or the antigen-binding fragment thereof according to embodiment 37, wherein the heavy chain constant region CH
   (i) comprises or consists of an amino acid sequence having at least 85%, 90%, 91%, 92%, 93%, 94%, 95%, 96%, 97%, 98%, or 99% identity to the amino acid sequence of SEQ ID NO: 8; or
   (ii) comprises or consists of the amino acid sequence of SEQ ID NO: 8; or
   the Fc region:
   (i) comprises or consists of an amino acid sequence having at least 85%, 90%, 91%, 92%, 93%, 94%, 95%, 96%, 97%, 98%, or 99% identity to the amino acid sequence of SEQ ID NO: 40; or
   (ii) comprises or consists of the amino acid sequence of SEQ ID NO: 40.
39. The anti-FAP antibody or the antigen-binding fragment thereof according to any one of embodiments 34-38, further comprising a light chain constant region, wherein for example, the light chain constant region is a lambda or kappa light chain constant region.
40. The anti-FAP antibody or the antigen-binding fragment thereof according to embodiment 39, wherein the light chain constant region
   (i) comprises or consists of an amino acid sequence having at least 85%, 90%, 91%, 92%, 93%, 94%, 95%, 96%, 97%, 98%, or 99% identity to the amino acid sequence of SEQ ID NO: 2; or
   (ii) comprises or consists of the amino acid sequence of SEQ ID NO: 2.
41. The anti-FAP antibody or the antigen-binding fragment thereof according to any one of embodiments 34-40, comprising a heavy chain, wherein the heavy chain comprises or consists of the amino acid sequence of SEQ ID NO: 14, 23, or 35, or an amino acid sequence having at least 85%, 90%, 91%, 92%, 93%, 94%, 95%, 96%, 97%, 98%, or 99% identity to the amino acid sequence.
42. The anti-FAP antibody or the antigen-binding fragment thereof according to any one of embodiments 34-41, comprising a light chain, wherein the light chain comprises or consists of the amino acid sequence of SEQ ID NO: 16, or an amino acid sequence having at least 85%, 90%, 91%, 92%, 93%, 94%, 95%, 96%, 97%, 98%, or 99% identity to the amino acid sequence.
43. The anti-FAP antibody or the antigen-binding fragment thereof according to embodiment 41 or 42, comprising a heavy chain and a light chain, wherein
   (i) the heavy chain comprises or consists of the amino acid sequence of SEQ ID NO: 14, or an amino acid sequence having at least 85%, 90%, 91%, 92%, 93%, 94%, 95%, 96%, 97%, 98%, or 99% identity to the amino acid sequence; and the light chain comprises or consists of the amino acid sequence of SEQ ID NO: 16, or an amino acid sequence having at least 85%, 90%, 91%, 92%, 93%, 94%, 95%, 96%, 97%, 98%, or 99% identity to the amino acid sequence;
   (ii) the heavy chain comprises or consists of the amino acid sequence of SEQ ID NO: 23, or an amino acid sequence having at least 85%, 90%, 91%, 92%, 93%, 94%, 95%, 96%, 97%, 98%, or 99% identity to the amino acid sequence; and the light chain comprises or consists of the amino acid sequence of SEQ ID NO: 16, or an amino acid sequence having at least 85%, 90%, 91%, 92%, 93%, 94%, 95%, 96%, 97%, 98%, or 99% identity to the amino acid sequence; or
   (iii) the heavy chain comprises or consists of the amino acid sequence of SEQ ID NO: 35, or an amino acid sequence having at least 85%, 90%, 91%, 92%, 93%, 94%, 95%, 96%, 97%, 98%, or 99% identity to the amino acid sequence; and the light chain comprises or consists of the amino acid sequence of SEQ ID NO: 16, or an amino acid sequence having at least 85%, 90%, 91%, 92%, 93%, 94%, 95%, 96%, 97%, 98%, or 99% identity to the amino acid sequence.
44. The anti-FAP antibody or the antigen-binding fragment thereof according to embodiment 43, comprising a heavy chain and a light chain, wherein the heavy chain and the light chain respectively comprise or consist of the amino acid sequences set forth in the following SEQ ID NOs:
   (i)SEQ ID NO: 14 and SEQ ID NO: 16;
   (ii)SEQ ID NO: 23 and SEQ ID NO: 16; or
   (iii)SEQ ID NO: 35 and SEQ ID NO: 16.
45. The fusion protein molecule according to any one of embodiments 34-44, wherein the anti-FAP antibody or the antigen-binding fragment thereof is a humanized antibody or a chimeric antibody.
46. The fusion protein molecule according to any one of embodiments 34-45, wherein the anti-FAP antibody or the antigen-binding fragment thereof is a monoclonal antibody.
47. The fusion protein molecule according to any one of embodiments 34-46, wherein the anti-FAP antibody or the antigen-binding fragment thereof is an antigen-binding fragment selected from a Fab, a Fab', a Fab'-SH, an Fv, a single-chain antibody (e.g., scFv), (Fab')₂, a single-domain antibody (e.g., VHH), a domain antibody (dAb), and a linear antibody.
48. A nucleic acid molecule, comprising or consisting of a nucleic acid sequence encoding the fusion protein molecule according to any one of embodiments 1-33 or any one of the chains of the antibody according to any one of embodiments 34-47.
49. An expression vector, comprising the nucleic acid molecule according to embodiment 48, wherein preferably, the expression vector is pCDNA, e.g., pCDNA3.1.
50. A host cell, comprising the nucleic acid molecule according to embodiment 48 or the expression vector according to embodiment 49, wherein preferably, the host cell is prokaryotic or eukaryotic, for example, a 293 cell or a CHO cell, e.g., a 293F cell, a 293T cell, or a CHO-S cell.
51. A method for preparing the fusion protein molecule according to any one of embodiments 1-33 or the antibody according to any one of embodiments 34-47, comprising: culturing a host cell comprising the nucleic acid molecule according to embodiment 48 or the expression vector according to embodiment 49 in a condition suitable for expressing a chain of the fusion protein, and optionally, recovering the fusion protein molecule or the antibody from the host cell (or the host cell culture medium).
52. A pharmaceutical composition or a medicament or a formulation, comprising the fusion protein molecule according to any one of embodiments 1-33 or the antibody according to any one of embodiments 34-47, and optionally, a pharmaceutical supplementary material.
53. A pharmaceutical combination product, comprising the fusion protein molecule according to any one of embodiments 1-33 or the antibody according to any one of embodiments 34-47, and one or more additional therapeutic agents (e.g., an antifibrotic, an additional antibody, or a small molecule drug).
54. A method for preventing or treating fibrosis (e.g., fibrosis in the lung, kidney, liver, or skin) in a subject, comprising: administering to the subject an effective amount of the fusion protein molecule according to any one of embodiments 1-33 or the antibody according to any one of embodiments 34-47, or the pharmaceutical composition or the formulation according to embodiment 52; or the pharmaceutical combination product according to embodiment 53.
55. A method for alleviating a fibrotic lesion (e.g., a fibrotic lesion in the lung, kidney, or liver, such as idiopathic pulmonary fibrosis, liver cirrhosis, scleroderma, or diabetic nephropathy) in a subject, comprising: administering to the subject an effective amount of the fusion protein molecule according to any one of embodiments 1-33 or the antibody according to any one of embodiments 34-47, or the pharmaceutical composition or the formulation according to embodiment 52; or the pharmaceutical combination product according to embodiment 53.
56. A method for inhibiting the proliferation of a fibroblast, e.g., an activated fibroblast, such as a (activated) fibroblast specifically expressing FAP, in a subject, comprising: administering to the subject an effective amount of the fusion protein molecule according to any one of embodiments 1-33 or the antibody according to any one of embodiments 34-47, or the pharmaceutical composition or the formulation according to embodiment 52; or the pharmaceutical combination product according to embodiment 53.
57. The method according to any one of embodiments 54-56, wherein FAP expression or elevated FAP expression (e.g., compared to a tissue/organ of a healthy subject or compared to a healthy tissue/organ of the same patient) is present in a tissue or organ with fibrosis of the subject.
58. The method according to embodiment 57, wherein FAP expression or elevated FAP expression (e.g., compared to a tissue/organ of a healthy subject or compared to a fibroblast in a healthy tissue/organ of the same patient) is present in a fibroblast in a tissue or organ with fibrosis of the subject.
59. The method according to any one of embodiments 54-56, wherein a FAP nucleic acid or an elevated FAP nucleic acid level (e.g., compared to a tissue/organ of a healthy subject or compared to a healthy tissue/organ of the same patient) is present in a tissue or organ with fibrosis of the subject.
60. The method according to embodiment 59, wherein a FAP nucleic acid or an elevated FAP nucleic acid level (e.g., compared to a tissue/organ of a healthy subject or compared to a fibroblast in a healthy tissue/organ of the same patient) is present in a fibroblast in a tissue or organ with fibrosis of the subject.

### BRIEF DESCRIPTION OF THE DRAWINGS

FIG. 1 shows the results of the relative binding activity assay (ELISA) of FAP antibodies to recombinant human FAP protein;
FIG. 2 shows the results of the binding assay (FACS) of FAP antibodies to 293T-cynomolgus FAP cells (2A) and 293T-human FAP cells (2B);
FIG. 3 shows the results of the binding assay of FAP antibodies and a DPP4 antibody to DPP4 cells;
FIG. 4 shows the results of the binding assay (FACS) of an optimized FAP antibody to 293T-cynomolgus FAP cells (A) and 293T-human FAP cells (B);
FIG. 5 shows the results of the binding assay (FACS) of huF19-LG-NGR-Trap to 293T-human FAP cells;
FIG. 6 shows the reporter gene activity of anti-FAP-Trap;
FIG. 7 shows the pathological section staining of the IPF model;
FIG. 8 shows the scoring of fibrosis staining (A) and fibrosis grading (B) in the IPF model;
FIG. 9 shows the pathological section staining of the UUO model;
FIG. 10 shows the fibrosis scoring and percentage in the UUO model;
FIG. 11 shows the structural schematic of the fusion protein targeting FAP and TGFβ.

### DETAILED DESCRIPTION

It will be appreciated that the present disclosure is not limited to the particular methodology, schemes, and reagents described herein, as these may vary. It will also be appreciated that the terms used herein are only intended to describe specific embodiments rather than limit the scope of the present disclosure, which will be limited only by the claims.

### I. Definitions

In order to illustrate the description, the following definitions will be used, and wherever appropriate, terms used in the singular form may also include the plural form, and vice versa. Unless otherwise defined, any technical and scientific term used herein has the same meaning as commonly understood by those of ordinary skill in the art to which the present disclosure belongs.

The term "about" used in combination with a numerical value is intended to encompass the numerical values in a range from a lower limit less than the specified numerical value by 5% to an upper limit greater than the specified numerical value by 5%.

As used herein, the term "and/or" refers to any one of the options or two or more or all of the options.

As used herein, the term "comprise" or "include" is intended to refer to the inclusion of the elements, integers, or steps, but not the exclusion of any other elements, integers, or steps. The term "comprise" or "include" used herein, unless indicated otherwise, also encompasses the situation where the entirety consists of the described elements, integers, or steps. For example, when referring to an antibody variable region "comprising" a specific sequence, it is also intended to encompass an antibody variable region consisting of the specific sequence.

When referring to "first" and "second" herein, it is only to distinguish two domains or two chains, and does not indicate the locations of the two domains in any way.

As used herein, the term "fibroblast activation protein (FAP)", unless otherwise indicated, refers to any native FAP from any vertebrate source, including mammals such as primates (e.g., human, see NM_004460.2, or cynomolgus monkey, see XM_005573320.2) and rodents (e.g., mouse). The term encompasses "full-length", unprocessed FAP, as well as any forms of FAP that result from processing in a cell. The amino acid sequence of an exemplary full-length human FAP protein can be retrieved with the accession number NM_004460.2, while the sequence of an exemplary full-length cynomolgus FAP protein can be retrieved with the accession number XM_005573320.2. The term also encompasses natural FAP variants, such as splice variants or allelic variants. Exemplary human and cynomolgus amino acid sequences are set forth in SEQ ID NO: 31 and SEQ ID NO: 32, respectively.

The terms "anti-FAP antibody", "anti-FAP", "FAP antibody", or "FAP-binding antibody" as used herein refer to an antibody capable of binding to FAP protein or a fragment thereof with sufficient affinity. In one embodiment, the anti-FAP antibody binds to a non-FAP protein to an extent of about 10%, about 20%, about 30%, about 40%, about 50%, about 60%, about 70%, about 80%, about 90%, or more, weaker than the antibody binding to FAP, as measured by radioimmunoassay (RIA), bioluminescence interferometry, or MSD assay. In some embodiments, the binding is measured by, e.g., radioimmunoassay (RIA), biolayer interferometry (BLI), bioluminescence interferometry, MSD assay or surface plasmon resonance (SPR), or flow cytometry.

The "TGFβR" or "TGFβ receptor" refers to a polypeptide having the amino acid sequence of a wild-type human TGFβ receptor, or a polypeptide having a sequence substantially identical to the wild-type amino acid sequence.

The "TGFβRII" or "TGFβ receptor II" refers to a polypeptide having the sequence of wild-type human TGFβ receptor type 2 isoform A (e.g., the amino acid sequence of NCBI reference sequence (RefSeq) accession number NP_001020018 or the amino acid sequence of Uniprot ID: P37173), or a polypeptide having the sequence of wild-type human TGFβ receptor type 2 isoform B (e.g., the amino acid sequence of NCBI RefSeq accession number NP_003233), or a polypeptide having a sequence substantially identical to the wild-type amino acid sequences. The TGFβRII may retain at least 75%, 80%, 85%, 90%, 95%, or 99% of the TGFβ-binding activity of the wild-type sequences, or a binding activity equal to or greater than those of the wild-type sequences. In one embodiment, the TGFβRII of the present disclosure comprises the amino acid sequence set forth in SEQ ID NO: 34, or an amino acid sequence having at least 85%, 86%, 87%, 88%, 89%, 90%, 91%, 92%, 93%, 94%, 95%, 96%, 97%, 98%, or 99% identity to the amino acid sequence set forth in SEQ ID NO: 34.

The "extracellular domain" or "ECD" refers to the extracellular domain of TGF βR, which is capable of binding to TGF-β and blocking its activity. In some embodiments, the ECD is determined according to Topological domain of Uniprot. In some embodiments, the ECD described herein also encompasses variant ECDs that retain the functionality of the original extracellular domain but have undergone certain modifications. In some embodiments, the modifications are a substitution or a truncation. In some embodiments, the substitution is, e.g., a substitution of G at position 20 with T, i.e., G20T; or a substitution of N at position 19 with E. In some embodiments, the truncation is an N-terminus truncation or a C-terminus truncation, by which a truncated N-terminus or a truncated C-terminus is obtained. In some embodiments, the truncation is an N-terminus truncation, e.g., a deletion of any or all of the amino acids at positions 1-19 at the N-terminus, e.g., a deletion of amino acids at positions 1-19 of the ECD, a deletion of amino acids at positions 4-19 of the ECD, or a deletion of amino acids at positions 18-19 of the ECD. In some embodiments, the ECD of the TGFβRII generally comprises or consists of the amino acid sequence set forth in SEQ ID NO: 24, 37, or 39, or an amino acid sequence having at least 85%, 86%, 87%, 88%, 89%, 90%, 91%, 92%, 93%, 94%, 95%, 96%, 97%, 98%, or 99% identity to the amino acid sequence set forth in SEQ ID NO: 24, 37, or 39.

The term "functional fragment" of a protein comprises a portion of the protein, which is a portion or a segment of the intact or complete protein that has fewer amino acid residues than that of the intact or complete protein, but still retains the desired protein functionality.

The terms "full antibody", "full-length antibody", "complete antibody", and "intact antibody" may be used interchangeably herein to refer to a naturally occurring glycoprotein comprising at least two heavy (H) chains and two light (L) chains interconnected by disulfide bonds. Each heavy chain consists of a heavy chain variable region (abbreviated herein as VH) and a heavy chain constant region. The heavy chain constant region consists of 3 domains, CH1, CH2, and CH3. Each light chain consists of a light chain variable region (abbreviated herein as VL) and a light chain constant region. The light chain constant region consists of one domain CL. The VH region and the VL region can be further divided into hypervariable regions (complementarity determining regions, or CDRs), with relatively conservative regions (framework regions, or FRs) inserted therebetween. Each VH or VL consists of three CDRs and four FRs, arranged from amino-terminus to carboxyl-terminus in the following order: FR1, CDR1, FR2, CDR2, FR3, CDR3, and FR4. The constant regions are not directly involved in the binding of antibodies to antigens, but exhibit a variety of effector functions. In some embodiments, the antibody heavy chain constant region HC of the present disclosure is a heavy chain constant region of IgG1, IgG2, IgG3, or IgG4, preferably a heavy chain constant region of IgG1. The term "antibody fragment" comprises a portion of an intact antibody. In a preferred embodiment, the antibody fragment is an antigen-binding fragment.

The term "antigen-binding fragment" is a portion or segment of an intact antibody or a complete antibody that has fewer amino acid residues than the intact antibody or the complete antibody, which can bind to an antigen or compete with an intact antibody (i.e., an intact antibody from which the antigen-binding fragment is derived) for binding to an antigen. The antigen-binding fragment may be prepared by recombinant DNA techniques, or by enzymatic or chemical cleavage of an intact antibody. The antigen-binding fragment includes, but is not limited to, a Fab, a Fab', F(ab')₂, an Fv, a single-chain Fv, a diabody, and a single-domain antibody (sdAb). The Fab fragment is a monovalent fragment consisting of VL, VH, CL, and CH1 domains and can be obtained, for example, by papain digestion of a complete antibody. In addition, the F(ab')₂, a dimer of the Fab', is a bivalent antibody fragment produced by pepsin digestion of a portion below disulfide bonds in a hinge region of a complete antibody. The F(ab')₂ can be reduced by disrupting the disulfide bonds in the hinge region in neutral conditions, and the F(ab')₂ dimer is thus converted into Fab' monomers. The Fab' monomer is substantially a Fab fragment with a hinge region (for more detailed descriptions of other antibody fragments, see Fundamental Immunology, edited by W. E. Paul, Raven Press, N.Y. (1993)). The Fv fragment consists of the VL and VH domains of a single arm of an antibody. In addition, although the two domains VL and VH of the Fv fragment are encoded by separate genes, the domains can be linked, using recombinant methods, by a synthetic linker peptide capable of making these two domains produced as a single protein chain in which the VL and VH regions are paired to form a single-chain Fv (scFv). The antibody fragment can be obtained by a chemical method, a recombinant DNA method, or a protease digestion method.

The "Fab fragment" and "Fab" can be used interchangeably herein to refer to an immunoglobulin fragment consisting of two polypeptide chains and comprising an immunoglobulin heavy chain variable domain VH, a heavy chain constant domain CH1, a light chain variable domain VL, and a light chain constant domain CL, wherein one polypeptide chain comprises, from N-terminus to C-terminus, a VH and one constant region selected from CH1 and CL, and the other polypeptide chain comprises, from N-terminus to C-terminus, a VL and another constant region selected from CL and CH1, wherein the VH and VL domains are paired to form an antigen-binding site. Herein, a Fab chain comprising a heavy chain constant region CH1 is also referred to as a "Fab heavy chain"; correspondingly, a Fab chain comprising a light chain constant region CL is also referred to as a "Fab light chain".

The term "target" refers to a bound substance against which a binding molecule (e.g., a fusion protein) is directed. The target may be an antigen, or may be a ligand or a receptor.

The term "antigen" refers to a molecule that induces an immune response. Such an immune response may involve antibody production or activation of specific immune cells, or both. Those skilled in the art will appreciate that any macromolecules, including substantially all proteins or peptides, can be used as antigens. In addition, an antigen may be derived from recombinant or genomic DNA. As used herein, the term "epitope" refers to a portion of an antigen that specifically interacts with an antibody molecule.

As used herein, the term "target-binding region" refers to a portion of a multispecific binding molecule, e.g., a fusion protein, that binds to a particular target or an antigen. The target-binding region may be, e.g., an antibody or immunoglobulin *per se* or an antibody fragment. Such target-binding region may or may not have a tertiary structure independent of the remainder of the bispecific antibody molecule, and may or may not bind to its target as a separate entity. The target-binding region may also be a receptor or a ligand, or a domain of a receptor capable of binding to a ligand. When the target-binding region is an antibody, the "target-binding region" is also referred to as the "antigen-binding region".

When referring to "a target-binding region derived from an antibody", it means that the binding domain of the target-binding region is or is derived from the binding domain of the antibody that specifically binds to an antigen; for example, the heavy chain variable region and/or the light chain variable region of the target-binding region is or is derived from the heavy chain variable region and/or the light chain variable region of the antibody; or 1, 2, 3, 4, 5, or 6 CDRs of the target-binding region are the CDRs of the antibody.

The term "derived from" means that the fragment in the target-binding region is substantially identical to the fragment of the antibody from which it is derived, but has a mutation(s), such as a substitution, a deletion, or an addition, at one or more positions. In one specific embodiment, the mutation is not in the CDR of the antibody.

The term "immunoglobulin" refers to a protein having the structure of a naturally occurring antibody. For example, an IgG immunoglobulin is a heterotetrameric glycoprotein of about 150,000 Daltons consisting of two light chains and two heavy chains which are disulfide-bonded. Each immunoglobulin heavy chain has a heavy chain variable region (VH), also known as a heavy chain variable domain, followed by three heavy chain constant domains (CH1, CH2, and CH3), from the N-terminus to the C-terminus. Similarly, each immunoglobulin light chain has a light chain variable region (VL), also known as a light chain variable domain, followed by a light chain constant domain (CL), from the N-terminus to the C-terminus. The heavy chains of an immunoglobulin can be assigned to one of five classes, α (IgA), δ (IgD), ε (IgE), γ (IgG), or µ (IgM), in which some classes can be further divided into subclasses such as γ1 (IgG1), γ2 (IgG2), γ3 (IgG3), γ4 (IgG4), α1 (IgA1), and α2 (IgA2). The light chains of an immunoglobulin can be divided into one of two classes, κ and λ, based on the amino acid sequence of constant domains thereof. The IgG immunoglobulin consists essentially of two Fab molecules and two dimerized Fc regions (Fc dimers) linked by an immunoglobulin hinge region.

The term "variable region" or "variable domain" refers to a domain of a heavy chain or light chain of an antibody involved in the binding of the antibody to an antigen. Variable regions of heavy and light chains of native antibodies generally have similar structures, wherein each domain comprises four conserved framework regions (FRs) and three complementarity determining regions.

The "complementarity determining region" or "CDR region" or "CDR" is a region in an antibody variable domain that is highly variable in sequence and forms a structurally defined loop ("hypervariable loop") and/or comprises antigen-contacting residues ("antigen contact sites"). The CDRs are primarily responsible for binding to antigenic epitopes. The CDRs of the heavy and light chains are generally referred to as CDR1, CDR2, and CDR3, and are numbered sequentially from the N-terminus. The CDRs located in the heavy chain variable domain of the antibody are referred to as HCDR1, HCDR2, and HCDR3, whereas the CDRs located in the light chain variable domain of the antibody are referred to as LCDR1, LCDR2, and LCDR3. In a given amino acid sequence of a light chain variable region or a heavy chain variable region, the exact amino acid sequence boundary of each CDR may be determined using any one or a combination of many well-known antibody CDR assignment schemes including, e.g., Chothia based on the three-dimensional structure of antibodies and the topology of the CDR loops (Chothia et al., (1989) Nature, 342: 877-883; Al-Lazikani et al., Standard conformations for the canonical structures of immunoglobulins, Journal of Molecular Biology, 273: 927-948 (1997)), Kabat based on antibody sequence variability (Kabat et al., Sequences of Proteins of Immunological Interest, 4th ed., U.S. Department of Health and Human Services, National Institutes of Health (1987)), AbM (University of Bath), Contact (University College London), International ImMunoGeneTics database (IMGT) (imgt.cines.fr/ on the World Wide Web), and North CDR definition based on the affinity propagation clustering using a large number of crystal structures.

The following are the regional ranges of the CDRs defined using the Kabat, AbM, Chothia, Contact, and IMGT schemes.

| CDR | Kabat scheme | AbM scheme | Chothia scheme | Contact scheme | IMGT scheme |
|---|---|---|---|---|---|
| LCDR1 (Kabat and Chothia numbering systems) | L24-L34 | L24-L34 | L26-L32 | L30-L36 | L27-L32 |
| LCDR2 (Kabat and Chothia numbering systems) | L50-L56 | L50-L56 | L50-L52 | L46-L55 | L50-L51 |
| LCDR3 (Kabat and Chothia numbering systems) | L89-L97 | L89-L97 | L91-L96 | L89-L96 | L89-L97 |
| HCDR1 (Kabat numbering system) | H31-H35B | H26-H35B | H26-H32...34 | H30-H35B | H26-H35B |
| HCDR1 (Chothia numbering system) | H31-H35 | H26-H35 | H26-H32 | H30-H35 | H26-H33 |
| HCDR2 (Kabat and Chothia numbering systems) | H50-H65 | H50-H58 | H52-H56 | H47-H58 | H51-H56 |
| HCDR3 (Kabat and Chothia numbering systems) | H95-H102 | H95-H102 | H96-H101 | H93-H101 | H93-H102 |

Unless otherwise stated, the term "CDR" or "CDR sequence" used herein encompasses CDR sequences determined by any one of the schemes described above. CDRs may also be determined based on having the same Kabat numbering positions as a reference CDR sequence (e.g., any one of the exemplary CDRs of the present disclosure). Unless otherwise stated, in the present disclosure, residue positions of an antibody variable region (including heavy chain variable region residues and light chain variable region residues) are determined according to the Kabat numbering system (Kabat et al., Sequences of Proteins of Immunological Interest, 5th ed., Public Health Service, National Institutes of Health, Bethesda, Md. (1991)).

In one embodiment, the HCDRs and LCDRs in the antibody of the present disclosure are determined according to the Kabat scheme.

It should be noted that the boundaries of the CDRs of the variable regions of the same antibody obtained on the basis of different assignment schemes may differ. That is, CDR sequences of the variable regions of the same antibody defined by different assignment schemes are different. Therefore, when it comes to defining an antibody with specific CDR sequences defined in the present disclosure, the scope of the antibody also encompasses such antibodies whose variable region sequences comprise the specific CDR sequences, but have claimed CDR boundaries different from the specific CDR boundaries defined by the present disclosure due to different schemes (e.g., different assignment scheme rules or their combinations) applied.

The term "Fc domain", "Fc region", or "Fc fragment" is used herein to define a C-terminal region of an immunoglobulin heavy chain, which comprises at least a portion of the constant region. The term includes Fc regions of native sequences and variant Fc regions. A native immunoglobulin "Fc domain" comprises two or three constant domains, i.e., a CH2 domain, a CH3 domain, and optionally a CH4 domain. For example, in native antibodies, an immunoglobulin Fc domain comprises the second and the third constant domains (CH2 domain and CH3 domain) derived from two heavy chains of IgG, IgA, and IgD antibodies; or comprises the second, the third, and the fourth constant domains (CH2 domain, CH3 domain, and CH4 domain) derived from two heavy chains of IgM and IgE antibodies. Unless otherwise stated herein, amino acid residues in the Fc region or the heavy chain constant region are numbered according to the EU numbering system (also known as the EU index) as described in, for example, Edelman, G.M. et al., Proc. Natl. Acad. USA, 63, 78-85 (1969) (https://pubmed.ncbi.nlm.nih.gov/5257969/), see also http://www.imgt.org/IMGTScientificChart/Numbering/Hu_IGHGnber.html. Herein, the terms "Fc domain", "Fc region", or "Fc fragment" does not comprise a heavy chain variable region VH and a light chain variable region VL as well as a heavy chain constant region CH1 and a light chain constant region CL of an immunoglobulin, but in some cases, it may comprise a hinge region at the N-terminus of the heavy chain constant region, such as EPKSS or EPKSC. In some embodiments, the heavy chain constant region Fc suitable for use in the present disclosure is from an antibody heavy chain constant region, e.g., a constant region of human IgG1, IgG2, IgG3, or IgG4, preferably from a constant region of IgG1.

Examples of "effector functions" of immunoglobulins include: C1q binding and complement-dependent cytotoxicity (CDC), Fc receptor binding, antibody-dependent cell-mediated cytotoxicity (ADCC), antibody-dependent cellular phagocytosis (ADCP), cytokine secretion, immune complex-mediated antigen uptake in antigen-presenting cells, down-regulation of cell surface receptors (e.g., B-cell receptors), and B-cell activation.

The term "chimeric antibody" is an antibody molecule in which: (a) a constant region or a portion thereof is altered, substituted, or exchanged such that antigen-binding sites are linked to constant regions of different or altered classes, effector functions, and/or species, or disparate molecules imparting new properties (e.g., enzymes, toxins, hormones, growth factors, and drugs) to chimeric antibodies, etc.; or (b) a constant region or a portion thereof is altered, substituted, or exchanged by variable regions with different or altered antigen-binding specificities. For example, a mouse antibody can be modified by substituting its constant region with a constant region from a human immunoglobulin. Due to the substitution with a human constant region, the chimeric antibody may retain its specificity for recognizing antigens, while having reduced immunogenicity in humans as compared to the original mouse antibody.

The "humanized antibody" is an antibody that retains the antigen-specific reactivity of a non-human antibody (such as a mouse monoclonal antibody) and has lower immunogenicity when administered to humans as a therapeutic agent. This can be achieved, for example, by retaining non-human antigen-binding sites and substituting the remainder of the antibodies with their human counterparts (i.e., the portions of the constant and variable regions not involved in binding are substituted with the corresponding portions of human antibodies).

As used herein, the term "anti", "binding", or "specific binding" means that the binding effect is selective for targets or antigens and may be distinguished from undesired or non-specific interactions. The ability of a binding site to bind to a particular target or an antigen may be determined by flow cytometry, enzyme-linked immunosorbent assay (ELISA), or conventional binding assays known in the art, such as radioimmunoassay (RIA), biolayer interferometry, MSD assay, or surface plasmon resonance (SPR).

The "affinity" or "binding affinity" refers to the inherent binding affinity that reflects the interaction between members of a binding pair. The affinity of molecule X for its partner Y may be generally represented by the dissociation constant (K_{D}), which is a ratio of the dissociation rate constant (K_{dis}) to the association rate constant (Kₒₙ). Affinity can be measured by common methods known in the art. One specific method for measuring affinity is the ForteBio kinetic binding assay described herein.

The "percent identity (%)" of an amino acid sequence refers to the percentage of amino acid residues in a candidate sequence that are the same as those of a specific amino acid sequence shown in this description when aligning the candidate sequence with the specific amino acid sequence shown in this description, with gaps introduced if necessary to achieve maximum percent sequence identity and without considering any conservative substitutions as part of sequence identity. In some embodiments, the present disclosure considers variants of the antibody molecule of the present disclosure that have a considerable degree of identity to the antibody molecule and the sequence thereof specifically disclosed herein. For example, the identity is at least 80%, 85%, 90%, 95%, 97%, 98%, 99%, or higher. The variants may comprise conservative modifications, or be conservatively modified variants.

For polypeptide sequences, the "conservative modifications" include substitutions of, deletions of, or additions to a polypeptide sequence but do not substantially change the desired functional activity of the polypeptide sequence. For example, conservative substitutions often result in the substitution of an amino acid with a chemically similar amino acid. Conservative substitution tables providing functionally similar amino acids are well known in the art. 8 groups comprising amino acids that are conservatively substituted with each other are listed as follows: 1) alanine (A) and glycine (G); 2) aspartic acid (D) and glutamic acid (E); 3) asparagine (N) and glutamine (Q); 4) arginine (R) and lysine (K); 5) isoleucine (I), leucine (L), methionine (M), and valine (V); 6) phenylalanine (F), tyrosine (Y), and tryptophan (W); 7) serine (S) and threonine (T); and 8) cysteine (C) and methionine (M). In some embodiments, the term "conservative sequence modification" is used to refer to an amino acid modification that does not significantly affect or change the binding characteristics for an antigen of interest of the antibody molecule or the binding protein molecule of the present disclosure comprising the amino acid sequence. For example, conservatively altered variants retain at least 80%, 85%, 90%, 95%, 98%, 99%, or higher, such as 100%-110% or higher, binding affinity for an antigen of interest relative to the parent antibody or binding protein.

The "single-chain variable fragment" or "scFv" is used herein to refer to a single-chain antibody fragment comprising a heavy chain variable domain VH and a light chain variable domain VL linked with a linker, wherein the VH and the VL are paired to form an antigen-binding site.

Herein, antibody constant regions or antibody constant domains, including CH1, CL, and Fc domains as well as CH2, CH3, and optionally CH4 domains that constitute the Fc domain, may be selected according to the expected function of the antibody molecule. For example, the constant region may be an IgA, IgD, IgE, IgG, or IgM region, particularly an immunoglobulin constant domain of human IgG, e.g., the constant domain of human IgG1, IgG2, IgG3, or IgG4, preferably the constant domain of human IgG1. As an example, a Fab fragment of the antibody may comprise CH and CL constant regions derived from IgG1. As yet another example, an Fc region of the antibody may comprise CH2 and CH3 domains derived from IgG1. The immunoglobulin constant region may have a native sequence or a variant sequence.

The term "linker" as used herein refers to any molecule that enables direct connection of different portions of a bispecific binding molecule. Examples of linkers to establish covalent linkages between different portions of a molecule include peptide linkers and non-protein polymers including, but not limited to, polyethylene glycol (PEG), polypropylene glycol, polyalkylene oxide, or copolymers of polyethylene glycol and polypropylene glycol. In some embodiments, the linker is a peptide linker (also referred to as a "linker peptide") and refers to a short amino acid sequence consisting of amino acids, such as glycine (G) and/or serine (S) and/or threonine (T) residues used alone or in combination, or a hinge region from an immunoglobulin, which is used for linking the amino acid sequence of a first portion of a binding molecule to a second portion of the binding molecule. For example, the peptide linker may link a first target-binding region of a binding molecule to a second target-binding region. For example, the peptide linker may also link one portion of an antibody to another portion of the antibody, e.g., a light chain variable region to a heavy chain variable region. Preferably, the peptide linker has a length sufficient to link two entities in a manner that maintains their conformation relative to each other without interference with the desired activities. Applicable linkers further include glycine-alanine polymers, glycine-serine polymers, alanine-serine polymers, and other flexible linkers. In one embodiment, the linker is, for example, a glycine-serine polymer, e.g., (G₄S)ₙ (SEQ ID NO: 43), wherein n = an integer from 1 to 10, e.g., 1, 2, 3, 4, 5, or 6.

In yet another embodiment, the linker peptide is a hinge region or a portion of a hinge region derived from an immunoglobulin, including a native hinge region or a portion thereof, or a mutated hinge region or a portion thereof. In one embodiment, the linker peptide is, for example, a hinge region or a portion thereof (e.g., EPKSC (SEQ ID NO: 44)) of an immunoglobulin (e.g., IgG, such as IgG1, IgG2, IgG3, or IgG4), or a mutated hinge region or a portion thereof (e.g., EPKSS (SEQ ID NO: 45)). Alternatively, the computer program simulation for three-dimensional structures of proteins and peptides or a phage display method can be used to rationally design a suitable flexible linker peptide.

The term "host cell" refers to a cell into which an exogenous polynucleotide has been introduced, including the progeny of such cells. Host cells include "transformants" and "transformed cells", which include primary transformed cells and progeny derived therefrom. Host cells are any type of cell system that may be used to produce the antibody molecule of the present disclosure, including eukaryotic cells, e.g., mammalian cells, insect cells, and yeast cells, and prokaryotic cells, e.g., *Escherichia coli* cells. Host cells include cultured cells, as well as cells within a transgenic animal, a transgenic plant, or a cultured plant tissue or animal tissue.

The term "vector" as used herein refers to a nucleic acid molecule capable of proliferating another nucleic acid to which it is linked. The term includes vectors that serve as self-replicating nucleic acid structures as well as vectors integrated into the genome of a host cell into which they have been introduced. The term "expression vector" refers to a vector comprising a recombinant polynucleotide, which comprises an expression control sequence effectively linked to a nucleotide sequence to be expressed. Expression vectors contain sufficient cis-regulatory elements for expression, and other elements for expression may be provided by a host cell or in an *in vitro* expression system. Expression vectors include all those known in the art, including cosmids, plasmids (e.g., naked or contained in liposomes), and viruses (e.g., lentiviruses, retroviruses, adenoviruses, and adeno-associated viruses) into which recombinant polynucleotides are incorporated.

The terms "individual" and "subject" are used interchangeably and refer to a mammal. The mammals include, but are not limited to, domesticated animals (e.g., cows, sheep, cats, dogs, and horses), primates (e.g., human and non-human primates such as monkeys), rabbits, and rodents (e.g., mice and rats). In particular, the individual is a human.

The term "treatment" (or "treat" or "treating") refers to slowing, interrupting, arresting, alleviating, stopping, lowering, or reversing the onset of symptoms, complications, or biochemical indications of a disease, or alleviating symptoms, or arresting or inhibiting the further progression of the disease, symptom, or disorder.

The term "prevention" (or "prevent" or "preventing") includes the inhibition of the development or progression of symptoms of a disease or disorder, or a specific disease or disorder.

The term "therapeutic agent" as described herein encompasses any substance effective in preventing or treating fibrosis, e.g., fibrotic lesions, including but not limited to antifibrotics, additional antibodies, or small molecule drugs.

The term "small molecule drug" refers to a low-molecular-weight organic compound capable of regulating biological processes. The "small molecule" is defined as a molecule with a molecular weight of less than 10 kD, usually less than 2 kD, and preferably less than 1 kD. The small molecule includes but is not limited to inorganic molecules, organic molecules, organic molecules containing inorganic components, molecules containing radioactive atoms, synthetic molecules, peptide mimetics, and antibody mimetics. As therapeutic agents, small molecules penetrate cells better, are less susceptible to degradation, and are less likely to induce an immune response compared to large molecules.

The term "effective amount" refers to an amount or dose of the antibody, fragment, composition, or combination of the present disclosure which generates expected effects in a patient in need of treatment or prevention after being administered to the patient in a single dose or multiple doses.

The term "therapeutically effective amount" refers to an amount effective to achieve a desired therapeutic result at a necessary dose for a necessary period of time. The therapeutically effective amount is also such an amount that any toxic or adverse effect of the fusion protein, the composition, or the combination is inferior to the therapeutically beneficial effect. The "therapeutically effective amount" preferably inhibits a measurable parameter or improves a measurable parameter by at least about 40%, and even more preferably by at least about 50%, 55%, 60%, 65%, 70%, 75%, 80%, 85%, 90%, or even 100%, relative to untreated subjects.

The term "prophylactically effective amount" refers to an amount effective to achieve a desired prophylactic result at a necessary dose for a necessary period of time. Generally, since a prophylactic dose is administered to a subject before or at an earlier stage of a disease, a prophylactically effective amount will be less than a therapeutically effective amount.

The term "fibrosis" is also known as fibrotic scarring, in which the connective tissue replaces a normal parenchymal tissue to an uncontrolled extent with excessive disorganized deposition of extracellular matrices. Fibrosis is generally caused by factors like chemical injury, persistent infection, autoimmune reaction, etc., and is initially a reversible process in which extracellular matrix components (e.g., collagen produced by fibroblasts) accumulate in an excessive, disorganized manner. With increasing degrees of severity, fibrosis severely affects the normal physiological functionality of tissues or organs. Such fibrosis is also known as a "fibrotic lesion" or a "fibrotic disease" of an organ or tissue. Fibrosis may result from various injuries or diseases or occur as a complication of various diseases, and may be a pathological feature of many diseases.

The term "pharmaceutical supplementary material" refers to a diluent, an adjuvant (e.g., Freund's adjuvant (complete and incomplete)), an excipient, a carrier, or a stabilizer, etc., that is administered with an active substance.

The term "pharmaceutical composition" refers to a composition that is present in a form allowing the effective biological activity of an active ingredient contained therein and does not contain additional ingredients having unacceptable toxicity to a subject to which the composition is administered.

The term "pharmaceutical combination or pharmaceutical combination product or combination product" refers to a non-fixed combination product or a fixed combination product, including but not limited to, a kit and a pharmaceutical composition. The term "non-fixed combination" means that the active ingredients (e.g., (i) the fusion protein of the present disclosure, and (ii) an additional therapeutic agent) are administered to a patient as separate entities either simultaneously without specific time limitation or sequentially at identical or different time intervals, wherein such administration provides two or more active agents at prophylactically or therapeutically effective levels in the patient. The term "fixed combination" means that two or more active agents are administered to a patient simultaneously in the form of a single entity. The dose and/or time intervals of two or more active agents are preferably selected such that the combined use of the ingredients can result in a therapeutic effect on the disease or disorder greater than that achieved by the use of any one of the ingredients alone. The ingredients may each take a separate formulation form and the formulation forms may be identical or different.

The term "combination therapy" refers to the administration of two or more therapeutic agents or modalities (e.g., radiotherapy or surgery) to treat the diseases as described herein. Such administration includes co-administration of these therapeutic agents in a substantially simultaneous manner, for example, in a single capsule with a fixed proportion of active ingredients. Alternatively, such administration includes co-administration of the active ingredients in a variety of or separate containers (e.g., tablets, capsules, powders, and liquids). The powder and/or liquid may be reconstituted or diluted to a desired dose before administration. In addition, such administration further includes using each type of the therapeutic agents at approximately the same time or in a sequential manner at different times. In any case, the therapeutic regimen will provide the beneficial effect of the pharmaceutical combination in the treatment of disorders or symptoms described herein.

The "subject/patient/individual sample" refers to a collection of cells or fluids obtained from a patient or a subject. The source of tissue or cell samples can be solid tissues, e.g., from fresh, frozen, and/or preserved organ or tissue samples or biopsy samples or puncture samples; blood or any blood component; body fluids such as tears, vitreous humors, cerebrospinal fluids, amniotic fluids, peritoneal fluids, or interstitial fluids; and cells from a subject at any time during pregnancy or development. In some embodiments, the tissue sample is a fibrotic tissue. Tissue samples may comprise compounds that are naturally not mixed with tissues, such as preservatives, anticoagulants, buffers, fixatives, nutrients, and antibiotics, etc.

### II. Fusion Protein

In some embodiments, the fusion protein molecule or the fragment thereof of the present disclosure binds to FAP (e.g., human FAP) with high affinity. In some embodiments, the fusion protein molecule or the fragment thereof of the present disclosure does not bind, or bind, with relatively low affinity, to DPP4 (e.g., human DPP4).

In some embodiments, the fusion protein molecule or the fragment thereof of the present disclosure binds to TGFβ (e.g., human TGFβ, such as human TGFβ1, TGFβ2, and/or TGFβ3) with relatively high affinity.

In some embodiments, the fusion protein molecule or the fragment thereof of the present disclosure binds to FAP (e.g., human FAP) with high affinity and to TGFβ (e.g., human TGFβ, such as human TGFβ1, TGFβ2, and/or TGFβ3) with relatively high affinity; optionally, the fusion protein of the present disclosure also binds to TGFβ (e.g., human TGFβ, such as human TGFβ1, TGFβ2, and/or TGFβ3) with lower affinity than that of binding to FAP.

In some embodiments, the fusion protein molecule or the fragment thereof of the present disclosure has binding affinity for human FAP comparable to or higher than that of known anti-FAP antibodies. In some embodiments, the fusion protein molecule or the fragment thereof of the present disclosure has binding affinity for human TGFβ higher than that of known fusion protein molecules. In some embodiments, the affinity of the fusion protein molecule or the fragment thereof is determined by bioluminescence interferometry.

In some embodiments, the fusion protein molecule or the fragment thereof of the present disclosure binds to human TGFβ1 with an equilibrium dissociation constant (K_{D}) of less than or equal to about 100 nM, 10 nM, 5 nM, 1 nM, 0.5 nM, or 0.1 nM.

In some embodiments, the fusion protein molecule or the fragment thereof of the present disclosure binds to human FAP with an equilibrium dissociation constant (K_{D}) of less than or equal to about 1 nM, 0.5 nM, 0.1 nM, 50 pM, 10 pM, 5 pM, 2 pM, or 1 pM.

In some embodiments, the molecule or the fragment thereof of the present disclosure is capable of blocking TGFβ (e.g., TGFβ1, TGFβ2, or TGFβ3) signaling.

In some embodiments, the molecule or the fragment thereof of the present disclosure is capable of targeting FAP or targeting a fibroblast, e.g., an activated fibroblast.

In some embodiments, the molecule or the fragment thereof of the present disclosure is more stable.

In some embodiments, the molecule or the fragment thereof of the present disclosure is capable of treating fibrosis, for example, inhibiting a fibrotic lesion, e.g., an activated fibroblast expressing FAP.

In some embodiments, the molecule or the fragment thereof of the present disclosure can be used to treat fibrosis. In some embodiments, the molecule or the fragment thereof of the present disclosure is capable of effectively inhibiting a fibrotic lesion.

In some embodiments, the fusion protein of the present disclosure comprises a first binding specificity for FAP and a second binding specificity for TGFβ.

Therefore, one aspect of the present disclosure relates to a fusion protein, comprising a first target-binding region and a second target-binding region, wherein the first target-binding region specifically binds to FAP, and the second target-binding region specifically binds to TGFβ.

In some embodiments, the first target-binding region and the second target-binding region are connected with a linker or directly connected without a linker.

In some embodiments, the first target-binding region is derived from an anti-FAP antibody, e.g., antibody F19 or a humanized antibody thereof (such as the humanized anti-FAP antibody described herein) or the anti-FAP antibody disclosed in WO2012020006, e.g., antibody 28H1, or antigen-binding fragments thereof.

The first target-binding region suitable for use in the fusion protein of the present disclosure may comprise or consist of the anti-FAP antibody or the antigen-binding fragment thereof of the present disclosure as long as it is capable of specifically binding to FAP, including, but not limited to, e.g., a full-length antibody, a single-chain Fv, a Fab, a Fab', (Fab)₂, a single-domain antibody, a VHH, a heavy-chain antibody, or the like specifically binding to FAP.

In some embodiments, the antigen-binding region is derived from an anti-FAP antibody, e.g., antibody F19 or a humanized antibody thereof (such as the humanized anti-FAP antibody described herein) or the anti-FAP antibody disclosed in WO2012020006, e.g., antibody 28H1.

In some embodiments, the antigen-binding region comprises 1, 2, 3, 4, 5, or 6 CDRs of an anti-FAP antibody, e.g., antibody F19 or a humanized antibody thereof (such as the humanized anti-FAP antibody described herein) or the anti-FAP antibody disclosed in WO2012020006, e.g., antibody 28H1.

In some embodiments, the antigen-binding region comprises 1, 2, and 3 heavy chain variable region CDRs (i.e, HCDR1, HCDR2, and HCDR3) of an anti-FAP antibody, e.g., antibody F19 or a humanized antibody thereof (such as the humanized anti-FAP antibody described herein) or the anti-FAP antibody disclosed in WO2012020006, e.g., antibody 28H1.

In some embodiments, the antigen-binding region comprises 1, 2, and 3 light chain variable region CDRs (i.e, LCDR1, LCDR2, and LCDR3) of an anti-FAP antibody, e.g., antibody F19 or a humanized antibody thereof (such as the humanized anti-FAP antibody described herein) or the anti-FAP antibody disclosed in WO2012020006, e.g., antibody 28H1.

In some embodiments, the antigen-binding region comprises 3 heavy chain variable region CDRs and 3 light chain variable region CDRs of an anti-FAP antibody, e.g., antibody F19 or a humanized antibody thereof (such as the humanized anti-FAP antibody described herein) or the anti-FAP antibody disclosed in WO2012020006, e.g., antibody 28H1.

In some embodiments, the antigen-binding region comprises the heavy chain variable region of an anti-FAP antibody, e.g., antibody F19 or a humanized antibody thereof (such as the humanized anti-FAP antibody described herein) or the anti-FAP antibody disclosed in WO2012020006, e.g., antibody 28H1.

In some embodiments, the antigen-binding region comprises the light chain variable region of an anti-FAP antibody, e.g., antibody F19 or a humanized antibody thereof (such as the humanized anti-FAP antibody described herein) or the anti-FAP antibody disclosed in WO2012020006, e.g., antibody 28H1.

In some embodiments, the antigen-binding region comprises the heavy and light chain variable regions of an anti-FAP antibody, e.g., antibody F19 or a humanized antibody thereof (such as the humanized anti-FAP antibody described herein) or the anti-FAP antibody disclosed in WO2012020006, e.g., antibody 28H1.

In some embodiments, the antigen-binding region comprises the heavy chain of an anti-FAP antibody, e.g., antibody F19 or a humanized antibody thereof (such as the humanized anti-FAP antibody described herein) or the anti-FAP antibody disclosed in WO2012020006, e.g., antibody 28H1.

In some embodiments, the antigen-binding region comprises the light chain of an anti-FAP antibody, e.g., antibody F19 or a humanized antibody thereof (such as the humanized anti-FAP antibody described herein) or the anti-FAP antibody disclosed in WO2012020006, e.g., antibody 28H1.

In some embodiments, the antigen-binding region comprises the heavy and light chains of an anti-FAP antibody, e.g., antibody F19 or a humanized antibody thereof (such as the humanized anti-FAP antibody described herein) or the anti-FAP antibody disclosed in WO2012020006, e.g., antibody 28H1.

The second target-binding region suitable for use in the fusion protein of the present disclosure may comprise any protein specifically binding to TGFβ or a functional fragment thereof, e.g., a TGFβ receptor, such as TGFβRII, or a functional domain thereof, such as an extracellular domain of TGFβ.

### III. Anti-FAP Antibody

In one aspect, the present disclosure provides a FAP antibody having high binding affinity for FAP, for example, higher than that of known FAP antibodies.

In some embodiments, the anti-FAP antibody or the antigen-binding fragment thereof of the present disclosure binds to FAP (e.g., human FAP or monkey FAP, such as cynomolgus FAP) with higher binding affinity. In some embodiments, the anti-FAP antibody or the antigen-binding fragment thereof of the present disclosure is capable of binding to both human FAP and monkey FAP, e.g., cynomolgus FAP.

In some embodiments, the anti-FAP antibody or the antigen-binding fragment thereof of the present disclosure binds to FAP expressed by a cell. In some embodiments, the affinity of the anti-FAP antibody for FAP expressed by a cell is determined by flow cytometry.

In some embodiments, the anti-FAP antibody or the antigen-binding fragment thereof of the present disclosure has better stability, for example, better than that of known FAP antibodies.

In some embodiments, the anti-FAP antibody or the antigen-binding fragment thereof of the present disclosure comprises 3 complementarity determining regions from a heavy chain variable region (HCDRs): HCDR1, HCDR2, and HCDR3.

In some embodiments, the anti-FAP antibody or the antigen-binding fragment thereof of the present disclosure comprises 3 complementarity determining regions from a light chain variable region (LCDRs): LCDR1, LCDR2, and LCDR3.

In some embodiments, the anti-FAP antibody or the antigen-binding fragment thereof of the present disclosure comprises 3 complementarity determining regions from a heavy chain variable region (HCDRs) and 3 complementarity determining regions from a light chain variable region (LCDRs).

In some aspects, the anti-FAP antibody or the antigen-binding fragment thereof of the present disclosure comprises a heavy chain variable region (VH). In some aspects, the anti-FAP antibody or the antigen-binding fragment thereof of the present disclosure comprises a light chain variable region (VL). In some aspects, the anti-FAP antibody or the antigen-binding fragment thereof of the present disclosure comprises a heavy chain variable region (VH) and a light chain variable region (VL). In some embodiments, the heavy chain variable region comprises 3 complementarity determining regions (CDRs) from a heavy chain variable region: HCDR1, HCDR2, and HCDR3. In some embodiments, the light chain variable region comprises 3 complementarity determining regions (CDRs) from a light chain variable region: LCDR1, LCDR2, and LCDR3.

In some embodiments, the anti-FAP antibody or the antigen-binding fragment thereof of the present disclosure further comprises an antibody heavy chain constant region CH. In some embodiments, the anti-FAP antibody or the antigen-binding fragment thereof of the present disclosure further comprises an antibody light chain constant region CL. In some embodiments, the anti-FAP antibody or the antigen-binding fragment thereof of the present disclosure further comprises a heavy chain constant region CH and a light chain constant region CL.

In some embodiments, the heavy chain variable region of the anti-FAP antibody of the present disclosure:
(i) comprises or consists of an amino acid sequence having at least 90%, 91%, 92%, 93%, 94%, 95%, 96%, 97%, 98%, or 99% identity to the amino acid sequence of SEQ ID NO: 7, 13, 21, or 30;
(ii) comprises or consists of the amino acid sequence of SEQ ID NO: 7, 13, 21, or 30; or
(iii) comprises or consists of an amino acid sequence having 1 or more (preferably no more than 10, and more preferably no more than 5, 4, 3, 2, or 1) amino acid modifications (preferably amino acid substitutions, and more preferably amino acid conservative substitutions) compared to the amino acid sequence of SEQ ID NO: 7, 13, 21, or 30, wherein preferably, the amino acid modifications do not occur in the CDRs.

In some embodiments, the light chain variable region of the anti-FAP antibody of the present disclosure:
(i) comprises or consists of an amino acid sequence having at least 90%, 91%, 92%, 93%, 94%, 95%, 96%, 97%, 98%, or 99% identity to the amino acid sequence of SEQ ID NO: 1, 15, or 27;
(ii) comprises or consists of the amino acid sequence of SEQ ID NO: 1, 15, or 27; or
(iii) comprises or consists of an amino acid sequence having 1 or more (preferably no more than 10, and more preferably no more than 5, 4, 3, 2, or 1) amino acid modifications (preferably amino acid substitutions, and more preferably amino acid conservative substitutions) compared to the amino acid sequence of SEQ ID NO: 1, 15, or 27, wherein preferably, the amino acid modifications do not occur in the CDRs.

In some embodiments, the 3 complementarity determining regions from the heavy chain variable regions (HCDRs) of the anti-FAP antibody of the present disclosure, HCDR1, HCDR2, and HCDR3, are selected from:
(i) the three complementarity determining regions HCDR1, HCDR2, and HCDR3 in the VH set forth in SEQ ID NO: 13 or 21; or
(ii) a sequence comprising at least one and no more than 5, 4, 3, 2, or 1 amino acid modifications (preferably amino acid substitutions, and more preferably conservative substitutions) in the three HCDRs relative to any sequence of (i).

For example, the CDRs are determined by the Kabat scheme.

In some embodiments, the 3 complementarity determining regions from the light chain variable regions (LCDRs) of the anti-FAP antibody of the present disclosure, LCDR1, LCDR2, and LCDR3, are selected from:
(i) the three complementarity determining regions LCDR1, LCDR2, and LCDR3 in the VL set forth in SEQ ID NO: 15; or
(ii) a sequence comprising at least one and no more than 5, 4, 3, 2, or 1 amino acid modifications (preferably amino acid substitutions, and more preferably conservative substitutions) in the three LCDRs relative to any sequence of (i).

For example, the CDRs are determined by the Kabat scheme.

In some embodiments, the anti-FAP antibody of the present disclosure comprises the 3 complementarity determining regions (HCDRs) in the heavy chain variable region consisting of the amino acid sequence of SEQ ID NO: 7, 13, 21, or 30 as well as the 3 complementarity determining regions (LCDRs) in the light chain variable region consisting of the amino acid sequence of SEQ ID NO: 1, 15, or 27.

In some embodiments, the HCDR1 comprises or consists of the amino acid sequence of SEQ ID NO: 10, or the HCDR1 comprises an amino acid sequence having one, two, or three modifications (preferably amino acid substitutions, and more preferably conservative substitutions) compared to the amino acid sequence of SEQ ID NO: 10.

In some embodiments, the HCDR2 comprises or consists of the amino acid sequence of SEQ ID NO: 11 or 22, or the HCDR2 comprises an amino acid sequence having one, two, or three modifications (preferably amino acid substitutions, and more preferably conservative substitutions) compared to the amino acid sequence of SEQ ID NO: 11 or 22.

In some embodiments, the HCDR3 comprises or consists of the amino acid sequence of SEQ ID NO: 12, or the HCDR3 comprises an amino acid sequence having one, two, or three modifications (preferably amino acid substitutions, and more preferably conservative substitutions) compared to the amino acid sequence of SEQ ID NO: 12.

In some embodiments, the LCDR1 comprises or consists of the amino acid sequence of SEQ ID NO: 4, or the LCDR1 comprises an amino acid sequence having one, two, or three modifications (preferably amino acid substitutions, and more preferably conservative substitutions) compared to the amino acid sequence of SEQ ID NO: 4.

In some embodiments, the LCDR2 comprises or consists of the amino acid sequence of SEQ ID NO: 5, or the LCDR2 comprises an amino acid sequence having one, two, or three modifications (preferably amino acid substitutions, and more preferably conservative substitutions) compared to the amino acid sequence of SEQ ID NO: 5.

In some embodiments, the LCDR3 comprises or consists of the amino acid sequence of SEQ ID NO: 6, or the LCDR3 comprises an amino acid sequence having one, two, or three modifications (preferably amino acid substitutions, and more preferably conservative substitutions) compared to the amino acid sequence of SEQ ID NO: 6.

In some specific embodiments of the present disclosure, the anti-FAP antibody or the antigen-binding fragment thereof of the present disclosure comprises the HCDR1, the HCDR2, the HCDR3, the LCDR1, the LCDR2, and/or the LCDR3 as described above.

In some specific embodiments of the present disclosure, the anti-FAP antibody or the antigen-binding fragment thereof of the present disclosure comprises the HCDR1, the HCDR2, the HCDR3, the LCDR1, the LCDR2, and the LCDR3 as described above.

In some specific embodiments of the present disclosure, the anti-FAP antibody or the antigen-binding fragment thereof of the present disclosure comprises an HCDR1, an HCDR2, an HCDR3, an LCDR1, an LCDR2, and an LCDR3, wherein
the HCDR1 comprises or consists of the amino acid sequence of SEQ ID NO: 10;
the HCDR2 comprises or consists of the amino acid sequence of SEQ ID NO: 11 or 22;
the HCDR3 comprises or consists of the amino acid sequence of SEQ ID NO: 12;
the LCDR1 comprises or consists of the amino acid sequence of SEQ ID NO: 4;
the LCDR2 comprises or consists of the amino acid sequence of SEQ ID NO: 5; and
the LCDR3 comprises or consists of the amino acid sequence of SEQ ID NO: 6.

In some specific embodiments of the present disclosure, the anti-FAP antibody or the antigen-binding fragment thereof of the present disclosure comprises: the HCDR1 set forth in SEQ ID NO: 10, the HCDR2 set forth in SEQ ID NO: 11 or 22, the HCDR3 set forth in SEQ ID NO: 12; the LCDR1 set forth in SEQ ID NO: 4, the LCDR2 set forth in SEQ ID NO: 5, and the LCDR3 set forth in SEQ ID NO: 6.

In some specific embodiments of the present disclosure, the anti-FAP antibody or the antigen-binding fragment thereof of the present disclosure comprises a VH and a VL, wherein:
the VH comprises or consists of the amino acid sequence set forth in SEQ ID NO: 7, 13, 21, or 30, or an amino acid sequence having at least 90%, 91%, 92%, 93%, 94%, 95%, 96%, 97%, 98%, or 99% identity to the amino acid sequence; and the VL comprises or consists of the amino acid sequence set forth in SEQ ID NO: 1, 15, or 27, or an amino acid sequence having at least 90%, 91%, 92%, 93%, 94%, 95%, 96%, 97%, 98%, or 99% identity to the amino acid sequence.

In some specific embodiments of the present disclosure, the anti-FAP antibody or the antigen-binding fragment thereof of the present disclosure comprises a VH and a VL, wherein the VH and the VL respectively comprise or consist of the amino acid sequences set forth as follows:
SEQ ID NO: 13 and SEQ ID NO: 15;
SEQ ID NO: 22 and SEQ ID NO: 15;
SEQ ID NO: 7 and SEQ ID NO: 1; or
SEQ ID NO: 30 and SEQ ID NO: 27.

In some embodiments of the present disclosure, the anti-FAP antibody or the antigen-binding fragment thereof of the present disclosure comprises a heavy chain. In some embodiments of the present disclosure, the anti-FAP antibody or the antigen-binding fragment thereof of the present disclosure comprises a light chain. In some embodiments of the present disclosure, the anti-FAP antibody or the antigen-binding fragment thereof of the present disclosure comprises a heavy chain and a light chain.

In some specific embodiments, the heavy chain of the anti-FAP antibody comprises or consists of the amino acid sequence of SEQ ID NO: 9, 14, 23, 35, 41, or 42, or an amino acid sequence having at least 85%, 90%, 91%, 92%, 93%, 94%, 95%, 96%, 97%, 98%, or 99% identity to the amino acid sequence.

In some specific embodiments, the light chain of the anti-FAP antibody comprises or consists of the amino acid sequence of SEQ ID NO: 3, 16, or 28, or an amino acid sequence having at least 85%, 90%, 91%, 92%, 93%, 94%, 95%, 96%, 97%, 98%, or 99% identity to the amino acid sequence.

In some specific embodiments, the anti-FAP antibody comprises a heavy chain and a light chain, wherein the heavy chain comprises or consists of the amino acid sequence set forth in SEQ ID NO: 9, 14, 23, 35, 41, or 42, or an amino acid sequence having at least 85%, 90%, 91%, 92%, 93%, 94%, 95%, 96%, 97%, 98%, or 99% identity to the amino acid sequence, and the light chain comprises or consists of the amino acid sequence set forth in SEQ ID NO: 3, 16, or 28, or an amino acid sequence having at least 85%, 90%, 91%, 92%, 93%, 94%, 95%, 96%, 97%, 98%, or 99% identity to the amino acid sequence.

In some specific embodiments, the anti-FAP antibody comprises a heavy chain and a light chain, wherein the heavy chain comprises or consists of the amino acid sequence set forth in SEQ ID NO: 9, 14, 23, 35, 41, or 42, and the light chain comprises or consists of the amino acid sequence set forth in SEQ ID NO: 3, 16, or 28.

In some specific embodiments, the anti-FAP antibody comprises a heavy chain and a light chain, wherein:
(i) the heavy chain comprises or consists of the amino acid sequence of SEQ ID NO: 9, or an amino acid sequence having at least 85%, 90%, 91%, 92%, 93%, 94%, 95%, 96%, 97%, 98%, or 99% identity to the amino acid sequence; and the light chain comprises or consists of the amino acid sequence of SEQ ID NO: 3, or an amino acid sequence having at least 85%, 90%, 91%, 92%, 93%, 94%, 95%, 96%, 97%, 98%, or 99% identity to the amino acid sequence;
(ii) the heavy chain comprises or consists of the amino acid sequence of SEQ ID NO: 14, or an amino acid sequence having at least 85%, 90%, 91%, 92%, 93%, 94%, 95%, 96%, 97%, 98%, or 99% identity to the amino acid sequence; and the light chain comprises or consists of the amino acid sequence of SEQ ID NO: 16, or an amino acid sequence having at least 85%, 90%, 91%, 92%, 93%, 94%, 95%, 96%, 97%, 98%, or 99% identity to the amino acid sequence;
(iii) the heavy chain comprises or consists of the amino acid sequence of SEQ ID NO: 23, or an amino acid sequence having at least 85%, 90%, 91%, 92%, 93%, 94%, 95%, 96%, 97%, 98%, or 99% identity to the amino acid sequence; and the light chain comprises or consists of the amino acid sequence of SEQ ID NO: 16, or an amino acid sequence having at least 85%, 90%, 91%, 92%, 93%, 94%, 95%, 96%, 97%, 98%, or 99% identity to the amino acid sequence;
(iv) the heavy chain comprises or consists of the amino acid sequence of SEQ ID NO: 35, or an amino acid sequence having at least 85%, 90%, 91%, 92%, 93%, 94%, 95%, 96%, 97%, 98%, or 99% identity to the amino acid sequence; and the light chain comprises or consists of the amino acid sequence of SEQ ID NO: 16, or an amino acid sequence having at least 85%, 90%, 91%, 92%, 93%, 94%, 95%, 96%, 97%, 98%, or 99% identity to the amino acid sequence; or
(v) the heavy chain comprises or consists of the amino acid sequence of SEQ ID NO: 41 or 42, or an amino acid sequence having at least 85%, 90%, 91%, 92%, 93%, 94%, 95%, 96%, 97%, 98%, or 99% identity to the amino acid sequence; and the light chain comprises or consists of the amino acid sequence of SEQ ID NO: 28, or an amino acid sequence having at least 85%, 90%, 91%, 92%, 93%, 94%, 95%, 96%, 97%, 98%, or 99% identity to the amino acid sequence.

In some specific embodiments, the anti-FAP antibody or the antigen-binding fragment thereof comprises a heavy chain and a light chain, wherein the heavy chain and the light chain respectively comprise or consist of the amino acid sequences set forth in the following SEQ ID NOs:
(i)SEQ ID NO: 9 and SEQ ID NO: 3;
(ii)SEQ ID NO: 14 and SEQ ID NO: 16;
(iii)SEQ ID NO: 23 and SEQ ID NO: 16;
(iv)SEQ ID NO: 35 and SEQ ID NO: 16;
(v)SEQ ID NO: 41 and SEQ ID NO: 28; or
(vi)SEQ ID NO: 42 and SEQ ID NO: 28.

In one embodiment of the present disclosure, the amino acid modification of the anti-FAP antibody described herein includes amino acid substitutions, insertions, or deletions. In a preferred embodiment, the amino acid modification described in the present disclosure occurs in a region outside CDRs (e.g., in an FR). More preferably, the amino acid modification described in the present disclosure occurs in a region outside the heavy chain variable regions and/or outside the light chain variable regions. Preferably, the amino acid modification described herein is an amino acid substitution, preferably a conservative substitution.

In one embodiment of the present disclosure, the amino acid modification of the anti-FAP antibody described in the present disclosure is a mutation enhancing the stability of the antibody, e.g., a mutation at a deamidation site in, e.g., the CDRs of the anti-FAP antibody, such as the HCDR2 of the anti-FAP antibody, including a G56R substitution.

In some embodiments, the anti-FAP antibody or the antigen-binding fragment thereof of the present disclosure has one or more of the following properties:
(i) showing the same or similar binding affinity and/or specificity for FAP as the antibody of the present disclosure;
(ii) inhibiting (e.g., competitively inhibiting) the binding of the antibody of the present disclosure to FAP;
(iii) binding to the same or overlapping epitope as the antibody of the present disclosure;
(iv) competing with the antibody of the present disclosure for binding to FAP; and
(v) having one or more biological properties of the antibody of the present disclosure.

In some embodiments, the anti-FAP antibody of the present disclosure is an antibody in the form of IgG1, IgG2, IgG3, or IgG4, e.g., an antibody in the form of IgG1. In some embodiments, the light chain constant region of the anti-FAP antibody of the present disclosure is a lambda or kappa light chain constant region, e.g., a Kappa light chain constant region.

In some embodiments, the anti-FAP antibody is a monoclonal antibody.

In some embodiments, the anti-FAP antibody is humanized.

In some embodiments, the anti-FAP antibody is a chimeric antibody.

In one embodiment, the anti-FAP antibody of the present disclosure further encompasses an antibody fragment (e.g., an antigen-binding fragment) thereof, preferably an antibody fragment selected from the following: a Fab, a Fab', a Fab'-SH, an Fv, a single-chain antibody (e.g., scFv), (Fab')₂, a single-domain antibody (e.g., VHH), a domain antibody (dAb), and a linear antibody.

### IV. TGFβ Receptor or Functional Fragment Thereof

Any TGFβ receptor or functional fragment thereof known in the art is suitable for use in the fusion protein molecule of the present disclosure, so long as it is capable of specifically binding to TGFβ. In some embodiments, the TGFβ receptor is a TGFβRII molecule.

In some embodiments, the second target-binding region of the fusion protein molecule of the present disclosure comprises, or is, a TGFβ receptor. In some embodiments, the second target-binding region of the fusion protein molecule of the present disclosure comprises, or is, a TGFβRII receptor molecule.

In some embodiments, the TGFβRII molecule of the present disclosure:
(i) comprises or consists of an amino acid sequence having at least 85%, 90%, 91%, 92%, 93%, 94%, 95%, 96%, 97%, 98%, or 99% identity to an amino acid sequence selected from SEQ ID NO: 34;
(ii) comprises or consists of an amino acid sequence selected from SEQ ID NO: 34; or
(iii) comprises or consists of an amino acid sequence having 1 or more (preferably no more than 20 or 10, and more preferably no more than 5, 4, 3, 2, or 1) amino acid modifications (preferably amino acid substitutions, and more preferably amino acid conservative substitutions) compared to an amino acid sequence selected from SEQ ID NO: 34.

In some embodiments, the second target-binding region of the fusion protein molecule of the present disclosure comprises, or is, a functional fragment of the TGFβ receptor. In some embodiments, the second target-binding region of the fusion protein molecule of the present disclosure comprises, or is, a functional fragment of the TGFβRII molecule. In some embodiments, the functional fragment of the TGFβ receptor comprises, or is, an extracellular functional domain (extracellular domain, ECD) of the TGFβ receptor.

In some embodiments, the ECD is an ECD of a wild-type TGFβ receptor, e.g., an ECD of a wild-type TGFβRII molecule. In some embodiments, the ECD is a variant ECD comprising a modification relative to a wild-type ECD, e.g., a truncation or substitution. In some embodiments, the substitution is, e.g., a substitution of G at position 20 of the ECD with T, i.e., G20T; or a substitution of N at position 19 of the ECD with E, i.e., N19E, or a combination of the two. In some embodiments, the truncation is an N-terminus truncation or a C-terminus truncation, by which a truncated N-terminus or a truncated C-terminus is obtained. In some embodiments, the truncation is an N-terminus truncation, e.g., a deletion of any or all of the amino acids at positions 1-19 at the N-terminus, e.g., a deletion of amino acids at positions 1-19 of the ECD, a deletion of amino acids at positions 4-19 of the ECD, or a deletion of amino acids at positions 18-19 of the ECD.

In some embodiments, the ECD of the TGFβRII molecule:
(i) comprises or consists of an amino acid sequence having at least 85%, 90%, 91%, 92%, 93%, 94%, 95%, 96%, 97%, 98%, or 99% identity to an amino acid sequence selected from SEQ ID NO: 24;
(ii) comprises or consists of an amino acid sequence selected from SEQ ID NO: 24;
(iii) comprises or consists of an amino acid sequence having 1 or more (preferably no more than 20 or 10, and more preferably no more than 5, 4, 3, 2, or 1) amino acid modifications (preferably amino acid substitutions, and more preferably amino acid conservative substitutions) compared to an amino acid sequence selected from SEQ ID NO: 24; or
(iv) comprises an amino acid sequence having a truncation at the N-terminus and/or C-terminus compared to any one of (i)-(iii), wherein preferably, the truncation refers to a deletion of any or all of the amino acids at positions 1-19 at the N-terminus, e.g., a deletion of amino acids at positions 1-19 of the ECD, a deletion of amino acids at positions 4-19 of the ECD, or a deletion of amino acids at positions 18-19 of the ECD.

In some embodiments, the ECD of the TGFβRII molecule of the present disclosure:
(i) comprises or consists of an amino acid sequence having at least 85%, 90%, 91%, 92%, 93%, 94%, 95%, 96%, 97%, 98%, or 99% identity to an amino acid sequence selected from SEQ ID NO: 37, wherein preferably the amino acid sequence comprises T at position 20 (i.e., comprising G20T);
(ii) comprises or consists of an amino acid sequence selected from SEQ ID NO: 37;
(iii) comprises or consists of an amino acid sequence having 1 or more (preferably no more than 20 or 10, and more preferably no more than 5, 4, 3, 2, or 1) amino acid modifications (preferably amino acid substitutions, and more preferably amino acid conservative substitutions) compared to an amino acid sequence selected from SEQ ID NO: 37, wherein preferably the amino acid sequence comprises T at position 20 (i.e., comprising G20T); or
(iv) comprises an amino acid sequence having a truncation at the N-terminus and/or C-terminus compared to any one of (i)-(iii), wherein preferably, the truncation refers to a deletion of any or all of the amino acids at positions 1-19 at the N-terminus, e.g., a deletion of amino acids at positions 1-19 of the ECD, a deletion of amino acids at positions 4-19 of the ECD, or a deletion of amino acids at positions 18-19 of the ECD.

In some embodiments, the ECD of the TGFβRII molecule of the present disclosure:
(i) comprises or consists of an amino acid sequence having at least 85%, 90%, 91%, 92%, 93%, 94%, 95%, 96%, 97%, 98%, or 99% identity to an amino acid sequence selected from SEQ ID NO: 39, wherein preferably the amino acid sequence comprises E at position 19 (i.e., comprising N19E);
(ii) comprises or consists of an amino acid sequence selected from SEQ ID NO: 39;
(iii) comprises or consists of an amino acid sequence having 1 or more (preferably no more than 20 or 10, and more preferably no more than 5, 4, 3, 2, or 1) amino acid modifications (preferably amino acid substitutions, and more preferably amino acid conservative substitutions) compared to an amino acid sequence selected from SEQ ID NO: 39, wherein preferably the amino acid sequence comprises E at position 19 (i.e., comprising N19E); or
(iv) comprises an amino acid sequence having a truncation at the N-terminus and/or C-terminus compared to any one of (i)-(iii), wherein preferably, the truncation refers to a deletion of any or all of the amino acids at positions 1-19 at the N-terminus, e.g., a deletion of amino acids at positions 1-19 of the ECD, a deletion of amino acids at positions 4-19 of the ECD, or a deletion of amino acids at positions 18-19 of the ECD.

### V. Exemplary Fusion Protein

In some embodiments, the fusion protein molecule of the present disclosure comprises:
(i) a first target-binding region specifically binding to FAP, comprising an anti-FAP antibody or an antigen-binding fragment thereof; and
(ii) a second target-binding region specifically binding to TGFβ, comprising a TGFβ receptor or a functional fragment thereof, wherein for example, the TGFβ receptor is a TGFβRII molecule; for example, the functional fragment is an ECD.

In some embodiments, (i) and (ii) are connected with a linker. In some embodiments, (i) and (ii) are directly connected without a linker.

In some embodiments, the first target-binding region comprises an Fc region. The Fc region fragment suitable for use in the fusion protein of the present disclosure may be any antibody Fc region. The Fc region may include a native Fc region and a variant Fc region. The native sequence Fc domain encompasses naturally occurring Fc sequences of various immunoglobulins, such as Fc regions of various Ig subtypes and allotypes thereof (Gestur Vidarsson et al., IgG subclasses and allotypes: from structure to effector functions, 20 October 2014, doi: 10.3389/fimmu.2014.00520). For example, the Fc region of the antibody of the present disclosure may comprise two or three constant domains, i.e., a CH2 domain, a CH3 domain, and optionally a CH4 domain. In some embodiments, the antibody Fc region may also bear an IgG hinge region or a portion of the IgG hinge region, e.g., an IgG1 hinge region or a portion of the IgG1 hinge region, at the N-terminus.

The hinge region may comprise a mutation(s). In some embodiments, the hinge region may be EPKSS or EPKSC. Preferably, the Fc region of the antibody of the present disclosure comprises CH2-CH3 from the N-terminus to the C-terminus, or comprises hinge region-CH2-CH3 from the N-terminus to the C-terminus. In some embodiments, the Fc region suitable for use in the fusion protein molecule of the present disclosure is a human IgG Fc, e.g., a human IgG1 Fc, a human IgG2 Fc, a human IgG3, or a human IgG4 Fc. In one embodiment, the Fc region comprises or consists of the amino acid sequence of SEQ ID NO: 40, or an amino acid sequence having at least 90% identity, e.g., 95%, 96%, 97%, 99%, or higher identity, to the amino acid sequence.

The Fc region in the antibody or the fusion protein of the present disclosure may be mutated to acquire desired properties. In some embodiments, the lysine at the C-terminus of the Fc region is substituted, e.g., with alanine, to prevent cleavage of the fusion protein.

In one embodiment, the first target-binding region is a full-length antibody and one or more second target-binding regions are separately connected (with or without a linker) to the N-terminus and/or the C-terminus of the heavy chain and/or the light chain of the full-length antibody.

In one embodiment, the first target-binding region is a full-length antibody and one or more second target-binding regions are separately connected (with or without a linker) to the N-terminus and/or the C-terminus of the heavy chain of the full-length antibody.

In one embodiment, the first target-binding region is a full-length antibody and one or more second target-binding regions are separately connected (with or without a linker) to the C-terminus of the heavy chain of the full-length antibody.

In one embodiment, the second target-binding region comprises, or is, a functional fragment of a TGFβ receptor (e.g., a TGFβRII molecule), such as an ECD.

In one specific embodiment, the fusion protein molecule of the present disclosure comprises:
(i) chain A: an anti-FAP antibody light chain; and
(ii) chain B: an anti-FAP antibody heavy chain connected, at the N-terminus or C-terminus, to an ECD of a TGFβ receptor (e.g., a TGFβRII molecule) with or without a linker.

In one embodiment, the fusion protein molecule of the present disclosure consists of 2 chains A and 2 chains B.

In the embodiments of the present disclosure, the anti-FAP antibody light chain in chain A is as defined above. In some embodiments of the present disclosure, the anti-FAP antibody heavy chain in chain B is as defined above. In some embodiments of the present disclosure, the ECD of the TGFβRII receptor (e.g., a TGFβRII molecule) is as defined above.

In some specific embodiments of the present disclosure, the chain A of the fusion protein molecule of the present disclosure:
comprises or consists of the amino acid sequence set forth in SEQ ID NO: 16, or an amino acid sequence having at least 85%, 90%, 91%, 92%, 93%, 94%, 95%, 96%, 97%, 98%, or 99% identity to the amino acid sequence.

In some specific embodiments of the present disclosure, the chain B of the fusion protein molecule of the present disclosure: comprises or consists of the amino acid sequence set forth in SEQ ID NO: 25, 36, or 38, or an amino acid sequence having at least 85%, 90%, 91%, 92%, 93%, 94%, 95%, 96%, 97%, 98%, or 99% identity to the amino acid sequence.

In some specific embodiments of the present disclosure, the chain A of the fusion protein molecule of the present disclosure:
comprises or consists of the amino acid sequence set forth in SEQ ID NO: 28, or an amino acid sequence having at least 85%, 90%, 91%, 92%, 93%, 94%, 95%, 96%, 97%, 98%, or 99% identity to the amino acid sequence.

In some specific embodiments of the present disclosure, the chain B of the fusion protein molecule of the present disclosure:
comprises or consists of the amino acid sequence set forth in SEQ ID NO: 29, or an amino acid sequence having at least 85%, 90%, 91%, 92%, 93%, 94%, 95%, 96%, 97%, 98%, or 99% identity to the amino acid sequence.

### V. Nucleic Acid of Present Disclosure and Host Cell Comprising Same

In one aspect, the present disclosure provides a nucleic acid encoding any of the above antibodies or fusion protein molecules. For example, the nucleic acid of the present disclosure comprises a nucleic acid encoding the amino acid sequence set forth in SEQ ID NO: 13, 14, 15, 16, 21, 23, 25, 29, 35, 36, or 38, or a nucleic acid encoding an amino acid having at least 85%, 90%, 91%, 92%, 93%, 94%, 95%, 96%, 97%, 98%, or 99% identity to the amino acid sequence set forth in SEQ ID NO: 13, 14, 15, 16, 21, 23, 25, 29, 35, 36, or 38.

As will be appreciated by those skilled in the art, each antibody or polypeptide amino acid sequence may be encoded by a variety of nucleic acid sequences because of codon degeneracy. Nucleic acid sequences encoding the molecules of the present disclosure may be generated using methods well known in the art, e.g., *de novo* solid-phase DNA synthesis, or PCR amplification.

In one aspect, the present disclosure provides a nucleic acid encoding any of the above antibodies or any of the above antibody chains. When expressed in a suitable expression vector, a polypeptide encoded by the nucleic acid is capable of exhibiting binding capacity to human (and/or monkey (e.g., cynomolgus monkey)) FAP antigen.

In yet another aspect, the present disclosure provides a nucleic acid encoding any of the above fusion proteins. When expressed in a suitable expression vector, a polypeptide encoded by the nucleic acid is capable of exhibiting binding capacity to human or monkey (e.g., cynomolgus monkey) FAP antigen. In one embodiment, the nucleic acids encoding the chains of the fusion protein may be in the same vector or in different vectors. In yet another embodiment, the nucleic acids encoding the chains of the fusion protein may be introduced into the same or different host cells for expression. Thus, in some embodiments, the method for producing the fusion protein of the present disclosure comprises: culturing a host cell comprising nucleic acids encoding the chains of the molecule in a condition suitable for expressing the chains to generate the fusion protein of the present disclosure.

In one embodiment, provided is a vector comprising the nucleic acid. In one embodiment, the vector is an expression vector, e.g., a eukaryotic expression vector. The vector includes, but is not limited to, a virus, a plasmid, a cosmid, a λ phage, or a yeast artificial chromosome (YAC). In one embodiment, the vector is, for example, a pcDNA vector, such as pcDNA3.1.

In one embodiment, provided is a host cell comprising the nucleic acid or the vector, e.g., for cloning or expressing a vector encoding an anti-FAP antibody or a fusion protein. In one embodiment, the host cell is eukaryotic. In another embodiment, the host cell is selected from a yeast cell, a mammalian cell (e.g., a CHO cell (e.g., CHO-S, such as ExpiCHO-S) or 293 cell (e.g., 293F or HEK293 cell)), and other cells suitable for preparing an antibody or a fragment thereof. In one embodiment, the host cell is prokaryotic, e.g., a bacterium, such as *Escherichia coli.*

In one embodiment, the host cell is eukaryotic. In another embodiment, the host cell is selected from a yeast cell, a mammalian cell, and other cells suitable for preparing an antibody or a fragment thereof. For example, eukaryotic microorganisms, such as filamentous fungi or yeast, are suitable cloning or expression hosts for the vector encoding the antibody. For example, fungus and yeast strains in which a glycosylation pathway has been "humanized" produce antibodies having a partial or full human glycosylation pattern. Host cells suitable for expressing a glycosylated antibody are also derived from multicellular organisms (invertebrates and vertebrates). Vertebrate cells may also be used as hosts. For example, a mammalian cell line engineered to be suitable for suspension growth may be used. Other examples of useful mammalian host cell lines are monkey kidney CV1 line (COS-7) transformed with SV40, human embryonic kidney line (HEK293, 293F, or 293T cells), and the like. Other useful mammalian host cell lines include Chinese hamster ovary (CHO) cells, including DHFR-CHO cells, CHO-S cells, ExpiCHO, and the like; and myeloma cell lines such as Y0, NS0, and Sp2/0. Mammalian host cell lines suitable for producing antibodies are known in the art.

### VI. Production and purification of antibody or fusion protein molecules of the present disclosure

In one embodiment, the present disclosure provides a method for preparing the antibody, the fusion protein molecule, or the fragment thereof (preferably the antigen-binding fragment) of the present disclosure, comprising: culturing the host cell in a condition suitable for expressing a nucleic acid encoding the antibody, the fusion protein molecule, or the fragment thereof of the present disclosure, and optionally, isolating the antibody, the fusion protein, or the fragment thereof. In a certain embodiment, the method further comprises recovering the antibody, the fusion protein molecule, or the fragment thereof of the present disclosure from the host cell.

In one embodiment, provided is a method for preparing the antibody or the fusion protein molecule of the present disclosure, comprising: culturing a host cell comprising a nucleic acid encoding chains of the antibody or the fusion protein molecule such as chains A and B (optionally in one vector or two vectors) or an expression vector comprising the nucleic acid, as provided above, in a condition suitable for expressing the antibody or the fusion protein, and optionally recovering the antibody or the fusion protein molecule from the host cell (or the host cell culture medium).

For recombinant production of the antibody or the fusion protein molecule of the present disclosure, a nucleic acid encoding the chains of the antibody or the fusion protein is isolated and inserted into one or more vectors for further cloning and/or expression in a host cell. Methods known to those skilled in the art can be used to construct expression vectors. Once the expression vector comprising one or more nucleic acid molecules of the present disclosure has been prepared for expression, the expression vector may be transfected or introduced into suitable host cells. Various techniques may be used for this purpose, for example, protoplast fusion, calcium phosphate precipitation, electroporation, retroviral transduction, viral transfection, biolistics, liposome-based transfection, or other conventional techniques.

The antibody or the fusion protein molecule prepared as described herein may be purified by known techniques such as high-performance liquid chromatography, ion exchange chromatography, gel electrophoresis, affinity chromatography, and size exclusion chromatography. The actual conditions used for purifying a particular protein also depend on factors such as net charge, hydrophobicity, and hydrophilicity, and these will be apparent to those skilled in the art. The purity of the antibody molecule of the present disclosure may be determined by any one of a variety of well-known analytical methods including size exclusion chromatography, gel electrophoresis, high performance liquid chromatography, and the like.

### VII. Assay

The antibody or the fusion protein provided herein may be identified, screened, or characterized for its physical/chemical properties and/or biological activities through a variety of assays known in the art. In one aspect, the antibody or the fusion protein of the present disclosure is tested for its FAP- and/or TGFβ-binding activity, e.g., by known methods such as bioluminescence interferometry and the like.

The present disclosure further provides an assay for identifying an antibody or a fusion protein having a biological activity. The biological activity may include, for example, binding to FAP or TGFβ (e.g., binding to human FAP or TGFβ), blocking the TGFβ (e.g., TGFβ1, 2, or 3) signaling, reversing the inhibition of T cells by TGFβ (e.g., TGFβ1, 2, or 3), and/or activating T cells, the inhibition of fibroblasts or fibrotic lesions, and the like.

In some certain embodiments, the antibody or the fusion protein of the present disclosure is tested for such biological activities.

The cells for use in any of the above *in-vitro* assays include cells naturally expressing FAP or TGFβ or those engineered to express or overexpress FAP or TGFβ. Such cells also include cells transfected with a DNA encoding FAP or TGFβ that express FAP or TGFβ or that express FAP or TGFβ in an abnormal condition.

### VIII. Pharmaceutical composition and formulation

In some embodiments, the present disclosure provides a composition comprising any of the antibody, the fusion protein molecule, or the fragment thereof described herein, preferably the composition is a pharmaceutical composition. In one embodiment, the composition further comprises a pharmaceutical supplementary material. In one embodiment, the composition, e.g., the pharmaceutical composition, comprises the antibody, the fusion protein molecule, or the fragment thereof of the present disclosure, and a combination of one or more additional therapeutic agents.

The present disclosure also includes compositions (including pharmaceutical compositions or pharmaceutical formulations) comprising the antibody, the fusion protein molecule, or the fragment thereof described herein, or compositions (including pharmaceutical compositions or pharmaceutical formulations) comprising a polynucleotide encoding the antibody, the fusion protein molecule, or the fragment thereof described herein. Such compositions may also comprise a suitable pharmaceutical supplementary material, such as a pharmaceutical carrier, a pharmaceutical excipient, including a buffer, known in the art.

As used herein, the "pharmaceutical carrier" includes any and all solvents, dispersion media, isotonic agents and absorption delaying agents, and the like that are physiologically compatible.

For use and application of the pharmaceutical supplementary materials, see Handbook of Pharmaceutical Excipients, 8th ed., R. C. Rowe, P. J. Seskey and S. C. Owen, Pharmaceutical Press, London, Chicago.

The composition of the present disclosure may be in a variety of forms. These forms include, for example, liquid, semi-solid, and solid dosage forms, such as liquid solutions (e.g., solutions for injection and solutions for infusion), pulvis or suspensions, liposomes, and suppositories. The preferred form depends on the intended mode of administration and therapeutic use.

The pharmaceutical formulation comprising the antibody described herein may be prepared by mixing the antibody or the fusion protein molecule of the present disclosure of a desired purity with optionally one or more pharmaceutical supplementary materials, preferably in the form of a lyophilized formulation or an aqueous solution.

The pharmaceutical composition or the formulation of the present disclosure may further comprise more than one active ingredient required by a particular indication treated, preferably those having complementarity activity without adversely affecting one another. For example, it may be desirable to also provide an additional therapeutic agent, such as an antifibrotic, a chemotherapeutic agent, a cytokine, a cytotoxic agent, an additional antibody, a small molecule drug, or the like. The active ingredients are properly present in combination in amounts effective for the intended use.

### IX. Pharmaceutical combination and kit

In some embodiments, the present disclosure further provides a pharmaceutical combination or pharmaceutical combination product comprising the antibody, the fusion protein molecule, or the fragment thereof of the present disclosure, and one or more additional therapeutic agents (e.g., an antifibrotic, a chemotherapeutic agent, a cytokine, a cytotoxic agent, an additional antibody, a small molecule drug, or the like).

The present disclosure is also intended to provide a kit of parts comprising the pharmaceutical combination of the present disclosure, preferably in the form of pharmaceutical dose units. The dose units may thus be provided according to the dose regimen or the interval of administration.

In one embodiment, the kit of parts according to the present disclosure comprises, in one package:
- a first container, containing a pharmaceutical composition comprising the antibody, the fusion protein molecule, or the fragment thereof described herein;
- a second container, containing a pharmaceutical composition comprising an additional therapeutic agent.

### X. Therapeutic use

In one aspect, the present disclosure provides a method for preventing or treating fibrosis (e.g., a fibrotic lesion) in a subject, comprising: administering to the subject an effective amount of the antibody, the fusion protein molecule or the fragment thereof, the pharmaceutical composition, the pharmaceutical combination, or the kit of the present disclosure.

In some embodiments, FAP (for example, at an elevated level, e.g., a nucleic acid or protein level) is present in the patient or a tissue/organ of the patient, for example, as compared to a tissue/organ of a healthy subject or a healthy tissue/organ of the same patient.

In one specific embodiment, the antibody, the fusion protein molecule, or the fragment thereof of the present disclosure is capable of inhibiting the proliferation of a fibroblast, e.g., an activated fibroblast, such as a (activated) fibroblast specifically expressing FAP.

In some embodiments, FAP (for example, at an elevated level, e.g., a nucleic acid or protein level) is present in a fibroblast of the patient or a tissue/organ of the patient, for example, as compared to a fibroblast of a tissue/organ of a healthy subject or a healthy tissue/organ of the same patient.

In one specific embodiment, the antibody, the fusion protein molecule, or the fragment thereof of the present disclosure is capable of reducing fibrosis, e.g., a fibrotic lesion. In some embodiments, the fibrosis is selected from a fibrosis in the lung, kidney, liver, or skin. In some embodiments, the fibrotic lesion is selected from a fibrotic lesion in the lung, kidney, or liver. In some embodiments, the fibrotic lesion is selected from idiopathic pulmonary fibrosis, liver cirrhosis, scleroderma, and diabetic nephropathy.

The subject may be a mammal, e.g., a primate, preferably a higher primate, such as a human (e.g., an individual suffering from or at risk of suffering from the disease described herein). In one embodiment, the subject is suffering from or at risk of suffering from the disease described herein. In some certain embodiments, the subject is or has been treated with other therapies, such as antifibrotic therapies.

In other aspects, the present disclosure provides use of an antibody, a fusion protein molecule or a fragment thereof, a pharmaceutical composition, a pharmaceutical combination, or a kit in producing or preparing a medicament for the use described herein, e.g., for use in preventing or treating the related disease or disorder mentioned herein.

In some embodiments, the antibody, the fusion protein molecule or the fragment thereof, the pharmaceutical composition, the pharmaceutical combination, or the kit of the present disclosure may delay the onset of a disorder and/or a symptom associated with the disorder.

In some embodiments, the antibody, the fusion protein molecule or the fragment thereof, or the pharmaceutical composition of the present disclosure may also be administered in combination with one or more additional therapies, e.g., therapeutic modalities and/or additional therapeutic agents, for the use described herein, e.g., for use in preventing and/or treating the related disease or disorder mentioned herein.

In some embodiments, the therapeutic agent is selected from an antifibrotic, an additional antibody, and a small molecule drug.

Such combination therapies encompass co-administration (for example, two or more therapeutic agents are contained in the same formulation or separate formulations) and separate administration, in which case the administration of the antibody, the fusion protein molecule, or the fragment thereof of the present disclosure may occur prior to, concurrently with, and/or subsequent to the administration of the additional therapeutic agent and/or agent.

The route of administration of the pharmaceutical composition is in accordance with known methods, e.g., oral, intravenous, intraperitoneal, intracerebral (intraparenchymal), intracerebroventricular, intramuscular, intraocular, intraarterial, intraportal, or intralesional route; by a sustained release system or by an implanted device. In some certain embodiments, the composition may be administered by bolus injection, by continuous infusion, or by an implanted device.

These and other aspects and embodiments of the present disclosure are described in the drawings (the description of the drawings follows) and in the following detailed description of the present disclosure and are illustrated in the following examples. Any or all of the features described above and throughout the present application may be combined in various embodiments of the present disclosure. The following examples are further illustrative of the present disclosure. However, it will be appreciated that the examples are described by way of illustration rather than limitation, and that various modifications can be made by those skilled in the art.

### Examples

### Example 1: FAP antibody humanization

### 1.1 Preparation of FAP antibody humanization

Antibody F19 is a murine antibody that specifically binds to FAP and has species cross-activity with human and monkey (WO1999057151A2). This example describes the humanization of antibody F19 as a candidate molecule.

The murine antibody F19 was humanized using CDR grafting technology. Briefly, the F19 VH (SEQ ID NO: 7) and F19 VL (SEQ ID NO: 1) sequences described above were searched and aligned in the IMGT database, thereby giving human germline gene sequences IGHV1-8*02 and IGKV1-16*01 having high homology to the heavy chain variable region and the light chain variable region of F19, respectively. The sequences were used as the frameworks of humanized antibody variable regions; the CDRs of the murine antibody F19 were grafted into the corresponding heavy and light chain variable region frameworks of the humanized antibody to give humanized anti-FAP antibody variable regions. In order to maintain the affinity of the murine antibody for FAP, the humanized antibody variable regions obtained were back-mutated.

Thus, the humanized heavy chain variable region sequence (huF19-LG-VH; SEQ ID NO: 13) and light chain variable region sequence (huF19-LG-VL; SEQ ID NO: 15) of the FAP antibody were obtained, as shown in Table 1, where the back-mutation sites of the humanized sequences are also listed.

**Table 1. Heavy and light chain variable region sequences of humanized anti-FAP antibody huF19-LG**

| Name | Amino acid sequence | Back-mutation site |
|---|---|---|
| huF19-LG-VH | SEQ ID NO: 13 | M48I, V67A, R71V, N72G, Y91F |
| huF19-LG-VL | SEQ ID NO:15 | S46L, Y49F, S63T, S67F, T98F |

The variable region sequences of the humanized reference antibody (sibrotuzumab) are seen in Patent No. WO9957151, where the heavy chain variable region sequence is the amino acid sequence set forth in SEQ ID NO: 17, and the light chain variable region sequence is the amino acid sequence set forth in SEQ ID NO: 19.

GenScript Biotech Co., Ltd. was commissioned for codon optimization and gene synthesis on the basis of the amino acid sequences of the variable regions of huF19-LG and the reference antibody sibrotuzumab described above. Genes encoding the VH region and VL region of the antibodies were inserted into an expression vector pcDNA3.1(+) containing the encoding gene of human IgG1 heavy chain constant region (SEQ ID NO: 8)/the encoding gene of Kappa light chain constant region (SEQ ID NO: 2), to give plasmids expressing the full-length heavy chain of anti-FAP antibody huF19-LG or sibrotuzumab and plasmids expressing the full-length light chain of anti-FAP antibody huF19-LG or sibrotuzumab. The full-length heavy and light chains of huF19-LG (heavy chain: SEQ ID NO: 14; light chain: SEQ ID NO: 16) and sibrotuzumab (heavy chain: SEQ ID NO: 18; light chain: SEQ ID NO: 20) are shown in the sequence listing.

The plasmids encoding the full-length heavy chain sequence and the plasmids encoding the full-length light chain sequence obtained as described above were co-transfected into the ExpiCHO-S cells using the ExpiCHO^{™} expression system (ThermoFisher, Cat# A29133) to express the full-length humanized anti-FAP antibody huF19-LG and the reference antibody sibrotuzumab, according to the manufacturer's product instructions. The cells were cultured for 10-12 days after transfection. When the cell viability decreased to 60%-70%, the supernatant was collected, and the antibody expressed in the supernatant was purified using a MabSelect Sure protein A affinity chromatography system (GE healthcare).

The purified antibodies were concentrated, and sterile filtration was performed. The purity of the antibodies was determined by size exclusion chromatography (SEC). Specifically, the samples were loaded at 20 µg onto a TSK G3000SWXL column using 100 mM sodium phosphate + 100 mM Na₂SO₄ (pH 7.0) as a running buffer for 30 min. The collected effluent was measured using the Agilent 1220 HPLC system and the data were analyzed using OpenLAB software. The purity was greater than 90% for both the humanized anti-FAP antibody huF19-LG and the reference antibody sibrotuzumab, suggesting high purity and integrity and suitability for subsequent experiments.

### 1.2 Activity assay of humanized FAP antibody by ELISA

The relative binding activity of the antibody to recombinant human FAP protein was determined by ELISA.

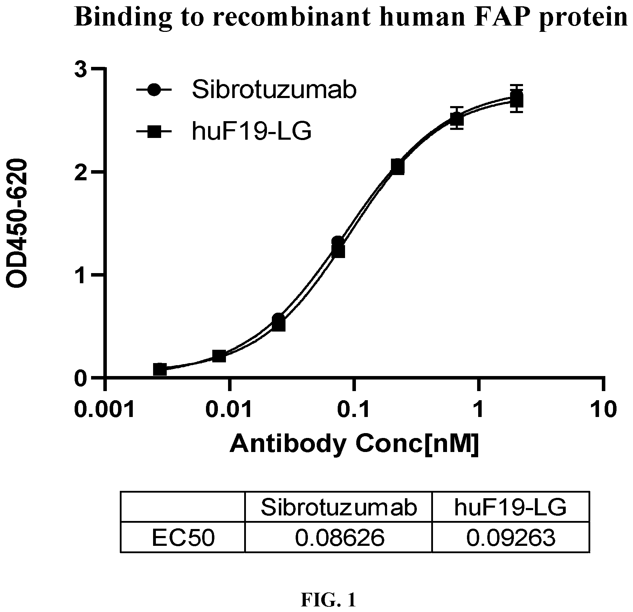

Human FAP protein (Sino Biological, Cat# 10464-H07H) was immobilized on a 96-well plate by incubation overnight in a PBS buffer at 4 °C. The plate was then blocked by incubation with a PBS containing 1% of BSA for 1 h at 37 °C. After blocking, the plate was washed thrice with PBST (PBS containing 0.05% of Tween 20). The anti-FAP antibodies (huF19-LG and sibrotuzumab) were serially three-fold diluted from a starting concentration of 2 nM in a binding buffer (PBS containing 0.05% of Tween 20 and 0.5% of BSA) and co-incubated with the immobilized protein for 1 h at 37 °C. After binding, the plate was washed thrice with PBST, incubated at 37 °C for 1 h with peroxidase-labeled goat anti-human IgG, F(ab')₂ fragment specific (Jackson Immuno Research, Cat# 109-035-097) diluted at 1/15,000 in the binding buffer, washed again, subjected to a chromogenic reaction with TMB, and quenched with 1 M H₂SO₄. The EC₅₀ (nM) and representative binding curves for the binding of the antibodies to human FAP are shown in FIG. 1.

The results indicate that humanized antibody huF19-LG specifically binds to human FAP and exhibits binding capacity comparable to the reference antibody sibrotuzumab.

### 1.3 Cell binding assay of humanized FAP antibody by flow cytometry

Whether the humanized antibody of the present disclosure can bind to human FAP protein stably expressed on the surface of 293T cells was determined by a cell binding assay. Firstly, the sequences encoding human FAP protein (NM_004460.2, SEQ ID NO: 31) and cynomolgus FAP (XM_005573320.2, SEQ ID NO: 32) were cloned into expression vector PIRES-puro3 (Clontech, Cat# 631619) by using the Lipofectamine^{™} 2000 (Invitrogen, Cat# 11668019) transfection reagent. The vectors were used to transfect 293T cells. 293T cells highly expressing human FAP (293T-human FAP) or the 293T cells highly expressing cynomolgus FAP (293T-cynomolgus FAP) were obtained by screening with 0.3 µg/mL puromycin (Gibco, Cat# A1113802).

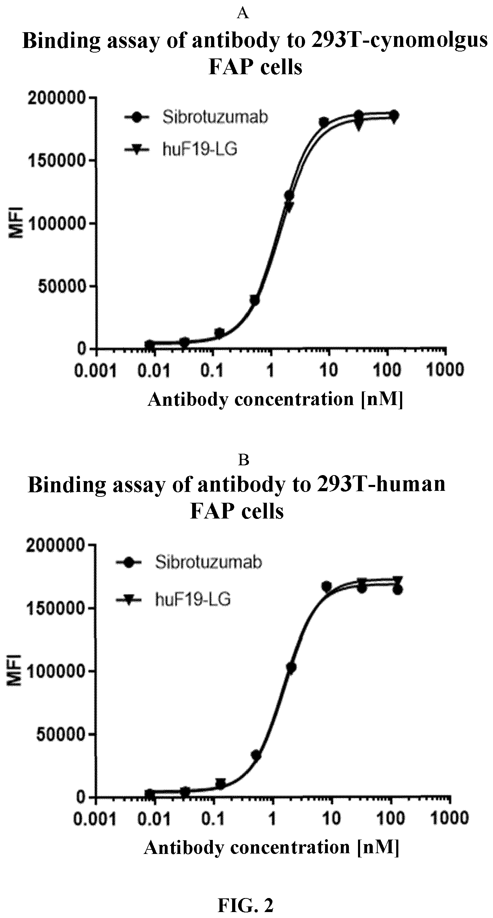

The human FAP antibodies (huF19-LG and sibrotuzumab) were 4-fold serially diluted from a starting concentration of 200 nM to a total of 8 concentrations and added to the cells for co-incubation at 4 °C for 30 min. The cells were washed twice with PBS, and then fluorescent secondary antibody R-PE-conjugated AffiniPure Goat Anti-Human IgG, Fcγ Fragment Specific (Jackson Immuno Research, Cat# 109-116-098) was added. The cells were incubated at 4 °C for 30 min. The cells were washed twice with PBS and then resuspended, and finally, fluorescence signals were detected using the BD Accuri C5 (BD Bioscience) flow cytometer. The results are shown in FIG. 2.

The results shown in FIG. 2 suggest that huF19-LG can bind to human FAP protein and cynomolgus FAP protein expressed on engineered 293T cells, with the binding capacities comparable to those of the reference antibody.

### 1.4 Binding of humanized FAP antibody to DPP4

Whether the humanized antibody in the present disclosure can bind to the human DPP4 protein stably expressed on the surface of engineered 293T cells was determined by a cell binding assay.

Firstly, the sequence encoding human DPP4 protein (NM_001935.3, SEQ ID NO: 33) was cloned into expression vector PIRES-puro3 (Clontech, Cat# 631619) by using the Lipofectamine^{™} 2000 (Invitrogen, Cat# 11668019) transfection reagent. The vector was used to transfect 293T cells. 293T cells highly expressing human DPP4 (293T-DPP4 cells) were obtained by screening with 0.3 µg/mL puromycin (Gibco, Cat# A1113802).

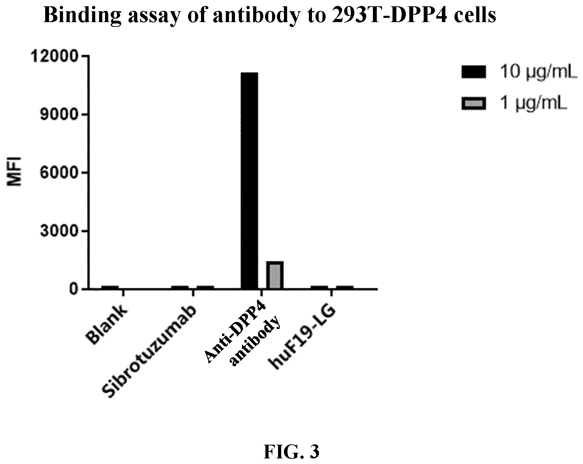

The anti-human FAP antibodies of interest (huF19-LG and Sibrotuzumab) were diluted to 10 µg/mL and 1 µg/mL with PBS, and 293T-DPP4 cells were resuspended and incubated for 30 min at 4 °C. The cells were washed twice with PBS, and then fluorescent secondary antibody R-PE-conjugated AffiniPure Goat Anti-Human IgG, Fcγ Fragment Specific (Jackson Immuno Research, Cat# 109-116-098) was added. The cells were incubated at 4 °C for 30 min. The cells were washed twice with PBS and then resuspended, and finally, fluorescence signals were detected using the BD Accuri C5 (BD Bioscience) flow cytometer. 293T-DPP4 cells were tested for the expression of DPP4 protein using FITC anti-human CD26 (DPP4; Biolegend, Cat# 302704) as the system positive control. The antibodies were diluted at 1:100 with PBS, and the cell pellet was resuspended and incubated at 4 °C for 30 min. The cells were washed twice with PBS and then resuspended in 100 µL of PBS, and finally, fluorescence signals were detected using the BD Accuri C5 (BD Bioscience) flow cytometer. The results are shown in FIG. 3.

The results shown in FIG. 3 suggest that huF19-LG does not bind to human DPP4 protein expressed on 293T cells.

### 1.5 Physicochemical analysis and stability assay of humanized anti-FAP antibody

The huF19-LG antibody was tested for stability at 40 °C. ELISA (see Example 1.2) and SEC (see Example 1.1) were conducted at 0 weeks (0W), 2 weeks (2W), or 4 weeks (4W) with the results shown as follows:

| Name | ELISA Relative activity | SEC Purity, % |
|---|---|---|
| huF19-LG-0W | 100% | 93.2 % |
| huF19-LG-2W | 93.8% | 89.5 % |
| huF19-LG-4W | 89.9% | 87.0 % |

The above results show that the purity was reduced during the storage process, and correspondingly, the activity was slightly reduced. It is supposed that the deamidation of asparagine in the CDR may contribute to the molecular instability. The position was then modified.

### Example 2: Sequence engineering of FAP antibody

### 2.1 Mutation to deamidation site

The heavy chain CDR was subjected to G56R (Kabat) mutation and the mutant full-length antibody sequence was expressed in a eukaryotic organism as in Example 1. The mutant antibody was designated as huF19-LG-NGR with the amino acid sequence of the variable region shown as follows:

| Name | Amino acid sequence | Mutation site |
|---|---|---|
| huF19-LG-NGR-VH | SEQ ID NO:21 | G56R mutation in CDR2 |
| huF19-LG-NGR-VL | SEQ ID NO:15 | |

### 2.2 Flow cytometric assay of mutant antibody molecule

Whether the mutant antibody in the present disclosure can bind to human or cynomolgus FAP protein stably expressed on the surface of 293T cells was determined by a cell binding assay. The 293T cells highly expressing human FAP or cynomolgus FAP are as described in Example 1.3. The antibodies huF19-LG-NGR and huF19-LG were 4-fold serially diluted from a starting concentration of 200 nM to a total of 8 concentrations and added to the cells for co-incubation at 4 °C for 30 min. The cells were washed twice with PBS, and then fluorescent secondary antibody R-PE-conjugated AffiniPure Goat Anti-Human IgG, Fcγ Fragment Specific (Jackson Immuno Research, Cat# 109-116-098) was added. The cells were incubated at 4 °C for 30 min. The cells were washed twice with PBS and then resuspended, and finally, fluorescence signals were detected using the BD Accuri C5 (BD Bioscience) flow cytometer.

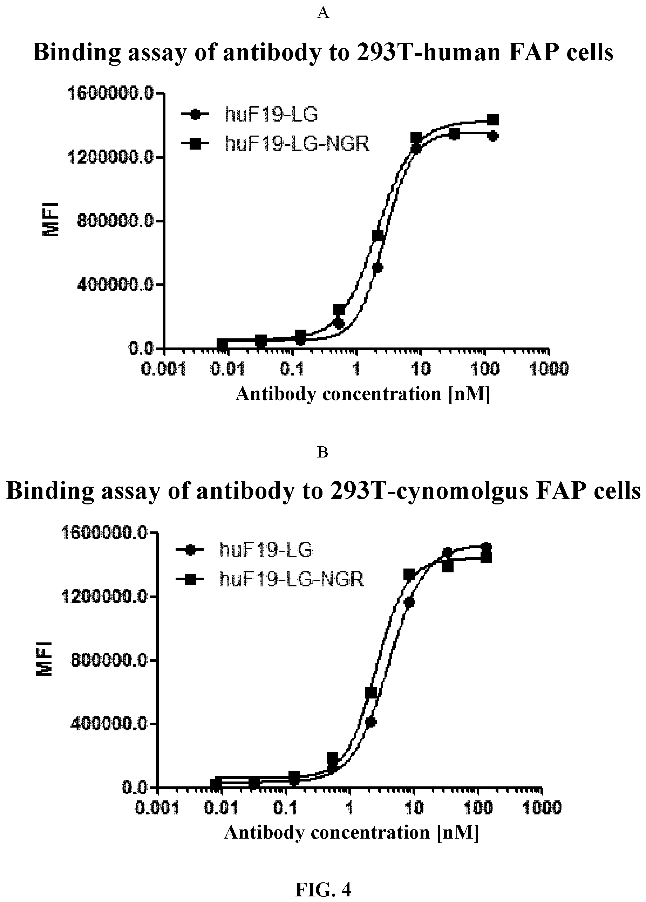

The results shown in FIG. 4 suggest that huF19-LG-NGR can bind to human FAP protein and cynomolgus FAP protein expressed on engineered 293T cells, with the binding capacities comparable to those of the antibody before mutation.

### 2.3 Storage stability assay of mutant antibody

The activity and purity of the molecule were determined by SEC after storage. See Example 1.5 for the specific procedures. The results are shown in the following table

| Name | Purity by SEC, % |
|---|---|
| huF19-LG-NGR-0W | 99.2% |
| huF19-LG-NGR-2W | 99.1% |
| huF19-LG-NGR-4W | 99.1% |

The above results show that the mutant antibody huF19-LG-NGR did not exhibit significant changes in purity, suggesting enhanced stability of the mutant antibody. huF19-LG-NGR was then used to construct antibody fusion proteins.

### Example 3: Antibody fusion proteins

### 3.1 huF19-LG-NGR-based antibody fusion protein molecules

### 3.1.1 Structural optimization and construction of molecules

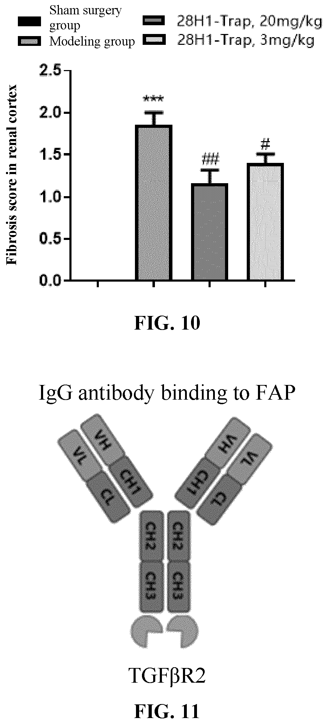

According to the present disclosure, a fusion protein LG-NGR-Trap was constructed as shown in FIG. 11. The FAP antibody moiety was derived from huF19-LG-NGR, and the Trap moieties were derived from the TGFβR2 extracellular domain (SEQ ID NO: 24, SEQ ID NO: 37 (G20T), and SEQ ID NO: 39 (N19E)). The anti-FAP antibody moiety targets FAP-expressing cells and the Trap moieties block the binding of free TGFβ molecule to TGFβR2 on cells. Also, the antibody can kill target cells by means of antibody-dependent cellular cytotoxicity (ADCC).

Antibody fusion proteins LG-NGR-Trap, LG-NGR-TrapE (N19E), and LG-NGR-TrapGT (G20T) as shown in FIG. 11 were obtained by using standard construction methods, and the amino acid sequences of the proteins are listed in the sequence listing. The sequences include, from the N terminus to the C terminus, the sequence derived from the humanized FAP antibody huF19-LG-NGR and the extracellular domain sequence of TGFβR2.

The antibody fusion protein molecules shown in FIG. 11 were constructed as follows:
LG-NGR-Trap heavy chain: The nucleotide sequence encoding the heavy chain variable region of the anti-FAP antibody was amplified and cloned into a pcDNA3.1 (+) vector containing the human IgG1 heavy chain constant region (SEQ ID NO: 8); the nucleotide sequence encoding the extracellular domain of TGF β R2 (SEQ ID NO: 24) was ligated to the C terminus of the above constant region, and the lysine at the terminus of the Fc was substituted with alanine to prevent cleavage. The expression vector of the LG-NGR-Trap heavy chain was thus obtained. The amino acid sequence of the LG-NGR-Trap heavy chain is set forth in SEQ ID NO: 25, including, from the N terminal to the C terminal: LG-NGR-VH-heavy chain constant region (terminal K-to-A mutation)-TGFβ extracellular domain.

The Trap moiety was subj ected to N19E or G20T mutation on the basis of the LG-NGR-Trap heavy chain, and a linker (G₄S)₄ was introduced during the amplification to give the LG-NGR-TrapE heavy chain (SEQ ID NO: 38 (N19E)) and the LG-NGR-TrapGT heavy chain (SEQ ID NO: 36 (G20T)).

LG-NGR-Trap light chain: the gene encoding the light chain variable region of huF19-LG was inserted into expression vector pcDNA3.1(+) containing the gene encoding the human Kappa light chain constant region (SEQ ID NO: 2) to give a plasmid of LG-NGR-Trap light chain (i.e., huF19-LG full-length light chain). The amino acid sequence of the LG-NGR-Trap light chain is set forth in SEQ ID NO: 16.

The above-described expression vectors (plasmids containing LG-NGR-Trap, LG-NGR-TrapE or LG-NGR-TrapGT heavy chain-encoding nucleic acid and huF19-LG light chain-encoding nucleic acid) were co-transfected into ExpiCHO-S cells, and antibody fusion proteins as shown in FIG. 11 were obtained by expression in a proper condition. The expression, purification, and preliminary analysis procedures were the same as those in Example 1.1.

### 3.1.2 Physicochemical and stability assay of antibody fusion protein

3.1.2.1 The purity of the obtained antibody fusion protein LG-NGR-Trap was determined by size exclusion chromatography. See Example 1 for the specific procedures. The purity of the LG-NGR-Trap was >95%, suggesting suitability for use in subsequent assays.

3.1.2.2 After a 1-d storage at pH 4.5, the purity of the obtained antibody fusion protein LG-NGR-Trap was determined again by size exclusion chromatography.

The results in Table 7 show that no substantial changes were observed in the SEC main peak of LG-NGR-Trap in the condition, indicating good stability of the molecule.

**Table 7. Purity of antibody fusion protein in storage condition**

| Name | Purity (%) |
|---|---|
| LG-NGR-Trap (PH4.5, 0d) | 97.4 |
| LG-NGR-Trap (PH4.5, 1d) | 97.6 |

### 3.1.3 Flow cytometric assay of antibody fusion protein

Whether the antibody fusion protein of the present disclosure can bind to human FAP protein stably expressed on the surface of engineered 293T cells was determined by a cell binding assay. The 293T cells highly expressing human FAP are as described in Example 1.3. LG-NGR-Trap was 4-fold serially diluted from a starting concentration of 150 nM to a total of 8 concentrations and added to the cells for co-incubation at 4 °C for 30 min. The cells were washed twice with PBS, and then fluorescent secondary antibody R-PE-conjugated AffiniPure Goat Anti-Human IgG, Fcγ Fragment Specific (Jackson Immuno Research, Cat# 109-116-098) was added. The cells were incubated at 4 °C for 30 min. The cells were washed twice with PBS and then resuspended, and finally, fluorescence signals were detected using the BD Accuri C5 (BD Bioscience) flow cytometer.

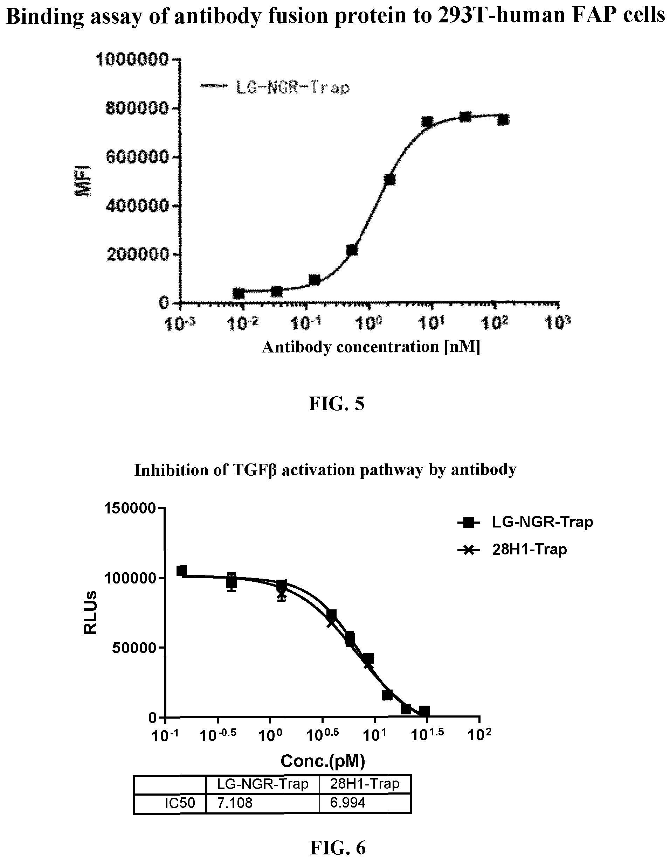

The results shown in FIG. 5 suggest that LG-NGR-Trap can well bind to human FAP protein expressed on engineered 293T cells.

### 3.1.4 Affinity assay of antibody fusion protein for human/cynomolgus FAP and TGFβ

In this study, the binding affinity of the antibody fusion protein LG-NGR-Trap for human FAP protein (Sino biological, Cat# 10464-H07H), human TGFβ1 protein (Sino biological, Cat# 10804-HNAC), human TGFβ2 protein (Novoprotein, Cat# CJ79), and human TGFβ3 protein (Novoprotein, Cat# CJ44) was determined using ForteBio Octet RED96 according to the manufacturer's instructions.

Briefly, an AHC sensor (ForteBio, Cat#: 18-5060) was placed in a running buffer (1× PBS, Hyclone, Cat#: SH30256.01, containing 0.02% Tween 20, pH 7.0) and pre-equilibrated at room temperature for 10 min. In a 96-well plate, the kinetic assay was performed according to the following procedures: a) the system was equilibrated to the baseline for 180 s with the running buffer; b) the antibody fusion protein LG-NGR-Trap diluted with the running buffer at a final concentration of 5 µg/mL was added and immobilized for 200 s; c) the system was equilibrated to the baseline for 300 s with the running buffer; and d) human FAP, TGFβ1, TGFβ2, and TGFβ3 proteins diluted with the running buffer at concentrations of 25 nM, 12.5 nM, 6.25 nM, 3.13 nM, 1.56 nM, 0.78 nM, and 0.39 nM were added to the wells and incubated for 200 s for association and for 600 s for dissociation. The experimental data were fitted and calculated using a Fortebio Data Analysis software 1: 1 binding model.

Table 10 summarizes the binding affinity of the antibody fusion protein LG-NGR-Trap for human FAP protein. Table 11 summarizes the binding affinity of the antibody fusion protein LG-NGR-Trap for human TGFβ1, TGFβ2, and TGFβ3 proteins.

**Table 10. Binding affinity of LG-NGR-Trap for human FAP protein**

| Antibody | Analyte | KD (M) | Kon (M⁻¹S⁻¹) | Koff (S⁻¹) | Full R^² |
|---|---|---|---|---|---|
| LG-NGR-Trap | Recombinant human FAP | <1.0E-12 | 8.99E+05 | <1.0E-07 | 0.9983 |

**Table 11. Binding affinity of LG-NGR-Trap for human TGFβ1, TGFβ2, and TGFβ3 proteins**

| Antibody | Analyte | KD (M) | Kon (M⁻¹S⁻¹) | Koff (S⁻¹) | Full R^² |
|---|---|---|---|---|---|
| LG-NGR-Trap | Recombinant human TGFβ1 | 2.52E-10 | 1.24E+06 | 3.13E-04 | 0.9847 |
| | Recombinant human TGFβ2 | 8.04E-10 | 1.70E+06 | 1.36E-03 | 0.9515 |
| | Recombinant human TGFβ3 | 1.82E-10 | 1.51E+06 | 2.75E-04 | 0.9894 |

Tables 10 and 11 show that the antibody fusion proteins constructed in the present application can specifically bind to FAP protein and TGFβ1, TGFβ2 and TGFβ3 proteins from human. The KD ratios of the antibody fusion protein obtained according to the present application for TGFβ proteins were two orders of magnitude lower than that for FAP protein, suggesting that the FAP with high affinity can effectively reduce the adverse effects of targeting TGF β.

### 3.2 Pharmacodynamic surrogate molecule

### 3.2.1 Construction of pharmacodynamic surrogate molecule

Since huF19-LG-NGR cannot bind to murine protein, it is necessary to add the construction of a surrogate antibody for binding to murine FAP during the pharmacodynamic evaluation stage. The surrogate antibody is derived from 28H1 in WO2012020006. The specific construction procedures are shown in Examples 1 and 3.1, with huF19-LG-NGR being replaced by 28H1. The constructed molecule was designated as 28H1-Trap.

### 3.2.2 Reporter gene assay

In this example, the activating effect of the anti-FAP-Trap antibody fusion protein on the 293T/SBE-*luc2P* reporter gene system was determined. A monoclonal strain 293T/SBE-luc2P stably expressing the SBE*-luc2P* reporter gene element was obtained by transferring a reporter gene element containing SBE*-luc2P* (plasmid: pGL4.48[luc2P/SBE/Hygro] Vector, Promega, Cat# E367A) into 293T cells and pressure screening and limited dilution with hygromycin (Gibco, Cat# 10687010). The added human TGFβ1 (Sino Biological, Cat# 10804-HNAC) factor can bind to TGFβR endogenously expressed on the surface of 293T cells to start a signal cascade to form SMAD complex, and the SMAD complex enters into the cell nucleus to activate the SBE reporter gene element to generate fluorescence signal. The anti-FAP-Trap antibody fusion protein can bind to the human TGFβ1 factor, thus blocking the activation of the SBE reporter gene element. The specific procedures are as follows: 293T/SBE-*luc2P* cells were collected by digestion and centrifugation. The supernatant was discarded, and the cell pellet was resuspended in a complete DMEM medium (Gibco, Cat# 11995-065) containing 10% inactivated fetal bovine serum (Gibco, Cat# 10099-141; inactivated in 56 °C water bath for 30 min). The cells were counted on a Countstar cytometer, and the cell suspension was diluted to 8 × 10⁵ cells/mL in the complete medium. A TGFβ1 protein working solution at 2× working concentration was prepared with the above culture medium, and the final working concentration was 0.4 ng/mL. The fusion proteins of interest (LG-NGR-Trap, 28H1-Trap, LG-NGR-TrapGT, and LG-NGR-TrapE) at 2× final detection concentration were prepared with the TGFβ1 protein working solution. The final detection concentrations of the antibodies of interest were 0.145 pM, 0.434 pM, 1.301 pM, 3.902 pM, 5.853 pM, 8.779 pM, 13.169 pM, 19.753 pM, and 29.630 pM. The 293T/SBE-*luc2P* cell suspension was seeded on a 384-well white assay plate (Corning, Cat# 3570) and loaded at 25 µL/well. The serially diluted antibody dilutions were loaded at 25 µL/well. The cells were incubated overnight (18-20 h) in an incubator at 37 °C, 5% CO₂, and saturated humidity. The next day, 25 µL of One-Glo^{™} Luciferase Assay Reagent (Promega, Cat# E6130) was added and the mixture was uniformly mixed. The luminescence signal was detected at room temperature using the fluorescence detection module of a multifunctional microplate reader (Tecan, F200 PRO).

The results are shown in FIG. 6 and the following table:

| Name | IC50 |
|---|---|
| 28H1-Trap | 6.99 |
| LG-NGR-Trap | 7.11 |
| LG-NGR-TrapGT | 7.32 |
| LG-NGR-TrapE | 7.44 |

LG-NGR-Trap, LG-NGR-TrapGT, and LG-NGR-TrapE exhibited no significant differences in the inhibition of TGFβ activation pathway from the reference 28H1-Trap. Since 28H1-Trap had been confirmed to bind to human FAP at cellular level (as in Example 3.1.3), it is determined that 28H1-Trap can simulate LG-NGR-Trap for *in-vivo* pharmacodynamic studies in mice.

### Example 4: Pharmacodynamic study in IPF animals

In this animal pharmacodynamic study, a bilateral idiopathic pulmonary fibrosis model was induced using bleomycin instillation into the trachea of mice supplied by KCI BioTech. The efficacy of the surrogate antibody fusion protein 28H1-Trap on pulmonary fibrosis was evaluated in this model.

C57BL/6 mice (Cavens Biogle (Suzhou) Co., Ltd.) aged 8-9 weeks were treated from the day of modeling and the induced pulmonary fibrosis model was established by tracheal instillation of bleomycin after the treatment. In the next three weeks, intraperitoneal doses were given twice weekly, and the body weight was measured once every two days. The specific treatments and dosages are shown in the following table:

**Table 12**

| Grouping | Number | IPF modeling | Treatment | Route of administration | Dose | Frequency of administration | Time of first dose | End of treatment |
|---|---|---|---|---|---|---|---|---|
| Group 1 | 12 | No | NA | NA | NA | NA | NA | 21 days from |
| Normal group | | | | | | | | modeling |
| Group 2 | 25 | Yes | PBS | Intraperitoneal | 10mL/kg | Twice every week | Preoperative dosing on modeling day | 21 days from modeling |
| Modeling group | | | | | | | | |
| Group 3 | 25 | Yes | 28H1-Trap | Intraperitoneal | 20mg/kg | Twice every week | Preoperative dosing on modeling day | 21 days from modeling |
| Group 4 | 25 | Yes | 28H1-Trap | Intraperitoneal | 3mg/kg | Twice every week | Preoperative dosing on modeling day | 21 days from modeling |

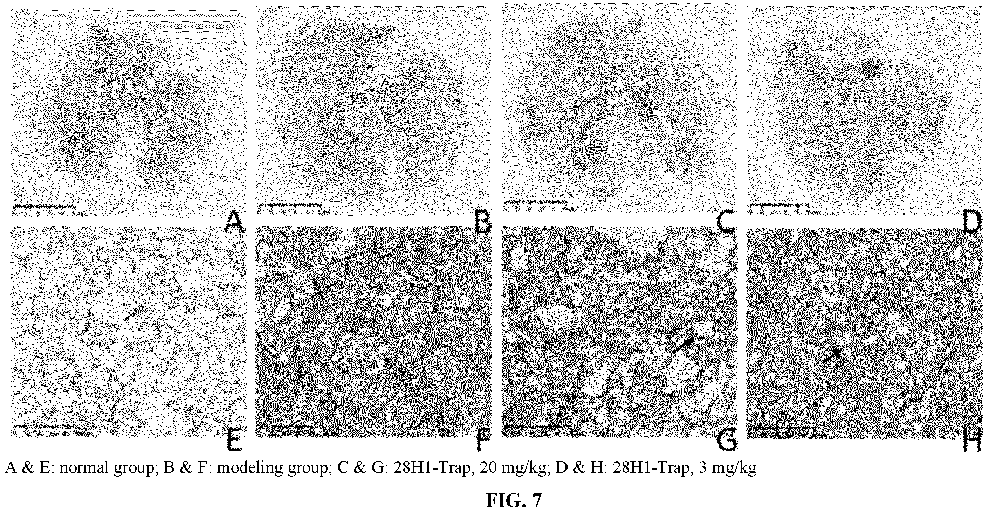

Mice were euthanized at the end of study. At the end of study, both lungs were collected from 15 surviving animals in each group (6 in the normal group) and fixed by formalin perfusion. Histological sections were made, stained with Masson, imaged (FIGs. 7A-H), and graded by pulmonary fibrosis scores. Pathological staining results are shown in FIG. 7, where black arrows indicate intact alveolar structures and the dark interstitial parts indicate collagen accumulation. The results show that the 28H1-Trap high dose group (C & G) could reduce the grade of pulmonary fibrotic lesions as compared to the modeling group (B & F, modeling group) (A & E: normal group, B & F: modeling group, C & G: 28H1-Trap, 20 mg/kg, D & H: 28H1-Trap, 3 mg/kg).

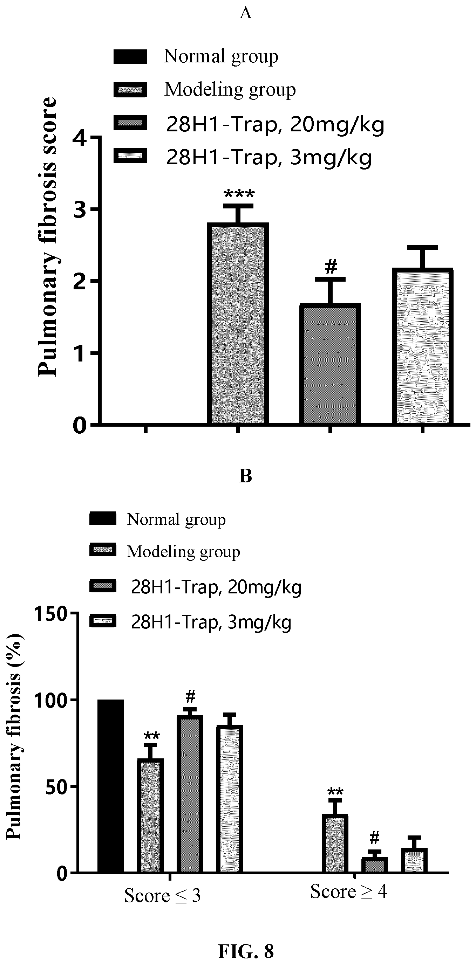

The results of the pulmonary fibrosis staining scoring, as shown in FIG. 8A, suggest that 28H1-Trap can reduce fibrotic lesions in the lungs at a high dose as compared to the modeling group. The results of the pulmonary fibrosis grading (Ashcroft scoring), as shown in FIG. 8B, suggest that 28H1-Trap reduced the pulmonary fibrotic lesions in a dose-dependent manner as compared to the modeling group.

### Example 5: Pharmacodynamic study in UUO animals

In this animal pharmacodynamic study, a renal interstitium fibrosis model was induced by left ureter ligation in mice supplied by KCI BioTech. The efficacy of the surrogate antibody fusion protein 28H1-Trap on renal fibrosis was evaluated in this model.

C57BL/6J mice (Shanghai Jihui Laboratory Animal Care Co., Ltd.) aged 8-9 weeks were randomized by weight. On the day of modeling (day 0), the mice in groups 2-4 were anesthetized with isoflurane. The abdominal cavity was opened along the ventral midline, and the left ureter of the animal was ligated. The abdomen was closed and the skin was sutured. The treatment was given intraperitoneally on the day of modeling. The animals in group 1 were anesthetized with isoflurane. The abdominal cavity was opened along the ventral midline and closed immediately, and the skin was sutured. In the next 10 days, the treatments were given intraperitoneally once every other day, and the body weight was measured once every two days. The specific treatments and dosages are shown in the following table:

**Table 13**

| Grouping | Number | UUO modeling | Treatment | Route of administration | Dose | Frequency of administration | Time of first dose | End of treatment |
|---|---|---|---|---|---|---|---|---|
| Group 1 | 7 | No | PBS | NA | NA | NA | NA | 10 days from modeling |
| Sham surgery group | | | | | | | | |
| Group 2 | 7 | Yes | PBS | Intraperitoneal | 10mL/kg | Once every other day | Postoperative dosing on modeling day | 10 days from modeling |
| Modeling group | | | | | | | | |
| Group 3 | 7 | Yes | 28H1-Trap | Intraperitoneal | 20mg/kg | Once every other day | Postoperative dosing on modeling day | 10 days from modeling |
| High-dose group | | | | | | | | |
| Group 4 | 7 | Yes | 28H1-Trap | Intraperitoneal | 3mg/kg | Once every other day | Postoperative dosing on modeling day | 10 days |
| Low-dose group | | | | | | | | from modeling |

The animals were given an I.P. injection of the test substance on days 0, 2, 4, 6, and 8, and the animals were given the analgesic meloxicam subcutaneously on days 1-3. The body weight was recorded daily. The kidney on the UUO side were scored for fibrosis by Masson staining.

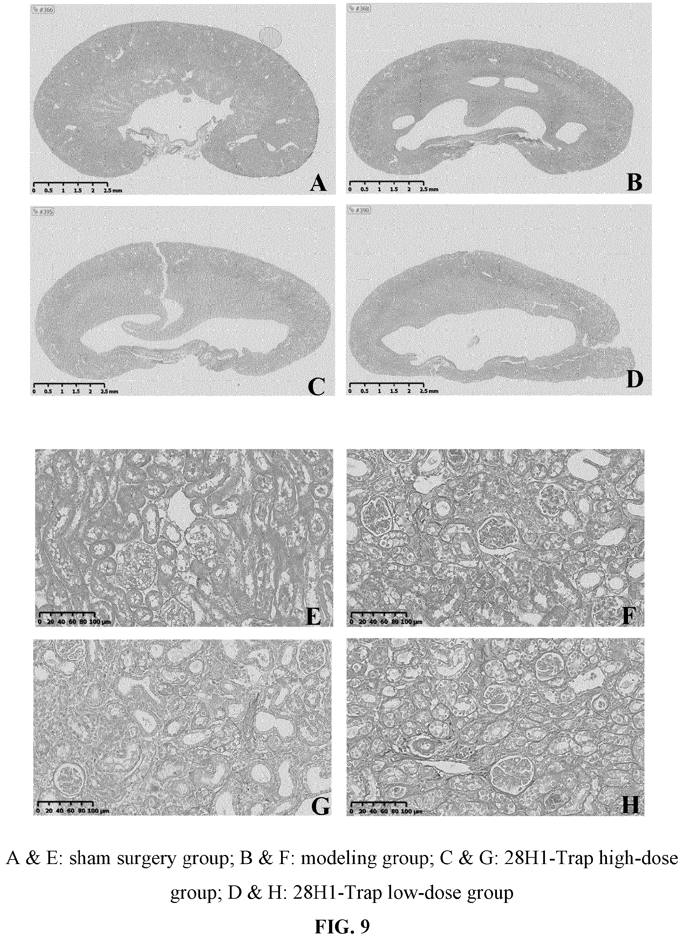

On day 10 from modeling, the animals were anesthetized with sodium pentobarbital and anatomized. The abdominal cavity was opened along the ventral midline after the complete anesthesia was confirmed, and the peripheral blood was collected from the inferior vena cava. The chest cavity was open along the thoracic diaphragm muscle and the costal cartilage to expose the heart, and the inferior vena cava was separated and blocked. Precooled normal saline was infused through the left ventricle for whole body perfusion. The kidney at the UUO side was taken after it faded. The perinephric membrane was carefully peeled off, the kidney was divided into a left half and a right half along the renal hilum. The left half was quickly frozen by liquid nitrogen and then cryopreserved at -80 °C. The right half was fixed in 200 mL of 10% formalin for 3 or more days, and stained with Masson (FIG. 9). The degree of fibrosis of the kidney was evaluated (see FIG. 10).

The pathological results (FIG. 9) and the kidney fibrosis score (FIG. 10) by Masson staining indicate that the test substance 28H1-Trap has a significant dose-dependent ameliorative effect on the fibrosis in the renal cortex of the kidney.

### SEQUENCE LISTING

| Sequence No. | Description | Specific sequence |
|---|---|---|
| 1 | F19 light chain variable region | DIVMSQSPSSLAVSVGEKVTMSCKSSQSLLYSRNQKNYLAWFQQKPGQSPKLLIFWASTRESGVPDR FTGSGFGTDFNLTISSVQAEDLAVYDCQQYFSYPLTFGAGTKLELK |
| 2 | Light chain constant region | |
| 3 | F19 light chain (F19L) | DIVMSQSPSSLAVSVGEKVTMSCKSSQSLLYSRNQKNYLAWFQQKPGQSPKLLIFWASTRESGVPDR FTGSGFGTDFNLTISSVQAEDLAVYDCQQYFSYPLTFGAGTKLELKRTVAAPSVFIFPPSDEQLKSGTA SVVCLLNNFYPREAKVQWKVDNALQSGNSQESVTEQDSKDSTYSLSSTLTLSKADYEKHKVYACEV THQGLSSPVTKSFNRGEC |
| 4 | F19-L CDR1 | KSSQSLLYSRNQKNYLA |
| 5 | F19-L CDR2 | WASTRES |
| 6 | F19-L CDR3 | QQYFSYPLT |
| 7 | F19 heavy chain variable region | EVQLQQSGPELVKPGASVKMSCKTSRYTFTEYTIHWVRQSHGKSLEWIGGINPNNGIPNYNQKFKGR ATLTVGKSSSTAYMELRSLTSEDSAVYFCARRRIAYGYDEGHAMDYWGQGTSVTVSS |
| 8 | Heavy chain constant region | |
| 9 | F19 heavy chain (F19H) | EVQLQQSGPELVKPGASVKMSCKTSRYTFTEYTIHWVRQSHGKSLEWIGGINPNNGIPNYNQKFKGR ATLTVGKSSSTAYMELRSLTSEDSAVYFCARRRIAYGYDEGHAMDYWGQGTSVTVSSASTKGPSVF PLAPSSKSTSGGTAALGCLVKDYFPEPVTVSWNSGALTSGVHTFPAVLQSSGLYSLSSVVTVPSSSLG TQTYICNVNHKPSNTKVDKKVEPKSCDKTHTCPPCPAPELLGGPSVFLFPPKPKDTLMISRTPEVTCV VVDVSHEDPEVKFNWYVDGVEVHNAKTKPREEQYNSTYRVVSVLTVLHQDWLNGKEYKCKVSNK ALPAPIEKTISKAKGQPREPQVYTLPPSRDELTKNQVSLTCLVKGFYPSDIAVEWESNGQPENNYKTT PPVLDSDGSFFLYSKLTVDKSRWQQGNVFSCSVMHEALHNHYTQKSLSLSPGK |
| 10 | F 19-H CDR1 | EYTIH |
| 11 | F 19-H CDR2 | GINPNNGIPNYNQKFKG |
| 12 | F19-HCDR3 | RRIAYGYDEGHAMDY |
| 13 | Humanized anti-FAP antibody huF19-LG heavy chain variable region huF 19-LG-VH | QVQLVQSGAEVKKPGASVKVSCKASGYTFTEYTIHWVRQATGQGLEWIGGINPNNGIPNYNQKFKG RATMTVGTSISTAYMELSSLRSEDTAVYFCARRRIAYGYDEGHAMDYWGQGTLVTVSS |
| 10 | huF 19-LG-VH CDR1 | EYTIH |
| 11 | huF 19-LG -VH CDR2 | GINPNNGIPNYNQKFKG |
| 12 | huF 19-LG - VH CDR3 | RRIAYGYDEGHAMDY |
| 8 | huF19-LG heavy chain constant region | |
| 14 | huF 19-LG heavy chain | QVQLVQSGAEVKKPGASVKVSCKASGYTFTEYTIHWVRQATGQGLEWIGGINPNNGIPNYNQKFKG RATMTVGTSISTAYMELSSLRSEDTAVYFCARRRIAYGYDEGHAMDYWGQGTLVTVSSASTKGPSV FPLAPSSKSTSGGTAALGCLVKDYFPEPVTVSWNSGALTSGVHTFPAVLQSSGLYSLSSVVTVPSSSL GTQTYICNVNHKPSNTKVDKKVEPKSCDKTHTCPPCPAPELLGGPSVFLFPPKPKDTLMISRTPEVTC VVVDVSHEDPEVKFNWYVDGVEVHNAKTKPREEQYNSTYRVVSVLTVLHQDWLNGKEYKCKVSN KALPAPIEKTISKAKGQPREPQVYTLPPSRDELTKNQVSLTCLVKGFYPSDIAVEWESNGQPENNYKT TPPVLDSDGSFFLYSKLTVDKSRWQQGNVFSCSVMHEALHNHYTQKSLSLSPGK |
| 15 | Humanized anti-FAP antibody huF19-LG light chain variable region huF 19-LG-VL | DIQMTQSPSSLSASVGDRVTITCKSSQSLLYSRNQKNYLAWFQQKPGKAPKLLIFWASTRESGVPSRF TGSGFGTDFTLTISSLQPEDFATYYCQQYFSYPLTFGQGTKLEIK |
| 4 | huF19-LG -VL CDR1 | KSSQSLLYSRNQKNYLA |
| 5 | huF 19-LG -VL CDR2 | WASTRES |
| 6 | huF 19-LG -VL CDR3 | QQYFSYPLT |
| 2 | huF 19-LG light chain constant region | |
| 16 | huF 19-LG light chain | DIQMTQSPSSLSASVGDRVTITCKSSQSLLYSRNQKNYLAWFQQKPGKAPKLLIFWASTRESGVPSRF TGSGFGTDFTLTISSLQPEDFATYYCQQYFSYPLTFGQGTKLEIKRTVAAPSVFIFPPSDEQLKSGTASV VCLLNNFYPREAKVQWKVDNALQSGNSQESVTEQDSKDSTYSLSSTLTLSKADYEKHKVYACEVTH QGLSSPVTKSFNRGEC |
| 17 | Sibrotuzumab heavy chain variable region | QVQLVQSGAEVKKPGASVKVSCKTSRYTFTEYTIHWVRQAPGQRLEWIGGINPNNGIPNYNQKFKG RVTITVDTSASTAYMELSSLRSEDTAVYYCARRRIAYGYDEGHAMDYWGQGTLVTVSS |
| 8 | Sibrotuzumab heavy chain constant region | |
| 18 | Sibrotuzumab heavy chain | QVQLVQSGAEVKKPGASVKVSCKTSRYTFTEYTIHWVRQAPGQRLEWIGGINPNNGIPNYNQKFKG RVTITVDTSASTAYMELSSLRSEDTAVYYCARRRIAYGYDEGHAMDYWGQGTLVTVSSASTKGPSV FPLAPSSKSTSGGTAALGCLVKDYFPEPVTVSWNSGALTSGVHTFPAVLQSSGLYSLSSVVTVPSSSL GTQTYICNVNHKPSNTKVDKKVEPKSCDKTHTCPPCPAPELLGGPSVFLFPPKPKDTLMISRTPEVTC VVVDVSHEDPEVKFNWYVDGVEVHNAKTKPREEQYNSTYRVVSVLTVLHQDWLNGKEYKCKVSN KALPAPIEKTISKAKGQPREPQVYTLPPSRDELTKNQVSLTCLVKGFYPSDIAVEWESNGQPENNYKT TPPVLDSDGSFFLYSKLTVDKSRWQQGNVFSCSVMHEALHNHYTQKSLSLSPGK |
| 19 | Sibrotuzumab light chain variable region | DIVMTQSPDSLAVSLGERATINCKSSQSLLYSRNQKNYLAWYQQKPGQPPKLLIFWASTRESGVPDR FSGSGFGTDFTLTISSLQAEDVAVYYCQQYFSYPLTFGQGTKVEIK |
| 2 | Sibrotuzumab light chain constant region | |
| 20 | Sibrotuzumab light chain | DIVMTQSPDSLAVSLGERATINCKSSQSLLYSRNQKNYLAWYQQKPGQPPKLLIFWASTRESGVPDR FSGSGFGTDFTLTISSLQAEDVAVYYCQQYFSYPLTFGQGTKVEIKRTVAAPSVFIFPPSDEQLKSGTA SVVCLLNNFYPREAKVQWKVDNALQSGNSQESVTEQDSKDSTYSLSSTLTLSKADYEKHKVYACEV THQGLSSPVTKSFNRGEC |
| 21 | Mutant humanized FAP antibody huF19-LG-NGR heavy chain variable region LG-NGR-VH | QVQLVQSGAEVKKPGASVKVSCKASGYTFTEYTIHWVRQATGQGLEWIGGINPNNRIPNYNQKFKG RATMTVGTSISTAYMELSSLRSEDTAVYFCARRRIAYGYDEGHAMDYWGQGTLVTVSS |
| 10 | huF19-LG-NGR-VH CDR1 | EYTIH |
| 22 | huF19-LG-NGR-VH CDR2(G56R(Kabat)) | GINPNNRIPNYNQKFKG |
| 12 | huF19-LG-NGR-VH CDR3 | RRIAYGYDEGHAMDY |
| 8 | huF 19-LG-NGR heavy chain constant region | |
| 23 | huF 19-LG-NGR heavy chain | QVQLVQSGAEVKKPGASVKVSCKASGYTFTEYTIHWVRQATGQGLEWIGGINPNNRIPNYNQKFKG RATMTVGTSISTAYMELSSLRSEDTAVYFCARRRIAYGYDEGHAMDYWGQGTLVTVSSASTKGPSV FPLAPSSKSTSGGTAALGCLVKDYFPEPVTVSWNSGALTSGVHTFPAVLQSSGLYSLSSVVTVPSSSL GTQTYICNVNHKPSNTKVDKKVEPKSCDKTHTCPPCPAPELLGGPSVFLFPPKPKDTLMISRTPEVTC VVVDVSHEDPEVKFNWYVDGVEVHNAKTKPREEQYNSTYRVVSVLTVLHQDWLNGKEYKCKVSN KALPAPIEKTISKAKGQPREPQVYTLPPSRDELTKNQVSLTCLVKGFYPSDIAVEWESNGQPENNYKT TPPVLDSDGSFFLYSKLTVDKSRWQQGNVFSCSVMHEALHNHYTQKSLSLSPGK |
| 15 | huF 19-LG-NGR light chain variable region huF 19-LG-VL | DIQMTQSPSSLSASVGDRVTITCKSSQSLLYSRNQKNYLAWFQQKPGKAPKLLIFWASTRESGVPSRF TGSGFGTDFTLTISSLQPEDFATYYCQQYFSYPLTFGQGTKLEIK |
| 4 | huF19-LG-NGR-VL CDR1 | KSSQSLLYSRNQKNYLA |
| 5 | huF19-LG-NGR-VL CDR2 | WASTRES |
| 6 | huF19-LG-NGR-VL CDR3 | QQYFSYPLT |
| 2 | huF 19-LG-NGR light chain constant region | |
| 16 | huF 19-LG-NGR light chain | DIQMTQSPSSLSASVGDRVTITCKSSQSLLYSRNQKNYLAWFQQKPGKAPKLLIFWASTRESGVPSRF TGSGFGTDFTLTISSLQPEDFATYYCQQYFSYPLTFGQGTKLEIKRTVAAPSVFIFPPSDEQLKSGTASV VCLLNNFYPREAKVQWKVDNALQSGNSQESVTEQDSKDSTYSLSSTLTLSKADYEKHKVYACEVTH QGLSSPVTKSFNRGEC |
| 24 | TGF βRII extracellular domain (ECD) | IPPHVQKSVNNDMIVTDNNGAVKFPQLCKFCDVRFSTCDNQKSCMSNCSITSICEKPQEVCVAVWRK NDENITLETVCHDPKLPYHDFILEDAASPKCIMKEKKKPGETFFMCSCSSDECNDNIIFSEEYNTSNPD |
| 25 | LG-NGR-Trap heavy chain: LG-NGR-VH (underlined)-heavy chain constant region (highlighted grey, terminal K-to-A mutation)-TGFβ extracellular domain (italic) | QVQLVQSGAEVKKPGASVKVSCKASGYTFTEYTIHWVRQATGQGLEWIGGINPNNRIPNYNQKFKG RATMTVGTSISTAYMELSSLRSEDTAVYFCARRRIAYGYDEGHAMDYWGQGTLVTVSSASTKGPSV FPLAPSSKSTSGGTAALGCLVKDYFPEPVTVSWNSGALTSGVHTFPAVLQSSGLYSLSSVVTVPSSSL GTQTYICNVNHKPSNTKVDKKVEPKSCDKTHTCPPCPAPELLGGPSVFLFPPKPKDTLMISRTPEVTC VVVDVSHEDPEVKFNWYVDGVEVHNAKTKPREEQYNSTYRVVSVLTVLHQDWLNGKEYKCKVSN KALPAPIEKTISKAKGQPREPQVYTLPPSRDELTKNQVSLTCLVKGFYPSDIAVEWESNGQPENNYKT TPPVLDSDGSFFLYSKLTVDKSRWQQGNVFSCSVMHEALHNHYTQKSLSLSPGAIPPHVQKSVNNDM IVTDNNGAVKFPQLCKFCDVRFSTCDNQKSCMSNCSITSICEKPQEVCVAVWRKNDENITLETVCHDPKLP YHDFILEDAASPKCIMKEKKKPGETFFMCSCSSDECNDNIIFSEEYNTSNPD |
| 26 | LG-NGR-Trap heavy chain constant region (IgG1, terminal K-to-A mutation) | ASTKGPSVFPLAPSSKSTSGGTAALGCLVKDYFPEPVTVSWNSGALTSGVHTFPAVLQSSGLYSLSSV VTVPSSSLGTQTYICNVNHKPSNTKVDKKVEPKSCDKTHTCPPCPAPELLGGPSVFLFPPKPKDTLMIS RTPEVTCVVVDVSHEDPEVKFNWYVDGVEVHNAKTKPREEQYNSTYRVVSVLTVLHQDWLNGKE YKCKVSNKALPAPIEKTISKAKGQPREPQVYTLPPSRDELTKNQVSLTCLVKGFYPSDIAVEWESNGQ PENNYKTTPPVLDSDGSFFLYSKLTVDKSRWQQGNVFSCSVMHEALHNHYTQKSLSLSPGA |
| 27 | 28H1-Trap light chain variable region (i.e., antibody 28H1 light chain variable region) | EIVLTQSPGTLSLSPGERATLSCRASQSVSRSYLAWYQQKPGQAPRLLIIGASTRATGIPDRFSGSGSGT DFTLTISRLEPEDFAVYYCQQGQVIPPTFGQGTKVEIK |
| 28 | 28H1-Trap light chain (i.e., antibody 28H1 light chain) | EIVLTQSPGTLSLSPGERATLSCRASQSVSRSYLAWYQQKPGQAPRLLIIGASTRATGIPDRFSGSGSGT DFTLTISRLEPEDFAVYYCQQGQVIPPTFGQGTKVEIKRTVAAPSVFIFPPSDEQLKSGTASVVCLLNN FYPREAKVQWKVDNALQSGNSQESVTEQDSKDSTYSLSSTLTLSKADYEKHKVYACEVTHQGLSSP VTKSFNRGEC |
| 29 | 28H1-Trap heavy chain: 28H1-VH (underlined)-heavy chain constant region (highlighted grey, terminal K-to-A mutation)-TGFβ extracellular domain (italic) | EVQLLESGGGLVQPGGSLRLSCAASGFTFSSHAMSWVRQAPGKGLEWVSAIWASGEQYYADSVKG RFTISRDNSKNTLYLQMNSLRAEDTAVYYCAKGWLGNFDYWGQGTLVTVSSASTKGPSVFPLAPSS KSTSGGTAALGCLVKDYFPEPVTVSWNSGALTSGVHTFPAVLQSSGLYSLSSVVTVPSSSLGTQTYIC NVNHKPSNTKVDKKVEPKSCDKTHTCPPCPAPELLGGPSVFLFPPKPKDTLMISRTPEVTCVVVDVSH EDPEVKFNWYVDGVEVHNAKTKPREEQYNSTYRVVSVLTVLHQDWLNGKEYKCKVSNKALPAPIE KTISKAKGQPREPQVYTLPPSRDELTKNQVSLTCLVKGFYPSDIAVEWESNGQPENNYKTTPPVLDSD GSFFLYSKLTVDKSRWQQGNVFSCSVMHEALHNHYTQKSLSLSPGAIPPHVQKSVNNDMIVTDNNGA VKFPQLCKFCDVRFSTCDNQKSCMSNCSITSICEKPQEVCVAVWRKNDENITLETVCHDPKLPYHDFILED AASPKCIMKEKKKPGETFFMCSCSSDECNDNIIFSEEYNTSNPD |
| 30 | 28H1-VH | EVQLLESGGGLVQPGGSLRLSCAASGFTFSSHAMSWVRQAPGKGLEWVSAIWASGEQYYADSVKG RFTISRDNSKNTLYLQMNSLRAEDTAVYYCAKGWLGNFDYWGQGTLVTVSS |
| 26 | 28H1-Trap heavy chain constant region (IgG1, terminal K-to-A mutation) | ASTKGPSVFPLAPSSKSTSGGTAALGCLVKDYFPEPVTVSWNSGALTSGVHTFPAVLQSSGLYSLSSV VTVPSSSLGTQTYICNVNHKPSNTKVDKKVEPKSCDKTHTCPPCPAPELLGGPSVFLFPPKPKDTLMIS RTPEVTCVVVDVSHEDPEVKFNWYVDGVEVHNAKTKPREEQYNSTYRVVSVLTVLHQDWLNGKE YKCKVSNKALPAPIEKTISKAKGQPREPQVYTLPPSRDELTKNQVSLTCLVKGFYPSDIAVEWESNGQ PENNYKTTPPVLDSDGSFFLYSKLTVDKSRWQQGNVFSCSVMHEALHNHYTQKSLSLSPGA |
| 24 | 28H1-Trap TGFβ extracellular domain | IPPHVQKSVNNDMIVTDNNGAVKFPQLCKFCDVRFSTCDNQKSCMSNCSITSICEKPQEVCVAVWRK NDENITLETVCHDPKLPYHDFILEDAASPKCIMKEKKKPGETFFMCSCSSDECNDNIIFSEEYNTSNPD |
| 31 | Human FAP | MKTWVKIVFGVATSAVLALLVMCIVLRPSRVHNSEENTMRALTLKDILNGTFSYKTFFPNWISGQEY LHQSADNNIVLYNIETGQSYTILSNRTMKSVNASNYGLSPDRQFVYLESDYSKLWRYSYTATYYIYD LSNGEFVRGNELPRPIQYLCWSPVGSKLAYVYQNNIYLKQRPGDPPFQITFNGRENKIFNGIPDWVYE EEMLATKYALWWSPNGKFLAYAEFNDTDIPVIAYSYYGDEQYPRTINIPYPKAGAKNPVVRIFIIDTT YPAYVGPQEVPVPAMIASSDYYFSWLTWVTDERVCLQWLKRVQNVSVLSICDFREDWQTWDCPKT QEHIEESRTGWAGGFFVSTPVFSYDAISYYKIFSDKDGYKHIHYIKDTVENAIQITSGKWEAINIFRVT QDSLFYSSNEFEEYPGRRNIYRISIGSYPPSKKCVTCHLRKERCQYYTASFSDYAKYYALVCYGPGIPI STLHDGRTDQEIKILEENKELENALKNIQLPKEEIKKLEVDEITLWYKMILPPQFDRSKKYPLLIQVYG GPCSQSVRSVFAVNWISYLASKEGMVIALVDGRGTAFQGDKLLYAVYRKLGVYEVEDQITAVRKFI EMGFIDEKRIAIWGWSYGGYVSSLALASGTGLFKCGIAVAPVSSWEYYASVYTERFMGLPTKDDNL EHYKNSTVMARAEYFRNVDYLLIHGTADDNVHFQNSAQIAKALVNAQVDFQAMWYSDQNHGLSG LSTNHLYTHMTHFLKQCFSLSD* |
| 32 | Cynomolgus FAP | MKTWVKIVFGVATSAVLALLVMCIVLRPPRVHNSEENTMRALTLKDILNGTFSYKTFFPNWISGQEY LHQSADNNIVLYNIETGQSYTILSNRTMKSVNASNYGLSPDRQFVYLESDYSKLWRYSYTATYYIYD LSNGEFVRGNELPRPIQYLCWSPVGSKLAYVYQNNIYLKQRPGDPPFQITFNGRENKIFNGIPDWVYE EEMLATKYALWWSPNGKFLAYAEFNDTDIPVIAYSYYGDEQYPRTINIPYPKAGAKNPFVRIFIIDTT YPAYVGPQEVPVPAMIASSDYYFSWLTWVTDERVCLQWLKRVQNVSVLSICDFREDWQTWDCPKT QEHIEESRTGWAGGFFVSTPVFSYDAISYYKIFSDKDGYKHIHYIKDTVENAIQITSGKWEAINIFRVT QDSLFYSSNEFEDYPGRRNIYRISIGSYPPSKKCVTCHLRKERCQYYTASFSDYAKYYALVCYGPGIPI STLHDGRTDQEIKILEENKELENALKNIQLPKEEIKKLEVDEITLWYKMILPPQFDRSKKYPLLIQVYG GPCSQSVRSVFAVNWISYLASKEGMVIALVDGRGTAFQGDKLLYAVYRKLGVYEVEDQITAVRKFI EMGFIDEKRIAIWGWSYGGYVSSLALASGTGLFKCGIAVAPVSSWEYYASVYTERFMGLPTKDDNL EHYKNSTVMARAEYFRNVDYLLIHGTADDNVHFQNSAQIAKALVNAQVDFQAMWYSDQNHGLSG LSTNHLYTHMTHFLKQCFSLSD* |
| 33 | Human DPP4 | MKTPWKVLLGLLGAAALVTIITVPVVLLNKGTDDATADSRKTYTLTDYLKNTYRLKLYSLRWISDH EYLYKQENNILVFNAEYGNSSVFLENSTFDEFGHSINDYSISPDGQFILLEYNYVKQWRHSYTASYDI YDLNKRQLITEERIPNNTQWVTWSPVGHKLAYVWNNDIYVKIEPNLPSYRITWTGKEDIIYNGITDW VYEEEVFSAYSALWWSPNGTFLAYAQFNDTEVPLIEYSFYSDESLQYPKTVRVPYPKAGAVNPTVKF FVVNTDSLSSVTNATSIQITAPASMLIGDHYLCDVTWATQERISLQWLRRIQNYSVMDICDYDESSGR WNCLVARQHIEMSTTGWVGRFRPSEPHFTLDGNSFYKIISNEEGYRHICYFQIDKKDCTFITKGTWEV IGIEALTSDYLYYISNEYKGMPGGRNLYKIQLSDYTKVTCLSCELNPERCQYYSVSFSKEAKYYQLRC SGPGLPLYTLHSSVNDKGLRVLEDNSALDKMLQNVQMPSKKLDFIILNETKFWYQMILPPHFDKSKK YPLLLDVYAGPCSQKADTVFRLNWATYLASTENIIVASFDGRGSGYQGDKIMHAINRRLGTFEVEDQ IEAARQFSKMGFVDNKRIAIWGWSYGGYVTSMVLGSGSGVFKCGIAVAPVSRWEYYDSVYTERYM GLPTPEDNLDHYRNSTVMSRAENFKQVEYLLIHGTADDNVHFQQSAQISKALVDVGVDFQAMWYT DEDHGIASSTAHQHIYTHMSHFIKQCFSLP* |
| 34 | TGFβRII | IPPHVQKSVNNDMIVTDNNGAVKFPQLCKFCDVRFSTCDNQKSCMSNCSITSICEKPQEVCVAVWRK NDENITLETVCHDPKLPYHDFILEDAASPKCIMKEKKKPGETFFMCSCSSDECNDNIIFSEEYNTSNPD LLLVIFQVTGISLLPPLGVAISVIIIFYCYRVNRQQKLSSTWETGKTRKLMEFSEHCAIILEDDRSDISST CANNINHNTELLPIELDTLVGKGRFAEVYKAKLKQNTSEQFETVAVKIFPYEEYASWKTEKDIFSDIN LKHENILQFLTAEERKTELGKQYWLITAFHAKGNLQEYLTRHVISWEDLRKLGSSLARGIAHLHSDH TPCGRPKMPIVHRDLKSSNILVKNDLTCCLCDFGLSLRLDPTLSVDDLANSGQVGTARYMAPEVLES RMNLENVESFKQTDVYSMALVLWEMTSRCNAVGEVKDYEPPFGSKVREHPCVESMKDNVLRDRG RPEIPSFWLNHQGIQMVCETLTECWDHDPEARLTAQCVAERFSELEHLDRLSGRSCSEEKIPEDGSLN TTK |
| 35 | Anti-FAP antibody heavy chain in fusion protein molecule | QVQLVQSGAEVKKPGASVKVSCKASGYTFTEYTIHWVRQATGQGLEWIGGINPNNRIPNYNQKFKG RATMTVGTSISTAYMELSSLRSEDTAVYFCARRRIAYGYDEGHAMDYWGQGTLVTVSSASTKGPSV FPLAPSSKSTSGGTAALGCLVKDYFPEPVTVSWNSGALTSGVHTFPAVLQSSGLYSLSSVVTVPSSSL GTQTYICNVNHKPSNTKVDKKVEPKSCDKTHTCPPCPAPELLGGPSVFLFPPKPKDTLMISRTPEVTC VVVDVSHEDPEVKFNWYVDGVEVHNAKTKPREEQYNSTYRVVSVLTVLHQDWLNGKEYKCKVSN KALPAPIEKTISKAKGQPREPQVYTLPPSRDELTKNQVSLTCLVKGFYPSDIAVEWESNGQPENNYKT TPPVLDSDGSFFLYSKLTVDKSRWQQGNVFSCSVMHEALHNHYTQKSLSLSPGA |
| 36 | LG-NGR-TrapGT | QVQLVQSGAEVKKPGASVKVSCKASGYTFTEYTIHWVRQATGQGLEWIGGINPNNRIPNYNQKFKG RATMTVGTSISTAYMELSSLRSEDTAVYFCARRRIAYGYDEGHAMDYWGQGTLVTVSSASTKGPSV FPLAPSSKSTSGGTAALGCLVKDYFPEPVTVSWNSGALTSGVHTFPAVLQSSGLYSLSSVVTVPSSSL GTQTYICNVNHKPSNTKVDKKVEPKSCDKTHTCPPCPAPELLGGPSVFLFPPKPKDTLMISRTPEVTC VVVDVSHEDPEVKFNWYVDGVEVHNAKTKPREEQYNSTYRVVSVLTVLHQDWLNGKEYKCKVSN KALPAPIEKTISKAKGQPREPQVYTLPPSRDELTKNQVSLTCLVKGFYPSDIAVEWESNGQPENNYKT TPPVLDSDGSFFLYSKLTVDKSRWQQGNVFSCSVMHEALHNHYTQKSLSLSPGAGGGGSGGGGSGG GGSGGGGSIPPHVQKSVNNDMIVTDNNTAVKFPQLCKFCDVRFSTCDNQKSCMSNCSITSICEKPQE VCVAVWRKNDENITLETVCHDPKLPYHDFILEDAASPKCIMKEKKKPGETFFMCSCSSDECNDNIIFS EEYNTSNPD |
| 37 | TGFβRII ECD (G20T) | IPPHVQKSVNNDMIVTDNNTAVKFPQLCKFCDVRFSTCDNQKSCMSNCSITSICEKPQEVCVAVWRK NDENITLETVCHDPKLPYHDFILEDAASPKCIMKEKKKPGETFFMCSCSSDECNDNIIFSEEYNTSNPD * |
| 38 | LG-NGR-TrapE | QVQLVQSGAEVKKPGASVKVSCKASGYTFTEYTIHWVRQATGQGLEWIGGINPNNRIPNYNQKFKG RATMTVGTSISTAYMELSSLRSEDTAVYFCARRRIAYGYDEGHAMDYWGQGTLVTVSSASTKGPSV FPLAPSSKSTSGGTAALGCLVKDYFPEPVTVSWNSGALTSGVHTFPAVLQSSGLYSLSSVVTVPSSSL GTQTYICNVNHKPSNTKVDKKVEPKSCDKTHTCPPCPAPELLGGPSVFLFPPKPKDTLMISRTPEVTC VVVDVSHEDPEVKFNWYVDGVEVHNAKTKPREEQYNSTYRVVSVLTVLHQDWLNGKEYKCKVSN KALPAPIEKTISKAKGQPREPQVYTLPPSRDELTKNQVSLTCLVKGFYPSDIAVEWESNGQPENNYKT TPPVLDSDGSFFLYSKLTVDKSRWQQGNVFSCSVMHEALHNHYTQKSLSLSPGAGGGGSGGGGSGG GGSGGGGSIPPHVQKSVNNDMIVTDNEGAVKFPQLCKFCDVRFSTCDNQKSCMSNCSITSICEKPQE VCVAVWRKNDENITLETVCHDPKLPYHDFILEDAASPKCIMKEKKKPGETFFMCSCSSDECNDNIIFS EEYNTSNPD |
| 39 | TGFβRII ECD (N19E) | IPPHVQKSVNNDMIVTDNEGAVKFPQLCKFCDVRFSTCDNQKSCMSNCSITSICEKPQEVCVAVWRK NDENITLETVCHDPKLPYHDFILEDAASPKCIMKEKKKPGETFFMCSCSSDECNDNIIFSEEYNTSNPD |
| 40 | Fc region | EPKSCDKTHTCPPCPAPELLGGPSVFLFPPKPKDTLMISRTPEVTCVVVDVSHEDPEVKFNWYVDGVE VHNAKTKPREEQYNSTYRVVSVLTVLHQDWLNGKEYKCKVSNKALPAPIEKTISKAKGQPREPQVY TLPPSRDELTKNQVSLTCLVKGFYPSDIAVEWESNGQPENNYKTTPPVLDSDGSFFLYSKLTVDKSR WQQGNVFSCSVMHEALHNHYTQKSLSLSPGK |
| 41 | 28H1 antibody heavy chain (terminal A mutation) | EVQLLESGGGLVQPGGSLRLSCAASGFTFSSHAMSWVRQAPGKGLEWVSAIWASGEQYYADSVKG RFTISRDNSKNTLYLQMNSLRAEDTAVYYCAKGWLGNFDYWGQGTLVTVSSASTKGPSVFPLAPSS KSTSGGTAALGCLVKDYFPEPVTVSWNSGALTSGVHTFPAVLQSSGLYSLSSVVTVPSSSLGTQTYIC NVNHKPSNTKVDKKVEPKSCDKTHTCPPCPAPELLGGPSVFLFPPKPKDTLMISRTPEVTCVVVDVSH EDPEVKFNWYVDGVEVHNAKTKPREEQYNSTYRVVSVLTVLHQDWLNGKEYKCKVSNKALPAPIE KTISKAKGQPREPQVYTLPPSRDELTKNQVSLTCLVKGFYPSDIAVEWESNGQPENNYKTTPPVLDSD GSFFLYSKLTVDKSRWQQGNVFSCSVMHEALHNHYTQKSLSLSPGA |
| 42 | 28H1 antibody heavy chain | EVQLLESGGGLVQPGGSLRLSCAASGFTFSSHAMSWVRQAPGKGLEWVSAIWASGEQYYADSVKG RFTISRDNSKNTLYLQMNSLRAEDTAVYYCAKGWLGNFDYWGQGTLVTVSSASTKGPSVFPLAPSS KSTSGGTAALGCLVKDYFPEPVTVSWNSGALTSGVHTFPAVLQSSGLYSLSSVVTVPSSSLGTQTYIC NVNHKPSNTKVDKKVEPKSCDKTHTCPPCPAPELLGGPSVFLFPPKPKDTLMISRTPEVTCVVVDVSH EDPEVKFNWYVDGVEVHNAKTKPREEQYNSTYRVVSVLTVLHQDWLNGKEYKCKVSNKALPAPIE KTISKAKGQPREPQVYTLPPSRDELTKNQVSLTCLVKGFYPSDIAVEWESNGQPENNYKTTPPVLDSD GSFFLYSKLTVDKSRWQQGNVFSCSVMHEALHNHYTQKSLSLSPGK |

## Claims

1. A fusion protein molecule specifically binding to human FAP and human TGFβ, e.g., human TGFβ1, TGFβ2, and/or TGFβ3.

2. The fusion protein molecule according to claim 1, comprising a first target-binding region and a second target-binding region, wherein the first target-binding region specifically binds to FAP, and the second target-binding region specifically binds to TGFβ.

3. The fusion protein molecule according to claim 2, wherein the first target-binding region and the second target-binding region are connected with a linker or directly connected without a linker; for example, the linker is (G₄S)ₙ, wherein n = 1, 2, 3, 4, 5, or 6, e.g., n = 4.

4. The fusion protein molecule according to claim 2 or 3, wherein the first target-binding region is derived from an anti-FAP antibody; e.g., an F19 antibody or a humanized antibody thereof or a 28H1 antibody or a humanized antibody thereof.

5. The fusion protein molecule according to any one of claims 2-4, wherein the first target-binding region comprises 3 CDRs of a heavy chain variable region VH: an HCDR1, an HCDR2, and an HCDR3, and 3 CDRs of a light chain variable region VL: an LCDR1, an LCDR2, and an LCDR3, wherein
(i) the HCDR1, the HCDR2, and the HCDR3 are selected from the three complementarity determining regions HCDR1, HCDR2, and HCDR3 in the VH set forth in SEQ ID NO: 7; and the LCDR1, the LCDR2, and the LCDR3 are the three complementarity determining regions LCDR1, LCDR2, and LCDR3 in the VL set forth in SEQ ID NO: 1;
(ii) the HCDR1, the HCDR2, and the HCDR3 are selected from the three complementarity determining regions HCDR1, HCDR2, and HCDR3 in the VH set forth in SEQ ID NO: 13; and the LCDR1, the LCDR2, and the LCDR3 are the three complementarity determining regions LCDR1, LCDR2, and LCDR3 in the VL set forth in SEQ ID NO: 15;
(iii) the HCDR1, the HCDR2, and the HCDR3 are selected from the three complementarity determining regions HCDR1, HCDR2, and HCDR3 in the VH set forth in SEQ ID NO: 21; and the LCDR1, the LCDR2, and the LCDR3 are the three complementarity determining regions LCDR1, LCDR2, and LCDR3 in the VL set forth in SEQ ID NO: 15;
(iv) the HCDR1, the HCDR2, and the HCDR3 are selected from the three complementarity determining regions HCDR1, HCDR2, and HCDR3 in the VH set forth in SEQ ID NO: 30; and the LCDR1, the LCDR2, and the LCDR3 are the three complementarity determining regions LCDR1, LCDR2, and LCDR3 in the VL set forth in SEQ ID NO: 27; or
(v) relative to the sequence in any one of (i)-(iv), a sequence comprising at least one and no more than 5, 4, 3, 2, or 1 amino acid modification (preferably amino acid substitution, and more preferably conservative substitution) in the three HCDRs or the three LCDRs; for example, the amino acid modification is a mutation enhancing the stability of the antibody, e.g., a mutation at a deamidation site in, e.g., the HCDR2, including a G56R substitution;
preferably, the CDRs are determined by the Kabat numbering scheme.

6. The fusion protein molecule according to any one of claims 2-4, wherein the first target-binding region comprises:
(i) the HCDR1 set forth in SEQ ID NO: 10, the HCDR2 set forth in SEQ ID NO: 11, the HCDR3 set forth in SEQ ID NO: 12; the LCDR1 set forth in SEQ ID NO: 4, the LCDR2 set forth in SEQ ID NO: 5, and the LCDR3 set forth in SEQ ID NO: 6;
(ii) the HCDR1 set forth in SEQ ID NO: 10, the HCDR2 set forth in SEQ ID NO: 22, the HCDR3 set forth in SEQ ID NO: 12; the LCDR1 set forth in SEQ ID NO: 4, the LCDR2 set forth in SEQ ID NO: 5, and the LCDR3 set forth in SEQ ID NO: 6;
(iii) the HCDR1, HCDR2, HCDR3 and LCDR1, LCDR2, and LCDR3 of the 28H1 antibody disclosed in WO2012020006; or
(iv) relative to the sequence in any one of (i), (ii), or (iii), a sequence comprising at least one and no more than 5, 4, 3, 2, or 1 amino acid modification (preferably amino acid substitution, and more preferably conservative substitution) in the three HCDRs or the three LCDRs; for example, the amino acid modification is a mutation enhancing the stability of the antibody, e.g., a mutation at a deamidation site in, e.g., the HCDR2, including a G56R substitution.

7. The fusion protein molecule according to any one of claims 2-6, wherein the first target-binding region comprises a heavy chain variable region VH, and the heavy chain variable region:
(i) comprises or consists of an amino acid sequence having at least 90%, 91%, 92%, 93%, 94%, 95%, 96%, 97%, 98%, or 99% identity to the amino acid sequence of SEQ ID NO: 7, 13, 21, or 30; or
(ii) comprises or consists of the amino acid sequence of SEQ ID NO: 7, 13, 21, or 30.

8. The fusion protein molecule according to any one of claims 2-7, wherein the first target-binding region comprises a light chain variable region VL, and the light chain variable region:
(i) comprises or consists of an amino acid sequence having at least 90%, 91%, 92%, 93%, 94%, 95%, 96%, 97%, 98%, or 99% identity to the amino acid sequence of SEQ ID NO: 1, 15, or 27; or
(ii) comprises or consists of the amino acid sequence of SEQ ID NO: 1, 15, or 27.

9. The fusion protein molecule according to any one of claims 2-8, comprising a heavy chain variable region VH and a light chain variable region VL, wherein:
(i) the heavy chain variable region comprises or consists of the amino acid sequence set forth in SEQ ID NO: 7, or an amino acid sequence having at least 90%, 91%, 92%, 93%, 94%, 95%, 96%, 97%, 98%, or 99% identity to the amino acid sequence; and the light chain variable region comprises or consists of the amino acid sequence set forth in SEQ ID NO: 1, or an amino acid sequence having at least 90%, 91%, 92%, 93%, 94%, 95%, 96%, 97%, 98%, or 99% identity to the amino acid sequence;
(ii) the heavy chain variable region comprises or consists of the amino acid sequence set forth in SEQ ID NO: 13, or an amino acid sequence having at least 90%, 91%, 92%, 93%, 94%, 95%, 96%, 97%, 98%, or 99% identity to the amino acid sequence; and the light chain variable region comprises or consists of the amino acid sequence set forth in SEQ ID NO: 15, or an amino acid sequence having at least 90%, 91%, 92%, 93%, 94%, 95%, 96%, 97%, 98%, or 99% identity to the amino acid sequence;
(iii) the heavy chain variable region comprises or consists of the amino acid sequence set forth in SEQ ID NO: 21, or an amino acid sequence having at least 90%, 91%, 92%, 93%, 94%, 95%, 96%, 97%, 98%, or 99% identity to the amino acid sequence; and the light chain variable region comprises or consists of the amino acid sequence set forth in SEQ ID NO: 15, or an amino acid sequence having at least 90%, 91%, 92%, 93%, 94%, 95%, 96%, 97%, 98%, or 99% identity to the amino acid sequence; or
(iv) the heavy chain variable region comprises or consists of the amino acid sequence set forth in SEQ ID NO: 30, or an amino acid sequence having at least 90%, 91%, 92%, 93%, 94%, 95%, 96%, 97%, 98%, or 99% identity to the amino acid sequence; and the light chain variable region comprises or consists of the amino acid sequence set forth in SEQ ID NO: 27, or an amino acid sequence having at least 90%, 91%, 92%, 93%, 94%, 95%, 96%, 97%, 98%, or 99% identity to the amino acid sequence.

10. The fusion protein molecule according to any one of claims 2-9, wherein the first target-binding region comprises a heavy chain variable region VH and a light chain variable region VL, and the VH and the VL respectively comprise or consist of the amino acid sequences set forth as follows:
(i) SEQ ID NO: 7 and SEQ ID NO: 1;
(ii) SEQ ID NO: 13 and SEQ ID NO: 15;
(iii) SEQ ID NO: 21 and SEQ ID NO: 15; or
(iv) SEQ ID NO: 30 and SEQ ID NO: 27.

11. The fusion protein molecule according to any one of claims 2-10, wherein the first target-binding region further comprises an Fc or a heavy chain constant region CH; for example,
the Fc region is the Fc region of an IgG1, IgG2, IgG3, or IgG4, preferably the Fc region of an IgG1; and optionally, amino acid K at the terminus of the Fc region is deleted or mutated to A; or
the antibody heavy chain constant region CH is the heavy chain constant region of an IgG1, IgG2, IgG3, or IgG4, preferably the heavy chain constant region of an IgG1; and optionally, amino acid K at the terminus of the heavy chain constant region is deleted or mutated to A.

12. The fusion protein molecule according to claim 11, wherein the heavy chain constant region CH:
(i) comprises or consists of an amino acid sequence having at least 85%, 90%, 91%, 92%, 93%, 94%, 95%, 96%, 97%, 98%, or 99% identity to the amino acid sequence of SEQ ID NO: 8; or
(ii) comprises or consists of the amino acid sequence of SEQ ID NO: 8; or
the Fc region:
(i) comprises or consists of an amino acid sequence having at least 85%, 90%, 91%, 92%, 93%, 94%, 95%, 96%, 97%, 98%, or 99% identity to the amino acid sequence of SEQ ID NO: 40; or
(ii) comprises or consists of the amino acid sequence of SEQ ID NO: 40.

13. The fusion protein molecule according to any one of claims 2-12, wherein the first target-binding region further comprises a light chain constant region; for example, the light chain constant region is a lambda or kappa light chain constant region.

14. The fusion protein molecule according to claim 13, wherein the light chain constant region:
(i) comprises or consists of an amino acid sequence having at least 85%, 90%, 91%, 92%, 93%, 94%, 95%, 96%, 97%, 98%, or 99% identity to the amino acid sequence of SEQ ID NO: 2; or
(ii) comprises or consists of the amino acid sequence of SEQ ID NO: 2.

15. The fusion protein molecule according to any one of claims 2-14, wherein the first target-binding region comprises a heavy chain, and the heavy chain comprises or consists of the amino acid sequence of SEQ ID NO: 9, 14, 23, 35, 41, or 42, or an amino acid sequence having at least 85%, 90%, 91%, 92%, 93%, 94%, 95%, 96%, 97%, 98%, or 99% identity to the amino acid sequence.

16. The fusion protein molecule according to any one of claims 2-15, wherein the first target-binding region comprises a light chain, and the light chain comprises or consists of the amino acid sequence of SEQ ID NO: 3, 16, or 28, or an amino acid sequence having at least 85%, 90%, 91%, 92%, 93%, 94%, 95%, 96%, 97%, 98%, or 99% identity to the amino acid sequence.

17. The fusion protein molecule according to claim 15 or 16, wherein the first target-binding region comprises a heavy chain and a light chain, wherein
(i) the heavy chain comprises or consists of the amino acid sequence of SEQ ID NO: 9, or an amino acid sequence having at least 85%, 90%, 91%, 92%, 93%, 94%, 95%, 96%, 97%, 98%, or 99% identity to the amino acid sequence; and the light chain comprises or consists of the amino acid sequence of SEQ ID NO: 3, or an amino acid sequence having at least 85%, 90%, 91%, 92%, 93%, 94%, 95%, 96%, 97%, 98%, or 99% identity to the amino acid sequence;
(ii) the heavy chain comprises or consists of the amino acid sequence of SEQ ID NO: 14, or an amino acid sequence having at least 85%, 90%, 91%, 92%, 93%, 94%, 95%, 96%, 97%, 98%, or 99% identity to the amino acid sequence; and the light chain comprises or consists of the amino acid sequence of SEQ ID NO: 16, or an amino acid sequence having at least 85%, 90%, 91%, 92%, 93%, 94%, 95%, 96%, 97%, 98%, or 99% identity to the amino acid sequence;
(iii) the heavy chain comprises or consists of the amino acid sequence of SEQ ID NO: 23, or an amino acid sequence having at least 85%, 90%, 91%, 92%, 93%, 94%, 95%, 96%, 97%, 98%, or 99% identity to the amino acid sequence; and the light chain comprises or consists of the amino acid sequence of SEQ ID NO: 16, or an amino acid sequence having at least 85%, 90%, 91%, 92%, 93%, 94%, 95%, 96%, 97%, 98%, or 99% identity to the amino acid sequence;
(iv) the heavy chain comprises or consists of the amino acid sequence of SEQ ID NO: 35, or an amino acid sequence having at least 85%, 90%, 91%, 92%, 93%, 94%, 95%, 96%, 97%, 98%, or 99% identity to the amino acid sequence; and the light chain comprises or consists of the amino acid sequence of SEQ ID NO: 16, or an amino acid sequence having at least 85%, 90%, 91%, 92%, 93%, 94%, 95%, 96%, 97%, 98%, or 99% identity to the amino acid sequence; or
(v) the heavy chain comprises or consists of the amino acid sequence of SEQ ID NO: 41 or 42, or an amino acid sequence having at least 85%, 90%, 91%, 92%, 93%, 94%, 95%, 96%, 97%, 98%, or 99% identity to the amino acid sequence; and the light chain comprises or consists of the amino acid sequence of SEQ ID NO: 28, or an amino acid sequence having at least 85%, 90%, 91%, 92%, 93%, 94%, 95%, 96%, 97%, 98%, or 99% identity to the amino acid sequence.

18. The fusion protein molecule according to claim 17, wherein the first target-binding region comprises a heavy chain and a light chain respectively comprising or consisting of the amino acid sequences set forth in the following SEQ ID NOs:
(i) SEQ ID NO: 9 and SEQ ID NO: 3;
(ii) SEQ ID NO: 14 and SEQ ID NO: 16;
(iii) SEQ ID NO: 23 and SEQ ID NO: 16;
(iv) SEQ ID NO: 35 and SEQ ID NO: 16;
(v) SEQ ID NO: 41 and SEQ ID NO: 28; or
(vi) SEQ ID NO: 42 and SEQ ID NO: 28.

19. The fusion protein molecule according to any one of claims 2-18, wherein the first target-binding region is a humanized antibody or a chimeric antibody.

20. The fusion protein molecule according to any one of claims 2-19, wherein the first target-binding region is a monoclonal antibody.

21. The fusion protein molecule according to any one of claims 2-20, wherein the first target-binding region is an antigen-binding fragment selected from a Fab, a Fab', a Fab'-SH, an Fv, a single-chain antibody (e.g., scFv), (Fab')₂, a single-domain antibody (e.g., VHH), a domain antibody (dAb), and a linear antibody.

22. The fusion protein molecule according to any one of claims 2-21, wherein the second target-binding region comprises a TGFβ receptor specifically binding to TGFβ or a functional fragment thereof.

23. The fusion protein molecule according to claim 22, wherein the TGFβ receptor is a TGFβRII molecule; e.g., the TGFβRII molecule:
(i) comprises or consists of an amino acid sequence having at least 85%, 90%, 91%, 92%, 93%, 94%, 95%, 96%, 97%, 98%, or 99% identity to an amino acid sequence selected from SEQ ID NO: 34;
(ii) comprises or consists of an amino acid sequence selected from SEQ ID NO: 34; or
(iii) comprises or consists of an amino acid sequence having 1 or more (preferably no more than 20 or 10, and more preferably no more than 5, 4, 3, 2, or 1) amino acid modifications (preferably amino acid substitutions, and more preferably amino acid conservative substitutions) compared to an amino acid sequence selected from SEQ ID NO: 34.

24. The fusion protein molecule according to claim 22 or 23, wherein the functional fragment comprises an ECD or is an ECD.

25. The fusion protein molecule according to claim 24, wherein the ECD is an ECD of a wild-type TGFβ receptor, e.g., an ECD of a wild-type TGFβRII molecule, or a variant ECD comprising a modification relative to a wild-type ECD; for example, the modification comprises a truncation at the N-terminus or the C-terminus or a substitution.

26. The fusion protein molecule according to claim 25, wherein
the substitution is selected from: a substitution of G at position 20 of the ECD with T, i.e., G20T; a substitution of N at position 19 of the ECD with E; and a combination of the two; or
the truncation is an N-terminus truncation, e.g., a deletion of any or all of the amino acids at positions 1-19 at the N-terminus, for example, selected from a deletion of amino acids at positions 1-19 of the ECD, a deletion of amino acids at positions 4-19 of the ECD, and a deletion of amino acids at positions 18-19 of the ECD.

27. The fusion protein molecule according to any one of claims 24-26, wherein the ECD is an ECD of a TGFβRII molecule; for example, the ECD of the TGFβRII molecule:
(i) comprises or consists of an amino acid sequence having at least 85%, 90%, 91%, 92%, 93%, 94%, 95%, 96%, 97%, 98%, or 99% identity to the amino acid sequence of SEQ ID NO: 24, 37, or 39;
(ii) comprises or consists of the amino acid sequence of SEQ ID NO: 24, 37, or 39;
(iii) comprises or consists of an amino acid sequence having 1 or more (preferably no more than 20 or 10, and more preferably no more than 5, 4, 3, 2, or 1) amino acid modifications (preferably amino acid substitutions, and more preferably amino acid conservative substitutions) compared to the amino acid sequence of SEQ ID NO: 24, 37, or 39; wherein preferably, the amino acid modification is selected from the presence of T at position 20 (i.e., comprising G20T), and/or the presence of E at position 19 (i.e., comprising N19E);
(iv) comprises an amino acid sequence having a truncation at the N-terminus and/or C-terminus compared to any one of (i)-(iii), wherein preferably, the truncation refers to a deletion of any or all of the amino acids at positions 1-19 at the N-terminus, e.g., a deletion of amino acids at positions 1-19 of the ECD, a deletion of amino acids at positions 4-19 of the ECD, or a deletion of amino acids at positions 18-19 of the ECD.

28. The fusion protein molecule according to any one of claims 1-27, comprising
(i) chain A: an anti-FAP antibody light chain; and
(ii) chain B: an anti-FAP antibody heavy chain connected, at the N-terminus or C-terminus, to a TGFβ receptor or a functional fragment thereof with or without a linker.

29. The fusion protein molecule according to claim 28, consisting of 2 chains A and 2 chains B.

30. The fusion protein molecule according to claim 28 or 29, wherein chain A comprises or consists of the amino acid sequence set forth in SEQ ID NO: 16, or an amino acid sequence having at least 85%, 90%, 91%, 92%, 93%, 94%, 95%, 96%, 97%, 98%, or 99% identity to the amino acid sequence.

31. The fusion protein molecule according to any one of claims 28-30, wherein chain B comprises or consists of the amino acid sequence set forth in SEQ ID NO: 25, 36, or 38, or an amino acid sequence having at least 85%, 90%, 91%, 92%, 93%, 94%, 95%, 96%, 97%, 98%, or 99% identity to the amino acid sequence.

32. The fusion protein molecule according to claim 28 or 29, wherein
chain A comprises or consists of the amino acid sequence set forth in SEQ ID NO: 16, or an amino acid sequence having at least 85%, 90%, 91%, 92%, 93%, 94%, 95%, 96%, 97%, 98%, or 99% identity to the amino acid sequence; and
chain B comprises or consists of the amino acid sequence set forth in SEQ ID NO: 25, 36, or 38, or an amino acid sequence having at least 85%, 90%, 91%, 92%, 93%, 94%, 95%, 96%, 97%, 98%, or 99% identity to the amino acid sequence; or
chain A comprises or consists of the amino acid sequence set forth in SEQ ID NO: 28, or an amino acid sequence having at least 85%, 90%, 91%, 92%, 93%, 94%, 95%, 96%, 97%, 98%, or 99% identity to the amino acid sequence; and
chain B comprises or consists of the amino acid sequence set forth in SEQ ID NO: 29, or an amino acid sequence having at least 85%, 90%, 91%, 92%, 93%, 94%, 95%, 96%, 97%, 98%, or 99% identity to the amino acid sequence.

33. The fusion protein molecule according to claim 32, wherein chain A comprises or consists of the amino acid sequence set forth in SEQ ID NO: 16, and chain B comprises or consists of the amino acid sequence set forth in SEQ ID NO: 25, 36, or 38; or chain A comprises or consists of the amino acid sequence set forth in SEQ ID NO: 28, and chain B comprises or consists of the amino acid sequence set forth in SEQ ID NO: 29.

34. An anti-FAP antibody or an antigen-binding fragment thereof, comprising:
the HCDR1 set forth in SEQ ID NO: 10, the HCDR2 set forth in SEQ ID NO: 22, the HCDR3 set forth in SEQ ID NO: 12; the LCDR1 set forth in SEQ ID NO: 4, the LCDR2 set forth in SEQ ID NO: 5, and the LCDR3 set forth in SEQ ID NO: 6.

35. An anti-FAP antibody or an antigen-binding fragment thereof, comprising a heavy chain variable region VH and a light chain variable region VL, wherein
(i) the heavy chain variable region comprises or consists of the amino acid sequence set forth in SEQ ID NO: 13, or an amino acid sequence having at least 90%, 91%, 92%, 93%, 94%, 95%, 96%, 97%, 98%, or 99% identity to the amino acid sequence; and the light chain variable region comprises or consists of the amino acid sequence set forth in SEQ ID NO: 15, or an amino acid sequence having at least 90%, 91%, 92%, 93%, 94%, 95%, 96%, 97%, 98%, or 99% identity to the amino acid sequence; or
(iii) the heavy chain variable region comprises or consists of the amino acid sequence set forth in SEQ ID NO: 21, or an amino acid sequence having at least 90%, 91%, 92%, 93%, 94%, 95%, 96%, 97%, 98%, or 99% identity to the amino acid sequence; and the light chain variable region comprises or consists of the amino acid sequence set forth in SEQ ID NO: 15, or an amino acid sequence having at least 90%, 91%, 92%, 93%, 94%, 95%, 96%, 97%, 98%, or 99% identity to the amino acid sequence.

36. The anti-FAP antibody or the antigen-binding fragment thereof according to claim 34 or 35, comprising a heavy chain variable region VH and a light chain variable region VL, wherein the VH and the VL respectively comprise or consist of the amino acid sequences set forth as follows:
(i) SEQ ID NO: 13 and SEQ ID NO: 15; or
(ii) SEQ ID NO: 21 and SEQ ID NO: 15.

37. The anti-FAP antibody or the antigen-binding fragment thereof according to any one of claims 34-36, further comprising an Fc or a heavy chain constant region CH, wherein for example,
the Fc region is the Fc region of an IgG1, IgG2, IgG3, or IgG4, preferably the Fc region of an IgG1; and optionally, amino acid K at the terminus of the Fc region is deleted or mutated to A; or
the antibody heavy chain constant region CH is the heavy chain constant region of an IgG1, IgG2, IgG3, or IgG4, preferably the heavy chain constant region of an IgG1; and optionally, amino acid K at the terminus of the heavy chain constant region is deleted or mutated to A.

38. The anti-FAP antibody or the antigen-binding fragment thereof according to claim 37, wherein the heavy chain constant region CH:
(i) comprises or consists of an amino acid sequence having at least 85%, 90%, 91%, 92%, 93%, 94%, 95%, 96%, 97%, 98%, or 99% identity to the amino acid sequence of SEQ ID NO: 8; or
(ii) comprises or consists of the amino acid sequence of SEQ ID NO: 8; or
the Fc region:
(i) comprises or consists of an amino acid sequence having at least 85%, 90%, 91%, 92%, 93%, 94%, 95%, 96%, 97%, 98%, or 99% identity to the amino acid sequence of SEQ ID NO: 40; or
(ii) comprises or consists of the amino acid sequence of SEQ ID NO: 40.

39. The anti-FAP antibody or the antigen-binding fragment thereof according to any one of claims 34-38, further comprising a light chain constant region, wherein for example, the light chain constant region is a lambda or kappa light chain constant region.

40. The anti-FAP antibody or the antigen-binding fragment thereof according to claim 39, wherein the light chain constant region:
(i) comprises or consists of an amino acid sequence having at least 85%, 90%, 91%, 92%, 93%, 94%, 95%, 96%, 97%, 98%, or 99% identity to the amino acid sequence of SEQ ID NO: 2; or
(ii) comprises or consists of the amino acid sequence of SEQ ID NO: 2.

41. The anti-FAP antibody or the antigen-binding fragment thereof according to any one of claims 34-40, comprising a heavy chain, wherein the heavy chain comprises or consists of the amino acid sequence of SEQ ID NO: 14, 23, or 35, or an amino acid sequence having at least 85%, 90%, 91%, 92%, 93%, 94%, 95%, 96%, 97%, 98%, or 99% identity to the amino acid sequence.

42. The anti-FAP antibody or the antigen-binding fragment thereof according to any one of claims 34-41, comprising a light chain, wherein the light chain comprises or consists of the amino acid sequence of SEQ ID NO: 16, or an amino acid sequence having at least 85%, 90%, 91%, 92%, 93%, 94%, 95%, 96%, 97%, 98%, or 99% identity to the amino acid sequence.

43. The anti-FAP antibody or the antigen-binding fragment thereof according to claim 41 or 42, comprising a heavy chain and a light chain, wherein
(i) the heavy chain comprises or consists of the amino acid sequence of SEQ ID NO: 14, or an amino acid sequence having at least 85%, 90%, 91%, 92%, 93%, 94%, 95%, 96%, 97%, 98%, or 99% identity to the amino acid sequence; and the light chain comprises or consists of the amino acid sequence of SEQ ID NO: 16, or an amino acid sequence having at least 85%, 90%, 91%, 92%, 93%, 94%, 95%, 96%, 97%, 98%, or 99% identity to the amino acid sequence;
(ii) the heavy chain comprises or consists of the amino acid sequence of SEQ ID NO: 23, or an amino acid sequence having at least 85%, 90%, 91%, 92%, 93%, 94%, 95%, 96%, 97%, 98%, or 99% identity to the amino acid sequence; and the light chain comprises or consists of the amino acid sequence of SEQ ID NO: 16, or an amino acid sequence having at least 85%, 90%, 91%, 92%, 93%, 94%, 95%, 96%, 97%, 98%, or 99% identity to the amino acid sequence; or
(iii) the heavy chain comprises or consists of the amino acid sequence of SEQ ID NO: 35, or an amino acid sequence having at least 85%, 90%, 91%, 92%, 93%, 94%, 95%, 96%, 97%, 98%, or 99% identity to the amino acid sequence; and the light chain comprises or consists of the amino acid sequence of SEQ ID NO: 16, or an amino acid sequence having at least 85%, 90%, 91%, 92%, 93%, 94%, 95%, 96%, 97%, 98%, or 99% identity to the amino acid sequence.

44. The anti-FAP antibody or the antigen-binding fragment thereof according to claim 43, comprising a heavy chain and a light chain, wherein the heavy chain and the light chain respectively comprise or consist of the amino acid sequences set forth in the following SEQ ID NOs:
(i) SEQ ID NO: 14 and SEQ ID NO: 16;
(ii) SEQ ID NO: 23 and SEQ ID NO: 16; or
(iii) SEQ ID NO: 35 and SEQ ID NO: 16.

45. The fusion protein molecule according to any one of claims 34-44, wherein the anti-FAP antibody or the antigen-binding fragment thereof is a humanized antibody or a chimeric antibody.

46. The fusion protein molecule according to any one of claims 34-45, wherein the anti-FAP antibody or the antigen-binding fragment thereof is a monoclonal antibody.

47. The fusion protein molecule according to any one of claims 34-46, wherein the anti-FAP antibody or the antigen-binding fragment thereof is an antigen-binding fragment selected from a Fab, a Fab', a Fab'-SH, an Fv, a single-chain antibody (e.g., scFv), (Fab')₂, a single-domain antibody (e.g., VHH), a domain antibody (dAb), and a linear antibody.

48. A nucleic acid molecule, comprising or consisting of a nucleic acid sequence encoding the fusion protein molecule according to any one of claims 1-33 or any one of the chains of the antibody according to any one of claims 34-47.

49. An expression vector, comprising the nucleic acid molecule according to claim 48, wherein preferably, the expression vector is pCDNA, e.g., pCDNA3.1.

50. A host cell, comprising the nucleic acid molecule according to claim 48 or the expression vector according to claim 49, wherein preferably, the host cell is prokaryotic or eukaryotic, for example, a 293 cell or a CHO cell, e.g., a 293F cell, a 293T cell, or a CHO-S cell.

51. A method for preparing the fusion protein molecule according to any one of claims 1-33 or the antibody according to any one of claims 34-47, comprising: culturing a host cell comprising the nucleic acid molecule according to claim 48 or the expression vector according to claim 49 in a condition suitable for expressing a chain of the fusion protein, and optionally, recovering the fusion protein molecule or the antibody from the host cell (or the host cell culture medium).

52. A pharmaceutical composition or a medicament or a formulation, comprising the fusion protein molecule according to any one of claims 1-33 or the antibody according to any one of claims 34-47, and optionally, a pharmaceutical supplementary material.

53. A pharmaceutical combination product, comprising the fusion protein molecule according to any one of claims 1-33 or the antibody according to any one of claims 34-47, and one or more additional therapeutic agents (e.g., an antifibrotic, an additional antibody, or a small molecule drug).

54. A method for preventing or treating fibrosis (e.g., fibrosis in the lung, kidney, liver, or skin) in a subject, comprising: administering to the subject an effective amount of the fusion protein molecule according to any one of claims 1-33 or the antibody according to any one of claims 34-47, or the pharmaceutical composition or the formulation according to claim 52; or the pharmaceutical combination product according to claim 53.

55. A method for alleviating a fibrotic lesion (e.g., a fibrotic lesion in the lung, kidney, or liver, such as idiopathic pulmonary fibrosis, liver cirrhosis, scleroderma, or diabetic nephropathy) in a subject, comprising: administering to the subject an effective amount of the fusion protein molecule according to any one of claims 1-33 or the antibody according to any one of claims 34-47, or the pharmaceutical composition or the formulation according to claim 52; or the pharmaceutical combination product according to claim 53.

56. A method for inhibiting the proliferation of a fibroblast, e.g., an activated fibroblast, such as a (activated) fibroblast specifically expressing FAP, in a subject, comprising: administering to the subject an effective amount of the fusion protein molecule according to any one of claims 1-33 or the antibody according to any one of claims 34-47, or the pharmaceutical composition or the formulation according to claim 52; or the pharmaceutical combination product according to claim 53.

57. The method according to any one of claims 54-56, wherein FAP expression or elevated FAP expression (e.g., compared to a tissue/organ of a healthy subject or compared to a healthy tissue/organ of the same patient) is present in a tissue or organ with fibrosis of the subject.

58. The method according to claim 57, wherein FAP expression or elevated FAP expression (e.g., compared to a tissue/organ of a healthy subject or compared to a fibroblast in a healthy tissue/organ of the same patient) is present in a fibroblast in a tissue or organ with fibrosis of the subject.

59. The method according to any one of claims 54-56, wherein a FAP nucleic acid or an elevated FAP nucleic acid level (e.g., compared to a tissue/organ of a healthy subject or compared to a healthy tissue/organ of the same patient) is present in a tissue or organ with fibrosis of the subject.

60. The method according to claim 59, wherein a FAP nucleic acid or an elevated FAP nucleic acid level (e.g., compared to a tissue/organ of a healthy subject or compared to a fibroblast in a healthy tissue/organ of the same patient) is present in a fibroblast in a tissue or organ with fibrosis of the subject.
